# EUROPEAN PATENT APPLICATION

(11) **EP 4 786 472 A1**
(43) Date of publication of application: **05.08.2026**
(21) Application number: 24870988.3
(22) Date of filing: 27.09.2024
(51) Int. Cl.: C07D 495/14, C07D 471/14, A61K 31/551, A61P 35/00, A61P 37/00

(54) **BRD4 PROTEIN DEGRADATION AGENT COMPOUND, AND PREPARATION METHOD AND USE THEREFOR**

(30) Priority: 27.09.2023 CN 202311263376; 27.09.2023 US 202318475989
(71) Applicant: Miracure Biotechnology Limited, Haidian, Beijing 100192 (CN)
(72) Inventor: LI, Xiaolin, Beijing 100192 (CN); QI, Longwu, Beijing 100192 (CN); CHEN, Xibo, Beijing 100192 (CN)
(74) Representative: Gille Hrabal Partnerschaftsgesellschaft mbB Patentanwälte
(86) International application number: PCT/CN2024/121820
(87) International publication number: WO 2025/067451

(57) **Abstract**

Provided are a compound as represented by formula (I), a racemate, stereoisomer, tautomer, isotope marker, nitrogen oxide, solvate, polymorph, metabolite, ester, and prodrug thereof, or a pharmaceutically acceptable salt thereof, which as drugs have good BRD4 inhibition and degradation effects, and can be used for preparing drugs related to BRD4 inhibition and degradation, and prevention and/or treatment of related diseases.

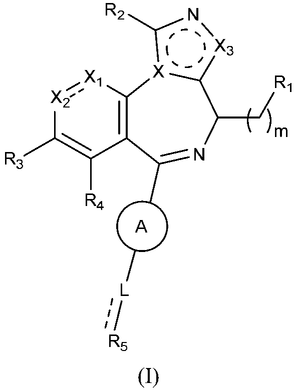

## Description

The disclosure claims priority to:
the prior application filed with the China National Intellectual Property Administration on September 27, 2023, with application No. 2023112633767, entitled "BRD4 PROTEIN DEGRADER COMPOUND, AND PREPARATION METHOD AND USE THEREOF";
and the prior application filed with the United States Patent and Trademark Office on September 27, 2023, with application No. 18/475,989, entitled BRD4 PROTEIN DEGRADER COMPOUND, AND PREPARATION METHOD AND USE THEREOF";

The entire contents of the prior applications are incorporated herein by reference.

### TECHNICAL FIELD

The present disclosure belongs to the field of pharmaceuticals, and particularly relates to a BRD4 protein degrader compound, and a preparation method and use thereof.

### BACKGROUND

Bromodomain (BRD) is an evolutionarily highly conserved protein domain capable of recognizing and binding to acetylated lysine residues of histones, which consists of four anti-parallel α helices (αZ, αA, αB, and αC) and two hydrophobic loops (ZA and BC) formed of approximately 110 amino acids. In the human genome, there are 61 bromodomains distributed in 46 bromodomain-containing proteins, which are classified into eight families according to structural or sequence similarity. Among them, the bromodomain and extraterminal domain (BET) protein family has been the most intensively and extensively studied.

The proteins of the BET family include BRD2 (bromodomain-containing protein 2), BRD3, BRD4, and testis-specific bromodomain-containing protein (testis-specific BRDT), which have structural similarity and each comprise two tandem bromodomains (BD1 and BD2) at the N-terminus and an extraterminal domain (ET). Further, BRD4 and BRDT contain an extended C-terminal domain (CTM). BET proteins are important epigenetic "readers" that can participate in transcriptional regulation and chromatin remodeling through the specific binding of BD1 and BD2 to N-ε-acetyllysine (Kac) residues in histones H3 and H4 or non-histones.

BRD4 includes three characteristic proteins differing in the C-terminal amino acid residue: one long form (BRD-L) and two short forms (BRD-S). The short forms BRD-S (a) and BRD-S (b) are produced by splicing of other mRNAs. The amino acids 1-719 of the three BRD4 proteins are similar, including two highly conserved N-terminal tandem bromodomains (BD1 and BD2), an N-terminal phosphorylation site cluster (NPS), a basic residue-rich interaction domain (BID), an extraterminal domain (ET), and a C-terminal phosphorylation site cluster (CPS).

Mutations in bromodomain-containing proteins have been observed to be associated with cancer and immune and neurological dysfunction. Moreover, recent findings have demonstrated that small molecule inhibition of the bromodomain of BRD4 may have clinical utility in a variety of human diseases ranging from autoimmunity to cardiac hypertrophy. This is possible because the underlying mechanism is transcriptional regulation. Thus, selective inhibition of bromodomains in this family creates various opportunities as novel therapeutic agents in human dysfunction. Thus, there is a need for treatment of cancer, immunological conditions and other bromodomain-related diseases, and for compounds that can inhibit or degrade bromodomains (such as BRD4).

### SUMMARY

To solve the problems in the prior art, the present disclosure provides a compound represented by formula (I) and a racemate, a stereoisomer, a tautomer, an isotopically labeled compound, a nitrogen oxide, a solvate, a polymorph, a metabolite, an ester, a prodrug or a pharmaceutically acceptable salt thereof: 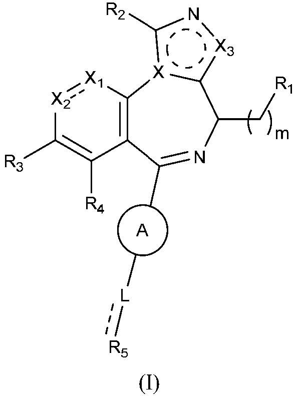 wherein,
------ represents a single bond or a double bond; 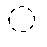 means that the ring in which it is present is aromatic; m is 1 or 2;
ring A is selected from the following groups unsubstituted or optionally substituted with one, two or more Rₐ: C₆₋₁₀ aryl and 5- to 10-membered heteroaryl; each Rₐ is identical or different and is independently selected from halogen, oxo (=O), C₁₋₁₂ alkyl, and C₁₋₁₂ alkoxy;
R₁ is selected from the following groups unsubstituted or optionally substituted with one, two or more R_{b}: 3- to 10-membered heterocyclyl, 5- to 10-membered heteroaryl, -C(O)OR₁₁, -C(O)N(R₁₂)(R₁₃), and -OC(O)R₁₄; R₁₁, R₁₂, R₁₃, and R₁₄ are identical or different and are independently selected from H, OH, NH₂, C₁₋₁₂ alkyl, C₃₋₁₂ cycloalkyl, 3- to 10-membered heterocyclyl, 5- to 10-membered heteroaryl, C₆₋₁₀ aryl, and 3- to 10-membered heterocyclyl-C₁₋₁₂ alkyl; each R_{b} is identical or different and is independently selected from OH, NH₂, C₁₋₁₂ alkyl, and C₂₋₁₂ alkynyl;
R₂ is selected from C₁₋₁₂ alkyl, C₁₋₁₂ alkoxy, halogenated C₁₋₁₂ alkyl, and halogenated C₁₋₁₂ alkoxy;
R₃ and R₄ are identical or different and are independently selected from H, C₁₋₁₂ alkyl, C₁₋₁₂ alkoxy, halogenated C₁₋₁₂ alkyl, and halogenated C₁₋₁₂ alkoxy;
when 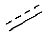 in 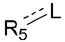 is a double bond, R₅ is selected from =NR₅₄ and 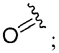
when 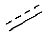 in 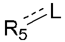 is a single bond, R₅ is selected from H, halogen, and the following groups unsubstituted or optionally substituted with one, two or more R_{c}: NH₂, C₁₋₁₂ alkyl, -C₁₋₁₂ alkyl-N(R₅₁)(R₅₂), -C(O)N(R₅₁)(R₅₂), -C₁₋₁₂ alkyl-O-R₅₃, -C(O)OR₅₃, -C₁₋₁₂ alkyl-NH-C(=NR₅₄)(N(R₅₁)(R₅₂)), -CH=N(R₅₄) , -S(O)₂R₅₅ and -C(O)R₅₅; R₅₁, R₅₂, R₅₃, R₅₄, and R₅₅ are identical or different and are independently selected from H, OH, NH₂, C₁₋₁₂ alkyl, C₁₋₁₂ alkoxy, C₁₋₁₂ alkyl-C(O)-, C₁₋₁₂ alkenyl -C(O)-, and C₁₋₁₂ alkyl-S(O)₂-; each R_{c} is identical or different and is independently selected from H, C₁₋₁₂ alkyl-C(O)-, Boc and Boc-O-;
X is selected from C and N;
X₁ is selected from S and C(R₆); R₆ is selected from H, C₁₋₁₂ alkyl, and C₁₋₁₂ alkoxy;
X₂ is absent or C(R₇); R₇ is selected from H, C₁₋₁₂ alkyl, and C₁₋₁₂ alkoxy;
X₃ is selected from N and C(R₈); R₈ is selected from H, C₁₋₁₂ alkyl, and C₁₋₁₂ alkoxy;
L is absent or selected from halogen, NH, and the following groups unsubstituted or optionally substituted with one, two or more R_{L}: C₁₋₁₂ alkylene, C₂₋₁₂ alkenylene, C₂₋₁₂ alkynylene, C₃₋₁₀ cycloalkylene, 3- to 10-membered heterocyclylene, C₆₋₁₀ arylene, and 5- to 10-membered heteroarylene; each R_{L} is identical or different and is independently selected from H, halogen, oxo (=O), C₁₋₁₂ alkyl, and C₁₋₁₂ alkoxy.

According to an embodiment of the present disclosure, ring A is selected from the following groups unsubstituted or optionally substituted with one, two or more Rₐ: phenyl and

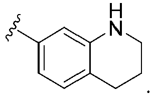

According to an embodiment of the present disclosure, ring A is selected from 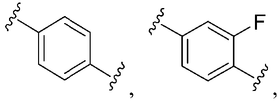 and

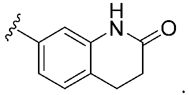

According to an embodiment of the present disclosure, R₁ is selected from the following groups unsubstituted or optionally substituted with one, two or more R_{b}: C₁₋₆ alkyl, 5- to 8-membered heterocyclyl, 5- to 6-membered heteroaryl(such as thiazolyl, oxazolyl, oxadiazolyl, triazolyl), -C(O)OR₁₁, -C(O)N(R₁₂)(R₁₃), and -OC(O)R₁₄; R₁₁, R₁₂, R₁₃, and R₁₄ are identical or different and are independently selected from H, OH, NH₂, C₁₋₆ alkyl, C₃₋₈ cycloalkyl, 3- to 8-membered heterocyclyl, 5- to 6-membered heteroaryl, C₆₋₁₀ aryl, and 3- to 8-membered heterocyclyl-C₁₋₆ alkyl; each R_{b} is identical or different and is independently selected from OH, NH₂, C₁₋₆ alkyl, C₂₋₆ alkynyl and - OC(O)R₁₄.

According to an embodiment of the present disclosure, R₁₁, R₁₂, R₁₃ and R₁₄ are identical or different and are independently selected from H, OH, NH₂, methyl, ethyl, isopropyl, tert-butyl, hydroxyphenyl, propynyl, hydroxyethyl, methylcyclopropyl,

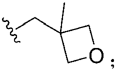

According to an embodiment of the present disclosure, R₁ is selected from

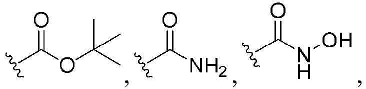

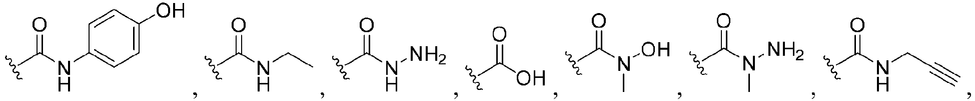

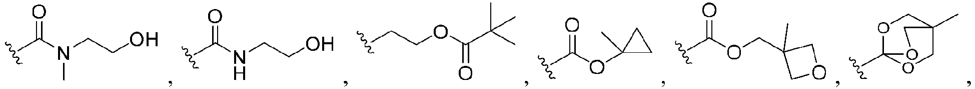

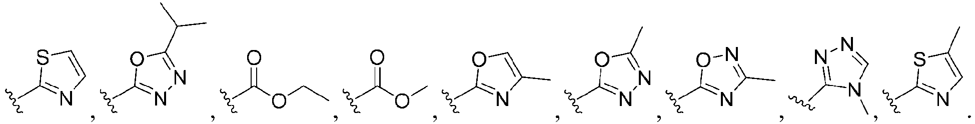

According to an embodiment of the present disclosure, R₂ is selected from C₁₋₆ alkyl, e.g., methyl.

According to an embodiment of the present disclosure, R₃ and R₄ are identical or different and are independently selected from H, C₁₋₆ alkyl, and C₁₋₆ alkoxy, e.g., H, methyl, or methoxy.

According to an embodiment of the present disclosure, when 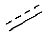 in 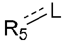 is a double bond, R₅ is selected from =NR₅₄, and 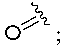 when 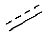 in 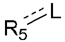 is a single bond, R₅ is selected from H, halogen, and the following groups unsubstituted or optionally substituted with one, two or more R_{c}: NH₂, C₁₋₆ alkyl, -C₁₋₆ alkyl-N(R₅₁)(R₅₂), -C(O)N(R₅₁)(R₅₂), -C₁₋₆ alkyl-O-R₅₃, -C(O)OR₅₃, -C₁₋₆ alkyl-NH-C(=NR₅₄)(N(R₅₁)(R₅₂)), -CH=N(R₅₄), -S(O)₂R₅₅ and -C(O)R₅₅; R₅₁, R₅₂, R₅₃, and R₅₄ are identical or different and are independently selected from H, OH, NH₂, C₁₋₆ alkyl, halogenated C₁₋₆ alkyl, C₂₋₆ alkynyl, C₁₋₆ alkoxy, C₁₋₆ alkyl-C(O)-, C₂₋₆ alkenyl-C(O)-, and C₁₋₆ alkyl-S(O)₂-; each R_{c} is identical or different and is independently selected from H,C₁₋₆ alkyl-C(O)-, Boc and Boc-O-.

According to an embodiment of the present disclosure, when 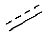 in 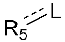 is a double bond, R₅ is selected from 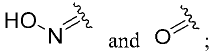 when 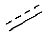 in 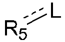 is a single bond, R₅ is selected from H, Cl, NH₂, methyl,

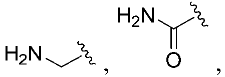

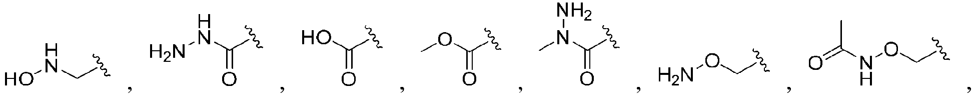

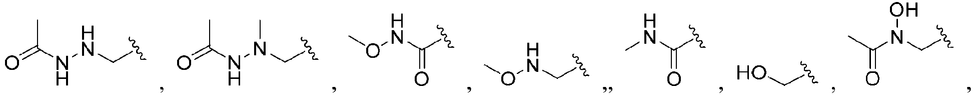

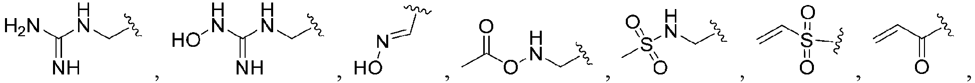

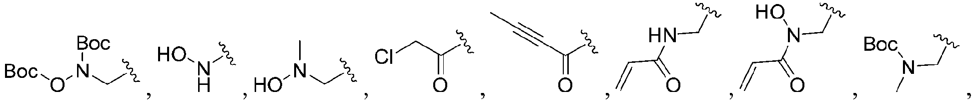

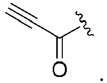

According to an embodiment of the present disclosure, X₁ is selected from S and C(R₆); R₆ is selected from H, C₁₋₆ alkyl, and C₁₋₆ alkoxy;
X₂ is absent or C(R₇); R₇ is selected from H, C₁₋₆ alkyl, and C₁₋₆ alkoxy;
when X₂ is absent, 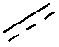 in 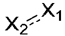 is a single bond, and X₁ is S; when X₂ is C(R₇), 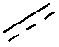 in 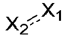 is a double bond, and X₁ is C(R₆).

According to an embodiment of the present disclosure, X₁ is selected from S and CH, and X₂ is absent or CH; when X₂ is absent, 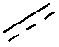 in 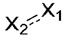 is a single bond, and X₁ is S; when X₂ is CH, 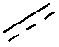 in 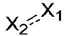 is a double bond, and X₁ is CH.

According to an embodiment of the present disclosure, X₃ is selected from N and CH.

According to an embodiment of the present disclosure, 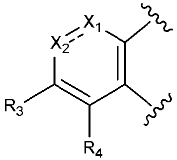 is selected from 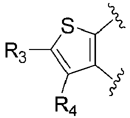 and

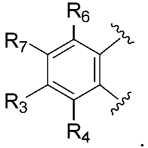

According to an embodiment of the present disclosure, 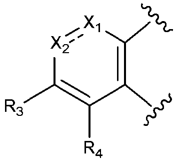 is selected from 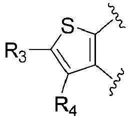 and

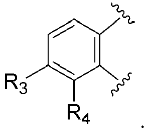

According to an embodiment of the present disclosure, 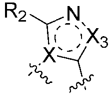 is selected from 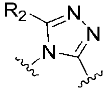 and

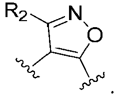

According to an embodiment of the present disclosure, L is absent or selected from halogen, NH, and the following groups unsubstituted or optionally substituted with one, two or more R_{L}: C₁₋₆ alkylene, C₂₋₆ alkenylene, C₂₋₆ alkynylene, C₃₋₈ cycloalkylene, 3- to 8-membered heterocyclylene, C₆₋₁₀ arylene, and 5- to 6-membered heteroarylene; each R_{L} is identical or different and is independently selected from H, halogen, oxo (=O), C₁₋₆ alkyl, and C₁₋₆ alkoxy.

According to an embodiment of the present disclosure, L is absent or selected from Cl, NH, and the following groups unsubstituted or optionally substituted with one, two or more R_{L}: vinylene, ethynylene, 2,3-dihydroisoxazolylene, pyrazolylene, thiazolylene, furylene, azetidinylene, pyrrolylene, 2-azaspiro[3.3]heptanylene, thienylene, phenylene, cyclopropylene, cyclohexenylene, or cyclopentenylene.

According to an embodiment of the present disclosure, L is absent or selected from Cl, NH,

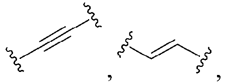

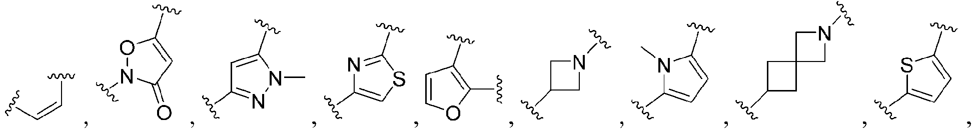

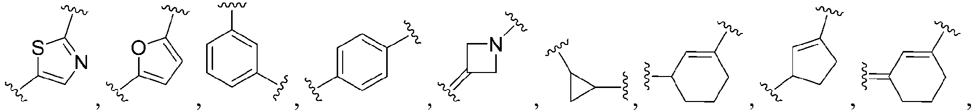

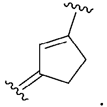

According to an embodiment of the present disclosure, the compound represented by formula (I) is selected from the following structure: 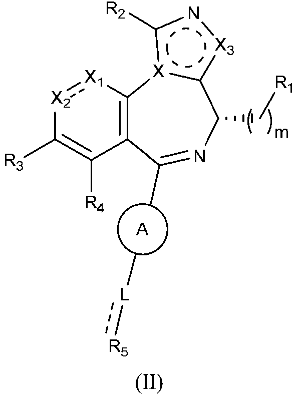 wherein, ------ represents a single bond or a double bond; 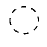 means that the ring in which it is present is aromatic; ring A, R₁, R₂, R₃, R₄, R₅, X₁, X₂, L, and m are as defined above.

According to an embodiment of the present disclosure, the compound represented by formula (I) is selected from the following structures: 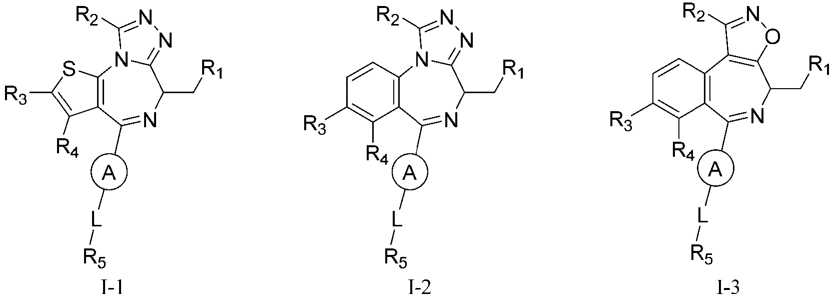 wherein, ring A, R₁, R₂, R₃, R₄, R₅, and L are as defined above.

The present disclosure also provides a compound represented by formula (III) and a racemate, a stereoisomer, a tautomer, an isotopically labeled compound, a nitrogen oxide, a solvate, a polymorph, a metabolite, an ester, a prodrug or a pharmaceutically acceptable salt thereof: 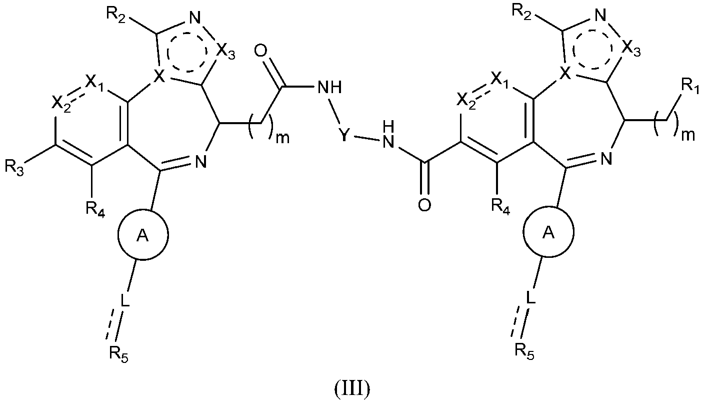 wherein, ring A, R₁, R₂, R₃, R₄, R₅, L, and m are as defined above; Y is selected from -(CH₂)ₚ-[O-(CH₂)_{q}]ᵣ, wherein p is selected from 1, 2, 3, 4, and 5, q is selected from 1, 2, 3, 4, and 5, and r is selected from 0, 1, 2, 3, 4, and 5. According to an embodiment of the present disclosure, exemplary specific compounds of the compound represented by formula (I) are as follows:

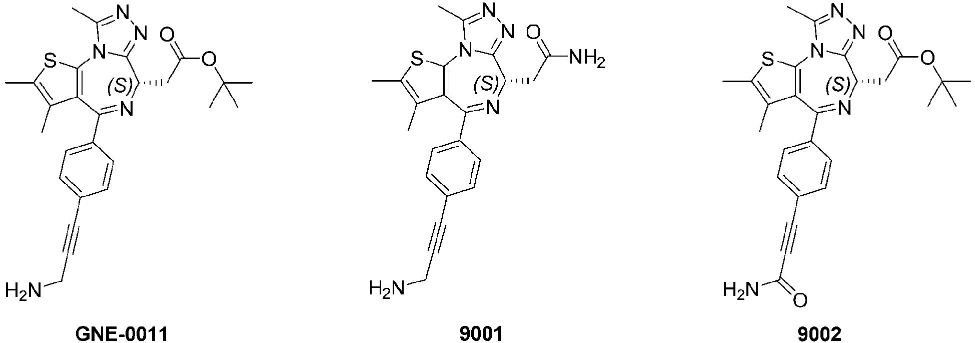

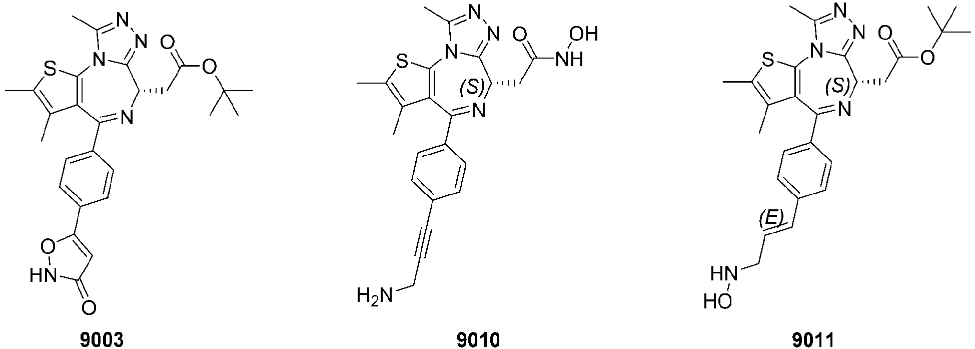

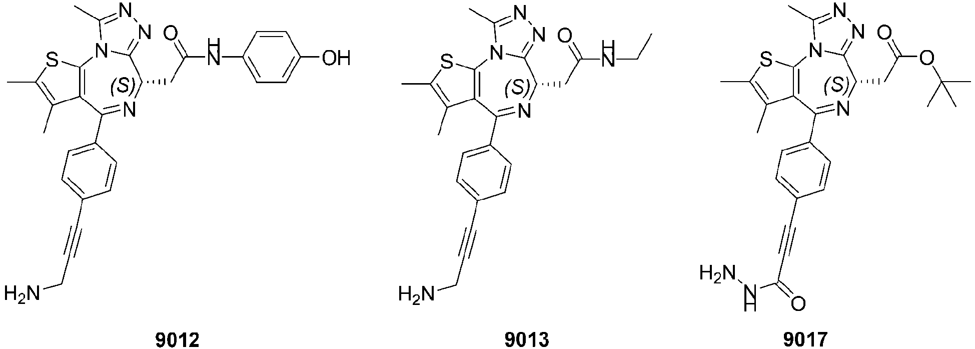

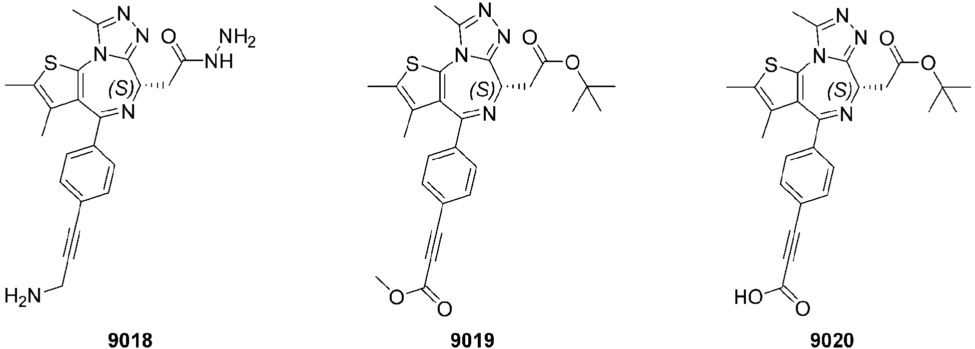

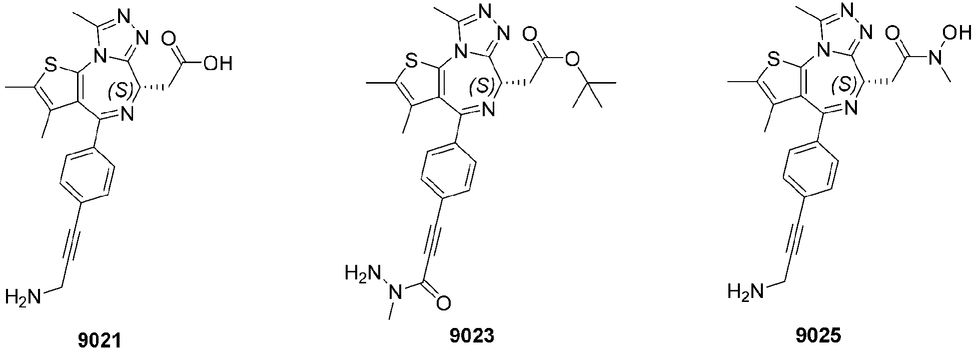

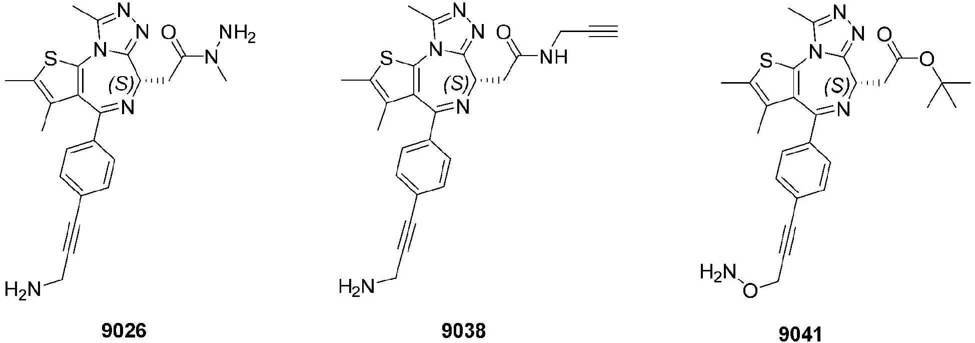

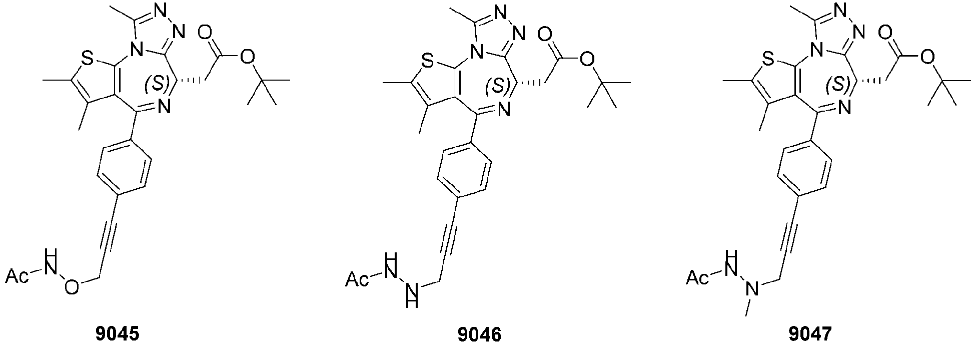

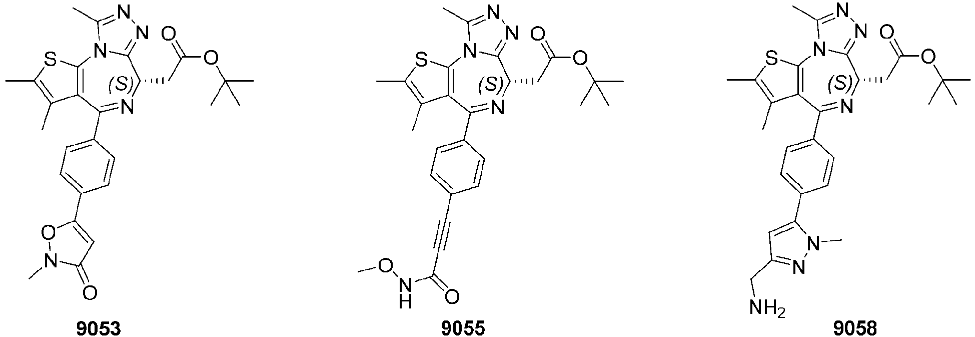

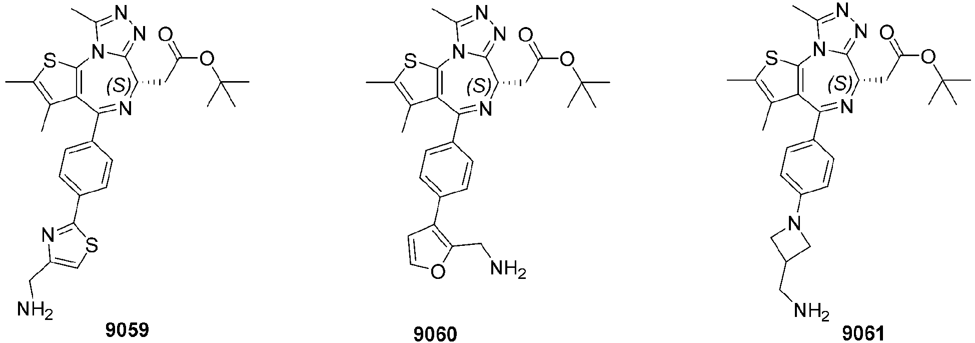

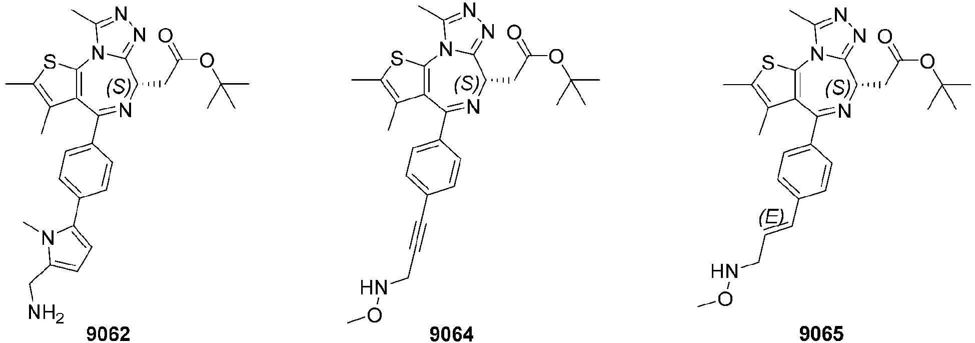

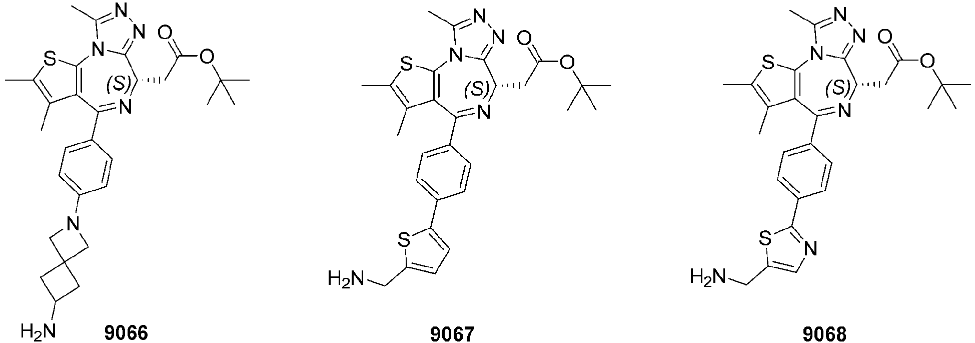

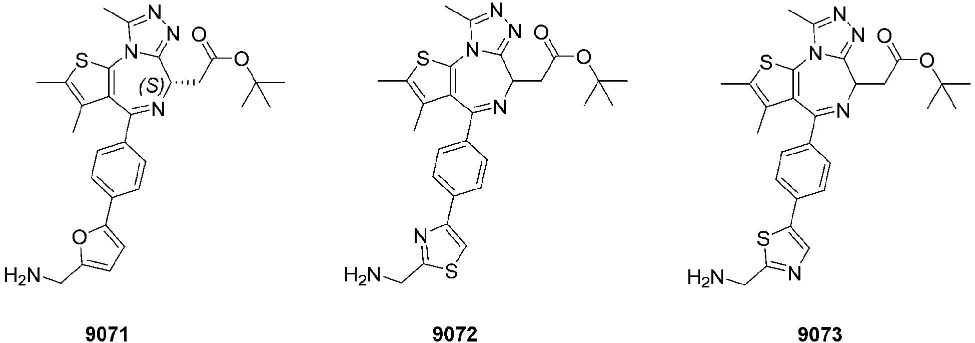

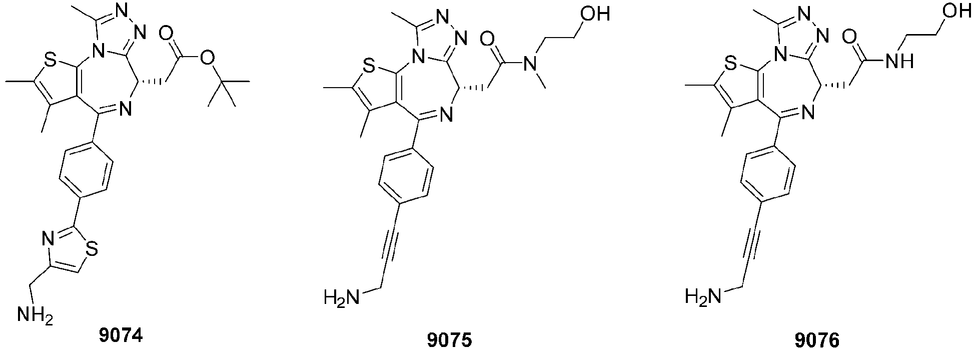

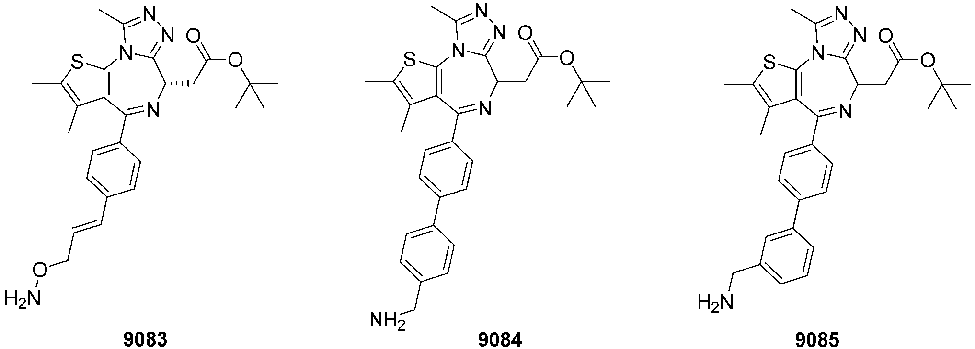

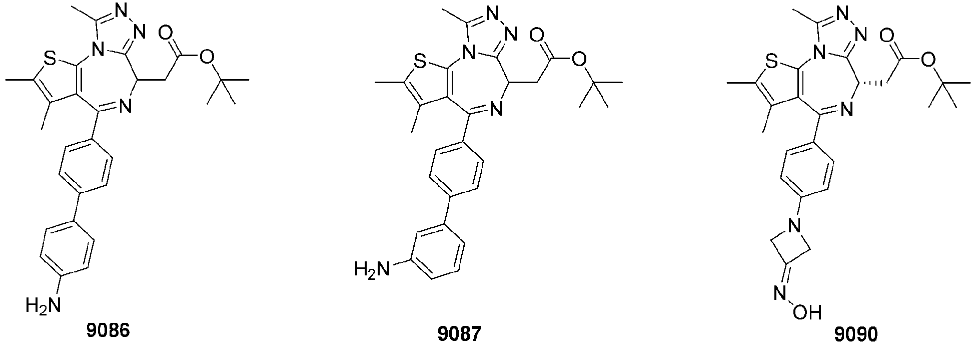

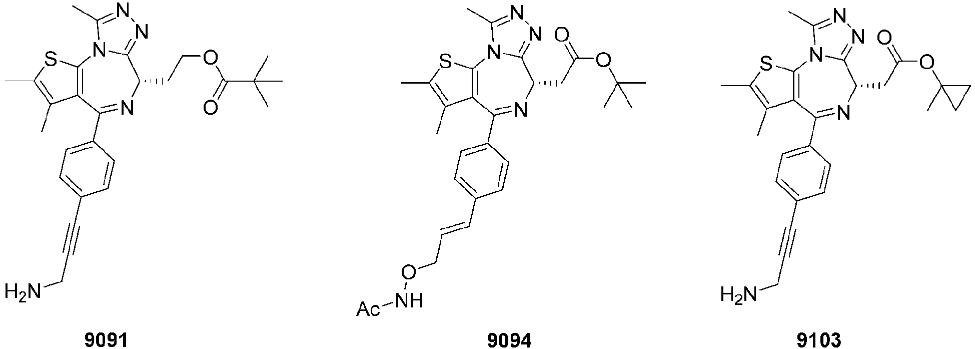

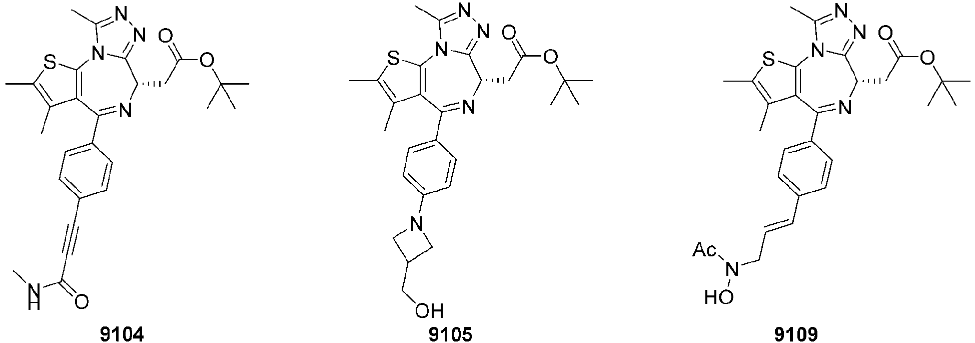

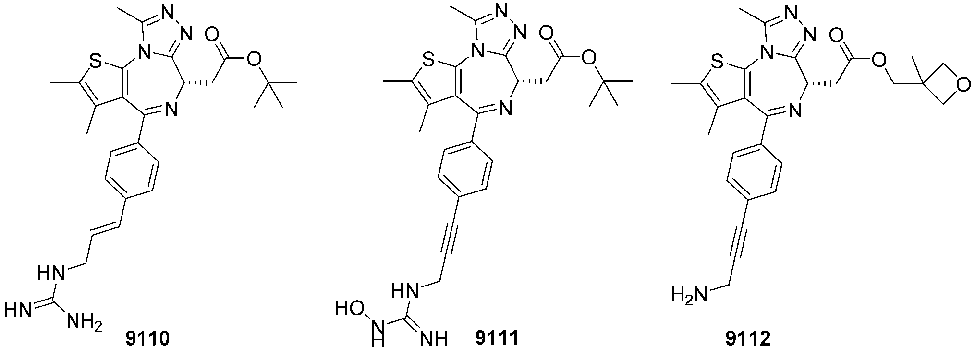

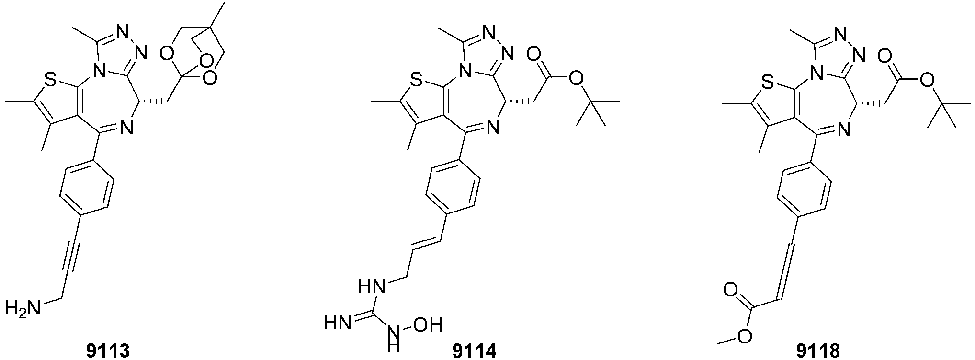

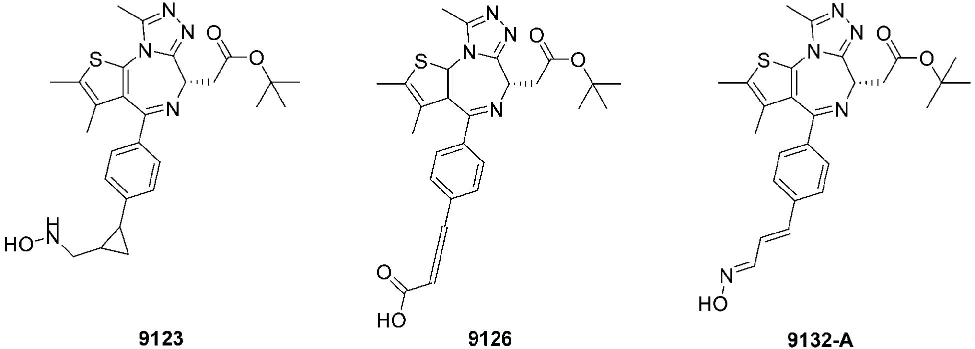

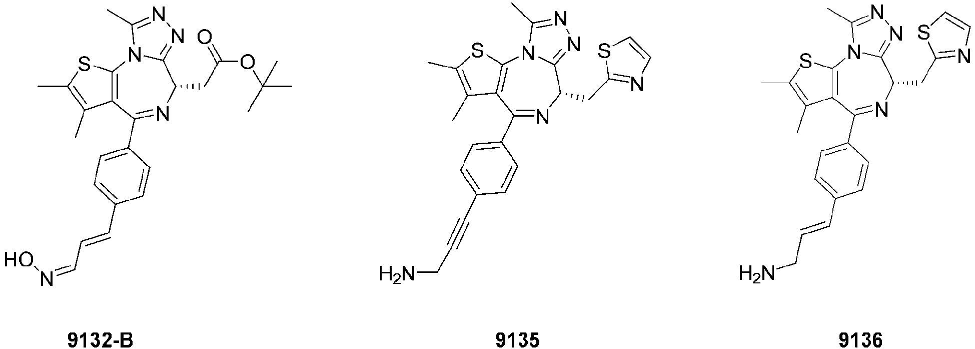

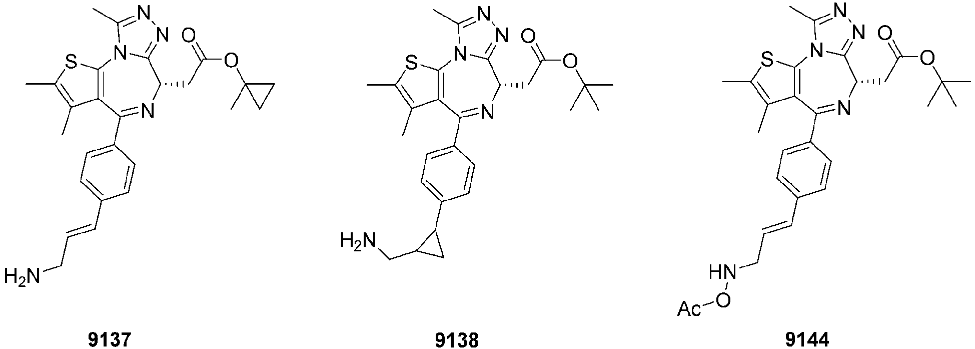

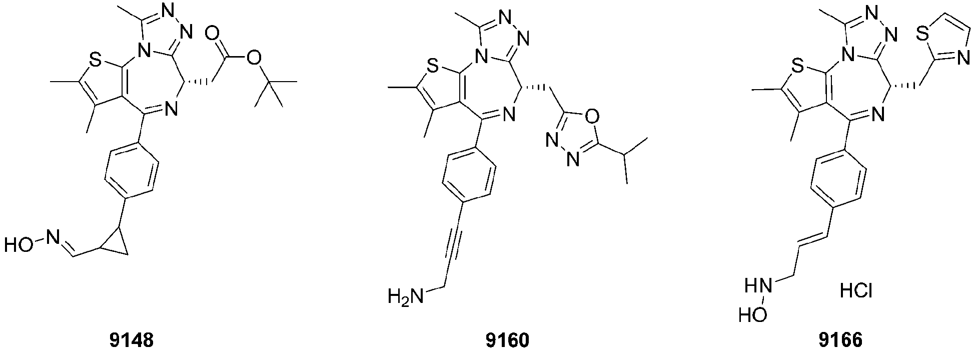

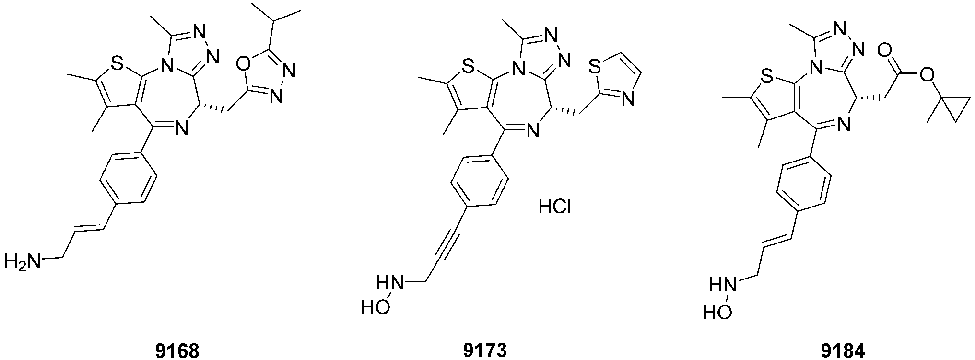

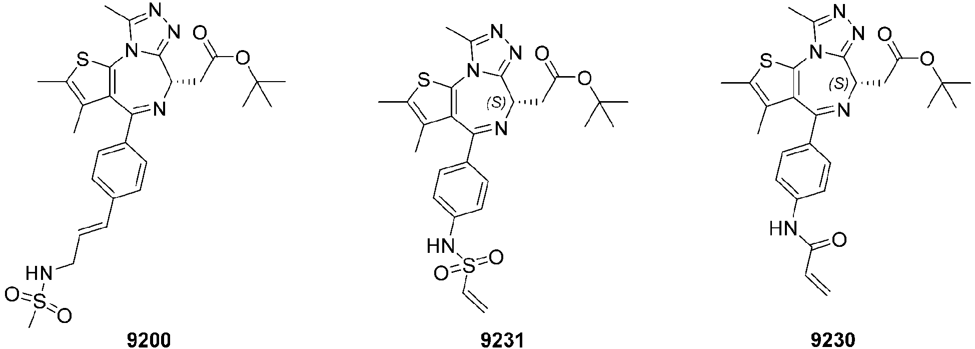

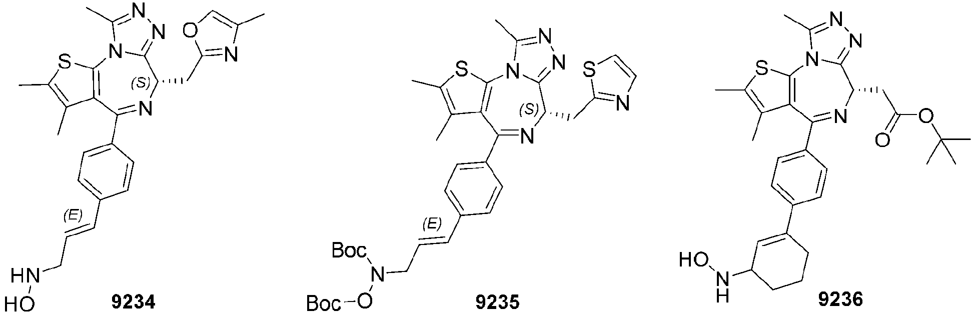

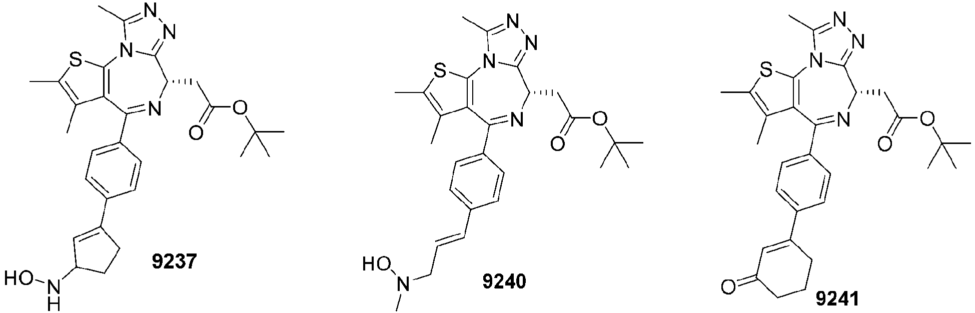

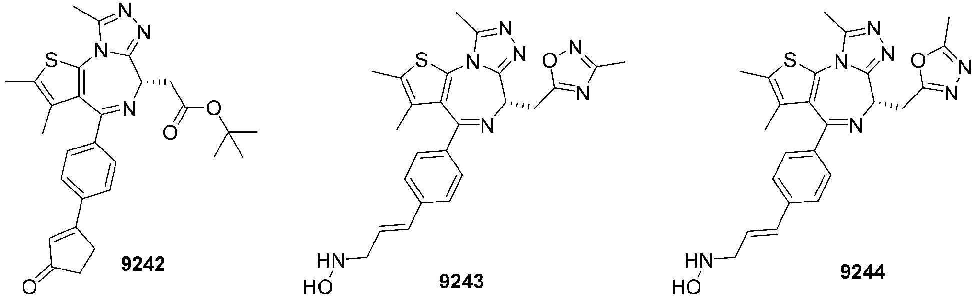

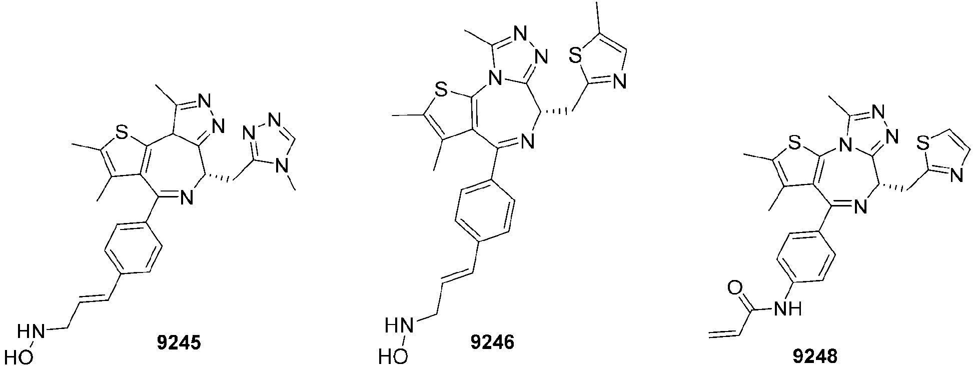

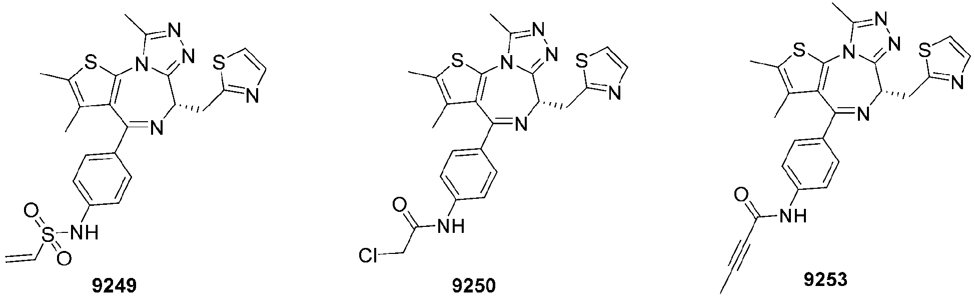

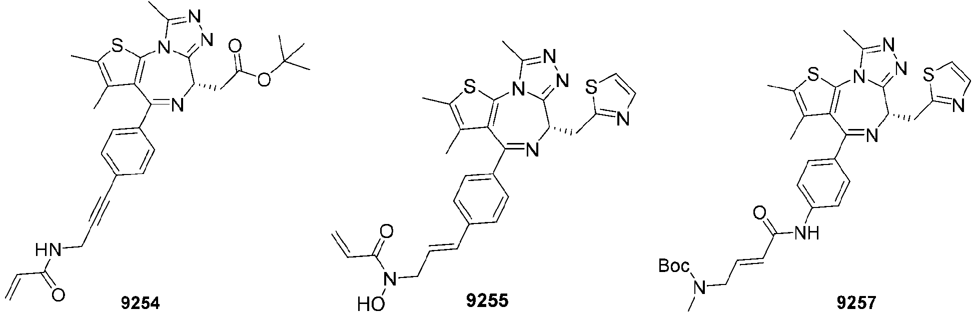

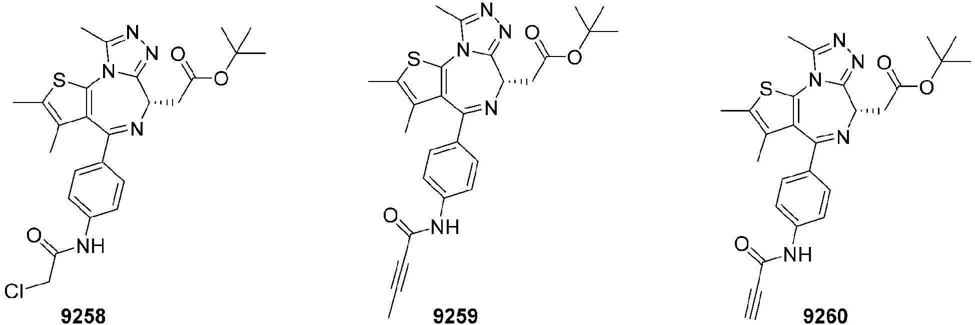

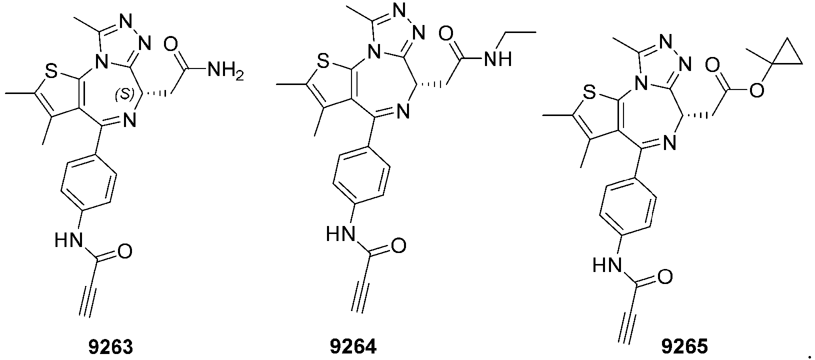

According to an embodiment of the present disclosure, exemplary specific compounds of the compound represented by formula (I) are as follows:

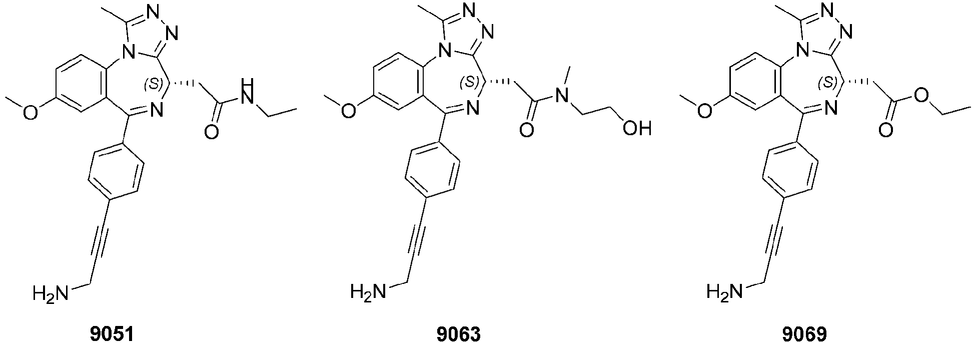

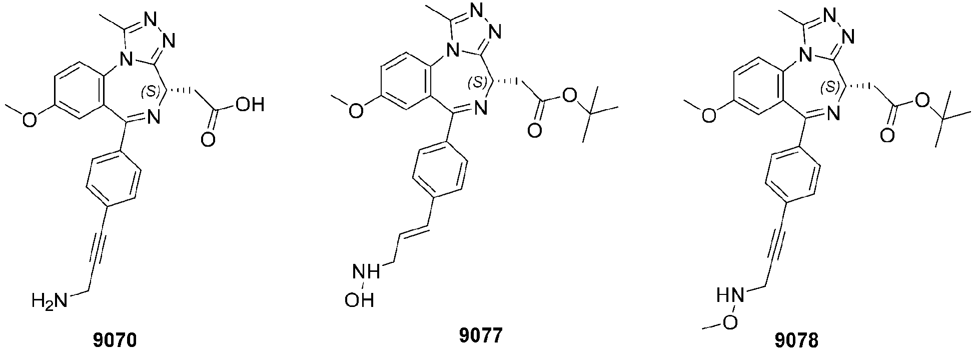

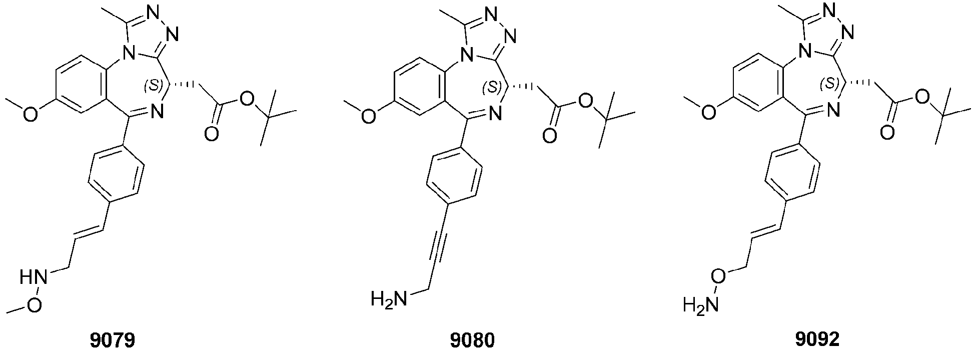

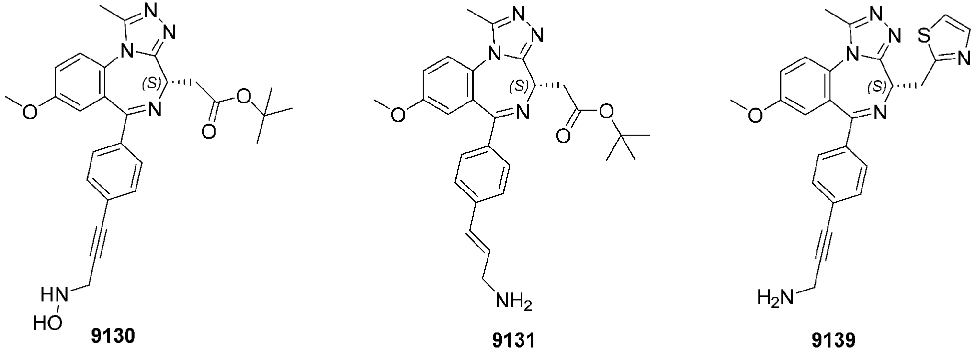

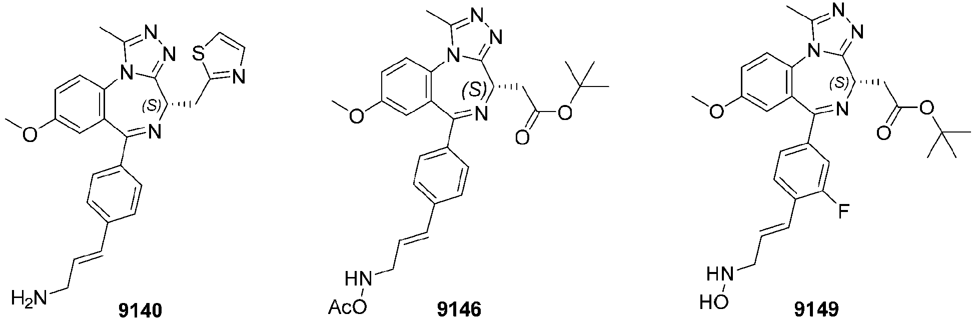

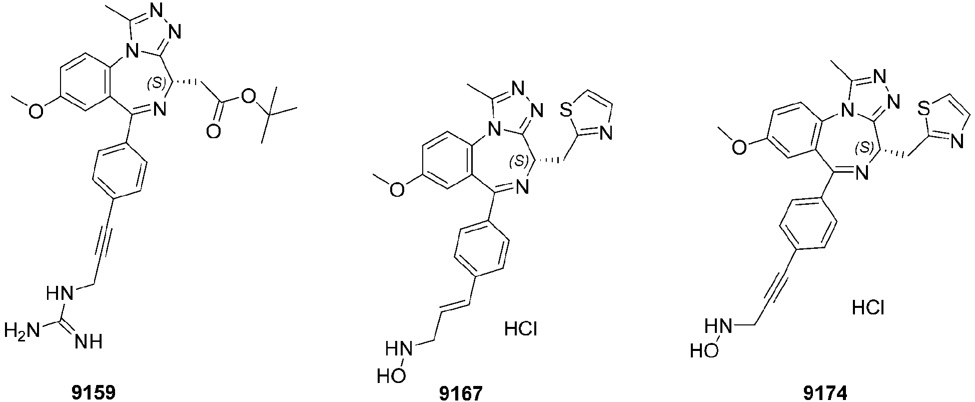

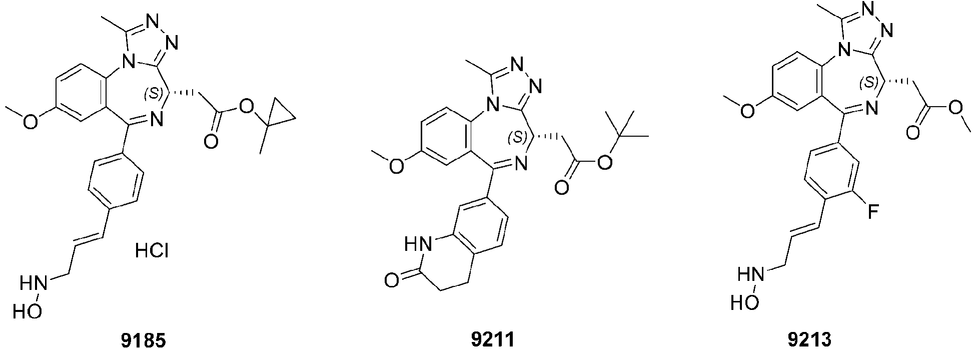

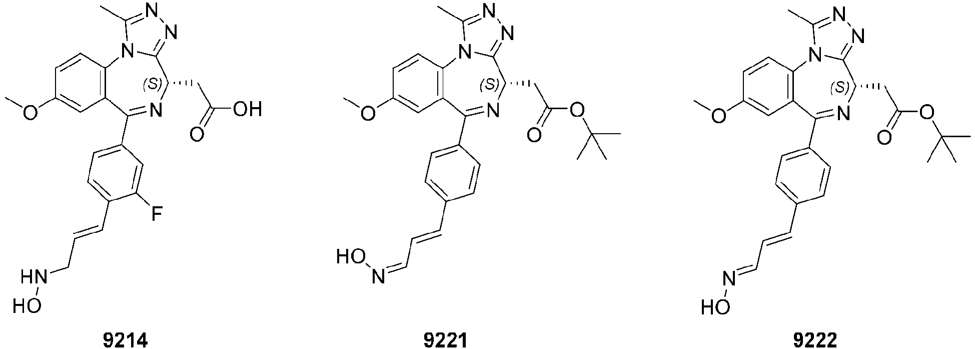

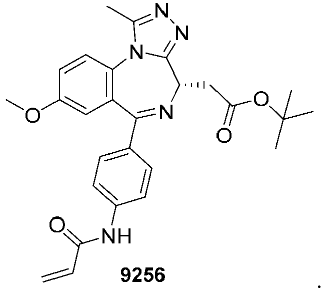

According to an embodiment of the present disclosure, exemplary specific compounds of the compound represented by formula (I) are as follows:

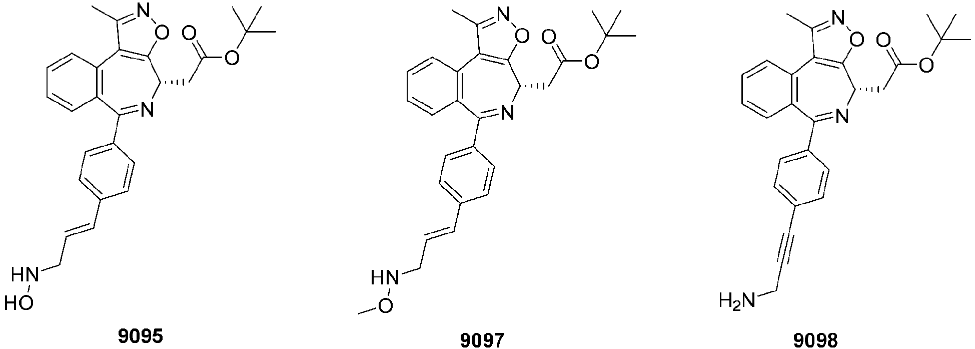

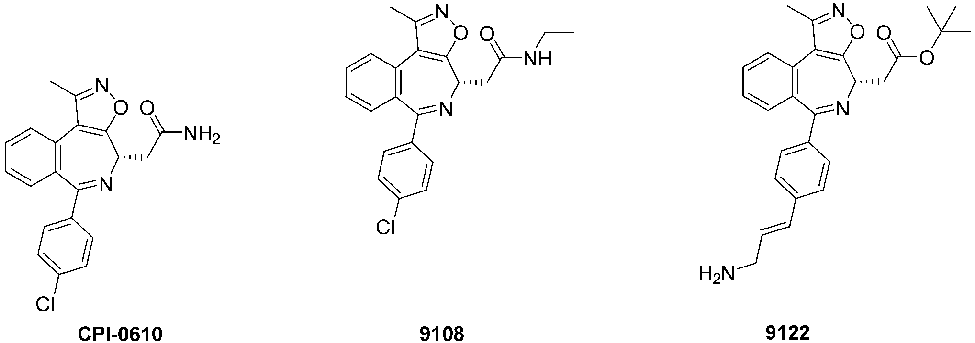

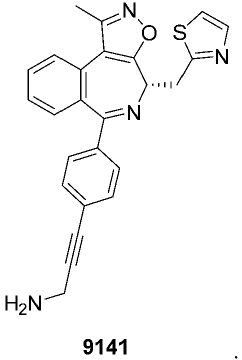

According to an embodiment of the present disclosure, exemplary specific compounds of the compound represented by formula (III) are as follows:

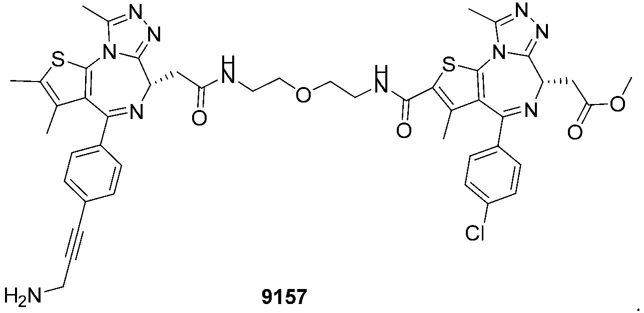

The present disclosure also provides a pharmaceutical composition comprising the compound represented by formula (I) and the racemate, the stereoisomer, the tautomer, the isotopically labeled compound, the nitrogen oxide, the solvate, the polymorph, the metabolite, the ester, the prodrug or the pharmaceutically acceptable salt thereof. According to an embodiment of the present disclosure, the pharmaceutical composition further comprises one or more pharmaceutically acceptable excipients.

According to an embodiment of the present disclosure, the pharmaceutical composition further comprises an additional bioactive agent.

The present disclosure also provides use of the compound represented by formula (I) and the racemate, the stereoisomer, the tautomer, the isotopically labeled compound, the nitrogen oxide, the solvate, the polymorph, the metabolite, the ester, the prodrug or the pharmaceutically acceptable salt thereof, and the pharmaceutical composition comprising the same for manufacturing a medicament for the prevention and/or treatment of a bromodomain-mediated disease or condition.

According to an embodiment of the present disclosure, a protein comprising the bromodomain is bromodomain-containing protein 4 (BRD4).

According to an embodiment of the present disclosure, the disease is cancer.

According to an embodiment of the present disclosure, the cancer is selected from the group consisting of: acoustic neuroma, acute leukemia, acute lymphocytic leukemia, acute myeloid leukemia, acute T cell leukemia, basal cell carcinoma, cholangiocarcinoma, bladder cancer, brain cancer, breast cancer, bronchial carcinoma, cervical cancer, chondrosarcoma, chordoma, choriocarcinoma, chronic leukemia, chronic lymphocytic leukemia, chronic myeloid (granulocytic) leukemia, chronic myelogenous leukemia, colon cancer, colorectal cancer, craniopharyngioma, cystadenocarcinoma, diffuse large B-cell lymphoma, dysplasia, embryonic carcinoma, endometrial cancer, endotheliosarcoma, ependymoma, epithelial cancer, erythroleukemia, esophagus cancer, estrogen receptor-positive breast cancer, essential thrombocythemia, Ewing's tumor, fibrosarcoma, follicular lymphoma, germ cell testicular cancer, glioma, glioblastoma, gliosarcoma, heavy chain disease, hemangioblastoma, hepatoma, hepatocellular carcinoma, hormone-insensitive prostate cancer, leiomyosarcoma, leukemia, liposarcoma, lung cancer, lymphangioendothelial sarcoma, lymphangiosarcoma, lymphoblastic leukemia, lymphomas (Hodgkin's lymphoma and non-Hodgkin's lymphoma), malignancies and hyperproliferative disorders of the bladder, breast, colon, lung, ovary, pancreas, prostate, skin, and uterus, lymphoid malignancies of T-cell or B-cell origin, medullary carcinoma, medulloblastoma, melanoma, meningioma, mesothelioma, multiple myeloma, myelogenous leukemia, myeloma, myxosarcoma, neuroblastoma, NUT midline carcinoma (NMC), non-small cell lung cancer, oligodendroglioma, oral cancer, osteogenic sarcoma, ovarian cancer, pancreatic cancer, papillary adenocarcinoma, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung cancer, solid tumors (carcinoma and sarcoma), small cell lung cancer, gastric cancer, squamous cell carcinoma, synovial tumor, sweat gland carcinoma, thyroid cancer, Waldenström macroglobulinemia, testiculoma, uterine cancer, and Wilms' tumor.

According to an embodiment of the present disclosure, the disease is an autoimmune disease or inflammatory disease selected from the group consisting of: Addison's disease, acute gout, ankylosing spondylitis, asthma, atherosclerosis, Behcet's disease, bullous skin disease, chronic obstructive pulmonary disease (COPD), Crohn's disease, dermatitis, eczema, giant cell arteritis, glomerulonephritis, hepatitis, hypophysitis, inflammatory bowel disease, kawasaki disease, lupus nephritis, multiple sclerosis, myocarditis, myositis, nephritis, organ transplant rejection, osteoarthritis, pancreatitis, pericarditis, polyarteritis nodosa, pneumonia, primary biliary cirrhosis, psoriasis, psoriatic arthritis, rheumatoid arthritis, scleritis, sclerosing cholangitis, sepsis, systemic lupus erythematosus, Takayasu arteritis, toxic shock, thyroiditis, type I diabetes, ulcerative colitis, uveitis, vitiligo, vasculitis, and Wegener's granulomatosis. According to an embodiment of the present disclosure, the disease or condition is selected from the group consisting of: AIDS; chronic kidney disease, diabetic nephropathy, hypertensive nephropathy, HIV-associated nephropathy, glomerulonephritis, lupus nephritis, IgA nephropathy, focal segmental glomerulosclerosis, membranous glomerulonephritis, minimal change nephropathy, polycystic kidney disease, and tubulointerstitial nephritis; acute kidney injury or disease or condition; obesity; dyslipidemia; hypercholesterolemia; Alzheimer's disease; metabolic syndrome; hepatic steatosis; type II diabetes; insulin resistance; and diabetic retinopathy.

The present disclosure also provides a method for degrading a bromodomain-containing protein in a cell, which comprises exposing the cell to an effective amount of the compound represented by formula (I) and the racemate, the stereoisomer, the tautomer, the isotopically labeled compound, the nitrogen oxide, the solvate, the polymorph, the metabolite, the ester, the prodrug or the pharmaceutically acceptable salt thereof, or a pharmaceutical composition comprising the same, wherein the compound achieves degradation of the bromodomain-containing protein.

According to an embodiment of the present disclosure, the bromodomain-containing protein is bromodomain-containing protein 4 (BRD4).

The present disclosure also provides a method for preventing and/or treating a BRD4-mediated disease or condition, which comprises administering to a patient in need thereof a therapeutically effective amount of the compound represented by formula (I) and the racemate, the stereoisomer, the tautomer, the isotopically labeled compound, the nitrogen oxide, the solvate, the polymorph, the metabolite, the ester, the prodrug or the pharmaceutically acceptable salt thereof, or the pharmaceutical composition comprising the same.

The present disclosure also provides the compound represented by formula (I) and the racemate, the stereoisomer, the tautomer, the isotopically labeled compound, the nitrogen oxide, the solvate, the polymorph, the metabolite, the ester, the prodrug or the pharmaceutically acceptable salt thereof, for use in the prevention and/or treatment of a BRD4-mediated disease or condition.

### Beneficial Effects

The present disclosure provides a compound represented by formula (I), which has a relatively good BRD4 protein degradation effect when used as a medicament, and can be used for manufacturing a medicament related to BRD4 inhibition and protein degradation and preventing and/or treating a related disease.

### Definitions and Description

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should be construed as being within the scope of the specification and/or the claims of the present application.

The term "alkyl" refers to a saturated aliphatic hydrocarbon group, which is a linear or branched group containing 1 to 12 (e.g., 1, 2, 3, 4, 5, 6, 7, 8, 9, 10, 11, and 12) carbon atoms, preferably an alkyl group containing 1 to 6 carbon atoms (C₁₋₆ alkyl). Non-limiting examples of the alkyl include methyl, ethyl, *n*-propyl, isopropyl, *n*-butyl, isobutyl, *tert*-butyl, *sec*-butyl*, n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, various branched isomers thereof, and the like. The alkyl may be substituted or unsubstituted. The term "alkoxy" refers to -O-(alkyl), wherein the alkyl is as defined herein. Non-limiting examples of the alkoxy include: methoxy, ethoxy, propoxy, and butoxy. The alkoxy may be substituted or unsubstituted.

The term "alkenyl" should be understood to refer to a linear or branched monovalent hydrocarbyl group containing 2 to 12 (e.g., 2, 3, 4, 5, 6, 7, 8, 9, 10, 11, and 12) carbon atoms, preferably an alkyl group containing 2 to 6 carbon atoms (C₂₋₆ alkenyl). It should be understood that in the case that the alkenyl contains more than one double bond, the double bonds can be separated from one another or conjugated. The alkenyl is, for example, ethenyl, allyl, (*E*)-2-methylethenyl, (*Z*)-2-methylethenyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, or 1-isopropylvinyl.

The term "alkynyl" should be understood to refer to a linear or branched monovalent hydrocarbyl group containing one or more triple bonds and having 2-12 carbon atoms, and is preferably "C₂₋₁₀ alkynyl". The term "C₂₋₁₀ alkynyl" should be understood to preferably refer to a linear or branched monovalent hydrocarbyl group containing one or more triple bonds and having 2, 3, 4, 5, 6, 7, 8, 9, or 10 carbon atoms, for example, having 2, 3, 4, 5, 6, 7, or 8 carbon atoms (i.e., "C₂₋₈ alkynyl"), having 2, 3, 4, 5, or 6 carbon atoms (i.e., "C₂₋₆ alkynyl"), or having 2 or 3 carbon atoms ("C₂₋₃ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl is ethynyl, prop-1-ynyl, or prop-2-ynyl.

The term "cycloalkyl" refers to a saturated or partially unsaturated monocyclic or polycyclic hydrocarbon substituent, and the cycloalkyl ring preferably contains 3 to 12 or 3 to 8 (e.g., 3, 4, 5, 6, 7, and 8) carbon atoms, and more preferably contains 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; polycyclic cycloalkyl groups include spiro, fused and bridged cycloalkyl groups.

The cycloalkyl rings include those in which the cycloalkyl as described herein (including monocyclic, spiro, fused and bridged ones) is fused to an aryl, heteroaryl or heterocycloalkyl ring, and the ring linked to a parent structure is the cycloalkyl. Non-limiting examples include 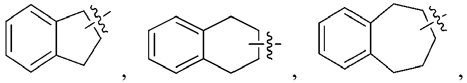 etc., and 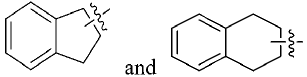 are preferred. The cycloalkyl may be substituted or unsubstituted.

The term "heterocyclyl" refers to a saturated or partially unsaturated monocyclic or polycyclic substituent containing 3 to 14 ring atoms, in which one or more ring atoms are heteroatoms selected from nitrogen, oxygen, and sulfur, wherein the sulfur may be optionally oxidized (that is, to form a sulfoxide or sulfone), but excluding the ring moiety -O-O-, -O-S- or -S-S-, and the remaining ring atoms are carbon atoms. Preferably, the heterocyclyl contains 3 to 12 (e.g., 3, 4, 5, 6, 7, 8, 9, 10, 11 and 12) ring atoms, of which 1 to 4 (e.g., 1, 2, 3 and 4) are heteroatoms; more preferably, the heterocyclyl contains 3 to 8 (e.g., 3, 4, 5, 6, 7 and 8) ring atoms, of which 1 to 3 (e.g., 1, 2 and 3) are heteroatoms; more preferably, the heterocyclyl contains 3 to 6 ring atoms, of which 1 to 3 are heteroatoms; most preferably, the heterocyclyl contains 5 or 6 ring atoms, of which 1 to 3 are heteroatoms. Non-limiting examples of monocyclic heterocyclyl include pyrrolidinyl, tetrahydropyranyl, 1,2,3,6-tetrahydropyridinyl, piperidyl, piperazinyl, morpholinyl, thiomorpholinyl, homopiperazinyl, etc. Polycyclic heterocyclyl includes spiroheterocyclyl, fused heterocyclyl, and bridged heterocyclyl.

The heterocyclyl rings include those in which the heterocyclyl as described herein (including monocyclic, spiro, fused and bridged ones) is fused to an aryl, heteroaryl or cycloalkyl ring, and the ring linked to a parent structure is the heterocyclyl. Non-limiting examples include: 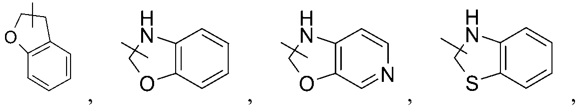 etc. The heterocyclyl may be substituted or unsubstituted.

The term "aryl" refers to a 6- to 14-membered all-carbon monocyclic or fused polycyclic (fused polycyclic rings are rings that share a pair of adjacent carbon atoms) group with a conjugated π-electron system, which is preferably 6-to 10-membered, e.g., phenyl and naphthyl. The aryl rings include those in which the aryl ring as described herein is fused to a heteroaryl, heterocyclyl or cycloalkyl ring, and the ring linked to a parent structure is the aryl ring. Non-limiting examples include: 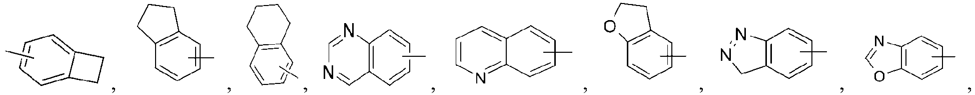 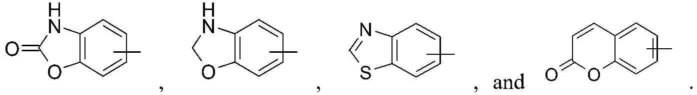 The aryl may be substituted or unsubstituted.

The term "heteroaryl" refers to a heteroaromatic system containing 1 to 4 (e.g., 1, 2, 3 and 4) heteroatoms and 5 to 14 ring atoms, wherein the heteroatoms are selected from oxygen, sulfur and nitrogen. The heteroaryl is preferably 5- to 10-membered (e.g., 5-, 6-, 7-, 8-, 9- or 10-membered), and is more preferably 5- or 6-membered, e.g., furanyl, thienyl, pyridinyl, pyrrolyl, N-alkylpyrrolyl, pyrimidinyl, pyrazinyl, pyridazinyl, imidazolyl, pyrazolyl, triazolyl, tetrazolyl, etc. The heteroaryl ring includes those in which the heteroaryl as described herein is fused to an aryl, heterocyclyl or cycloalkyl ring, and the ring linked to a parent structure is the heteroaryl ring. Non-limiting examples include: 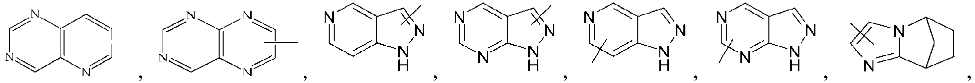 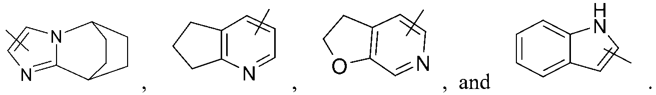 The heteroaryl may be substituted or unsubstituted.

It can be understood by those skilled in the art that the alkyl, alkenyl, alkenyl, cycloalkyl, heterocyclyl, aryl, heteroaryl, etc., include residues derived from removing one hydrogen atom from a parent ring atom, or residues derived from removing two hydrogen atoms from the same ring atom or two different ring atoms of the parent structure, i.e., "alkylene", "alkenylene", "alkynylene", "cycloalkylene", "heterocyclylene", "arylene", and "heteroarylene".

The term "halogen" refers to F, Cl, Br, or I.

"Optional" or "optionally" means that the subsequently described event or circumstance may, but not necessarily, occur, and the description includes instances where the event or circumstance occurs or does not occur. For example, "heterocycloalkyl optionally substituted with alkyl" means that the alkyl may, but not necessarily, be present, and the description includes the case where the heterocycloalkyl is substituted with the alkyl and the case where the heterocycloalkyl is not substituted with the alkyl.

The term "more" includes 3, 4, 5, and more.

The pharmaceutically acceptable salts of the compounds of the present disclosure may be inorganic or organic salts. If these compounds have basic centers, they can form acid addition salts; if these compounds have acidic centers, they can form base addition salts; if these compounds contain both acidic centers (e.g., carboxyl) and basic centers (e.g., amino), they can also form internal salts.

The compounds of the present disclosure may be in the form of a specific geometric isomer or stereoisomer, for example, cis and trans isomers, (-)- and (+)-enantiomers, (R)- and (S)-enantiomers, diastereoisomers, (D)-isomers, (L)-isomers, racemic mixtures and other mixtures, as well as an enantiomer- or diastereoisomer-enriched mixture, all of which are encompassed within the scope of the present disclosure. Substituents such as alkyl may have an additional asymmetric carbon atom. All these isomers and mixtures thereof are encompassed within the scope of the present disclosure.

The compounds and intermediates of the present disclosure may also be present in different tautomeric forms, and all such forms are included within the scope of the present disclosure. "Tautomers" refer to structural isomers of different energies that can interconvert via a low energy barrier. For example, proton tautomers (also referred to as proton transfer tautomers) include interconversion via proton migration, such as keto-enol isomerization, imine-enamine isomerization, and lactam-lactim isomerization. All tautomeric forms of all the compounds of the present disclosure are within the scope of the present disclosure. The name of a compound named in a single way does not exclude any tautomers.

The present disclosure also includes some isotopically-labeled compounds of the present disclosure which are identical to the structures described herein except that one or more atoms are replaced with atoms having atomic masses or mass numbers different from the atomic masses or mass numbers usually found in nature. Examples of isotopes that can be incorporated into the compounds of the present disclosure include isotopes of hydrogen, carbon, nitrogen, oxygen, phosphorus, sulfur, fluorine, iodine and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl. All isotopic variations of the compounds of the present disclosure, whether radioactive or not, are encompassed within the scope of the present disclosure.

Unless otherwise stated, when a position is specifically designated as deuterium (D), that position is understood to be deuterium with the abundance that is at least 1000 times greater than the natural abundance of deuterium (which is 0.015%) (i.e., at least 10% deuterium incorporation). In the exemplary compounds, the deuterium with the abundance that is greater than the natural abundance of deuterium may be deuterium with at least 1000 times greater abundance, deuterium with at least 2000 times greater abundance, deuterium with at least 3000 times greater abundance, deuterium with at least 4000 times greater abundance, deuterium with at least 5000 times greater abundance, deuterium with at least 6000 times greater abundance, or deuterium with higher abundance. Each available hydrogen atom linked to carbon atoms may be independently replaced with a deuterium atom. Those skilled in the art are able to synthesize the compounds in deuterated form by referring to the relevant literature. Commercially available deuterated starting materials can be used in preparing the compounds in deuterated form, or they can be synthesized using conventional techniques using deuterated reagents including, but not limited to, deuterated boranes, tri-deuterated boranes in tetrahydrofuran, deuterated lithium aluminum hydrides, deuterated iodoethanes, deuterated iodomethanes, and the like.

As used herein, "prodrug" refers to those which can be converted into biologically active compounds of the present disclosure under physiological conditions or through solvent hydrolysis. For example, the prodrug of the present disclosure can be prepared by modifying functional groups in the parent compound, and the modification can be carried out through conventional operations or removed in vivo to obtain the parent compound. For example, the prodrug of the present invention includes but not limited to a drug conjugate (such as antibody-drug conjugate) , a nanoparticle and a hydrosol which are prepared by using the compound of the present invention as a payload.

As used herein, "therapeutically effective amount" refers to an amount of the active compound or drug that causes a biological or medical response that researchers, veterinarians, physicians, other clinicians, etc., are looking for in tissues, systems, animals, individuals or humans, including one or more of the following effects: (1) disease prevention: for example, the prevention of a disease, disorder or condition in an individual who is susceptible to the disease, disorder or condition but has not yet experienced or exhibited the pathology or symptoms of the disease; (2) disease inhibition: for example, the inhibition of a disease, disorder or condition in an individual who is experiencing or exhibiting the pathology or symptoms of the disease, disorder or condition.(i.e., the prevention of the further development of the pathology and/or symptoms); and (3) disease alleviation: for example, the alleviation of a disease, disorder or condition in an individual who is experiencing or exhibiting the pathology or symptoms of the disease, disorder or condition (i.e., the reverse of the pathology and/or symptoms). In the context of a drug or pharmacologically active agent, "therapeutically effective amount" refers to an amount of the drug or medicament that is sufficient to achieve the desired effects without producing toxic effects. The effective amount varies from person to person, depending on the age and general condition of the subject and also on the particular active substance. The suitable effective amount in a case can be determined by those skilled in the art through routine tests.

### DETAILED DESCRIPTION

The embodiments of the present disclosure will be further illustrated in detail with reference to the following specific examples. It should be understood that the following examples are merely exemplary illustrations and explanations of the present disclosure, and should not be construed as limiting the protection scope of the present disclosure. All techniques implemented based on the content of the present disclosure described above are included within the protection scope of the present disclosure.

Unless otherwise stated, the starting materials and reagents used in the following examples are all commercially available products or can be prepared using known methods.

### Example 1-1

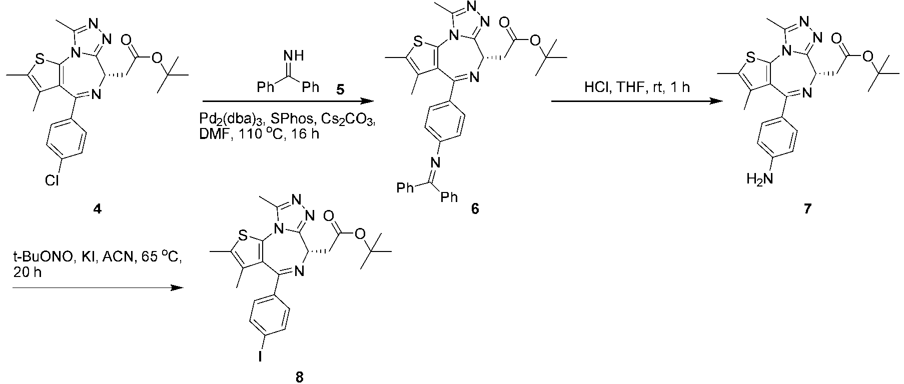

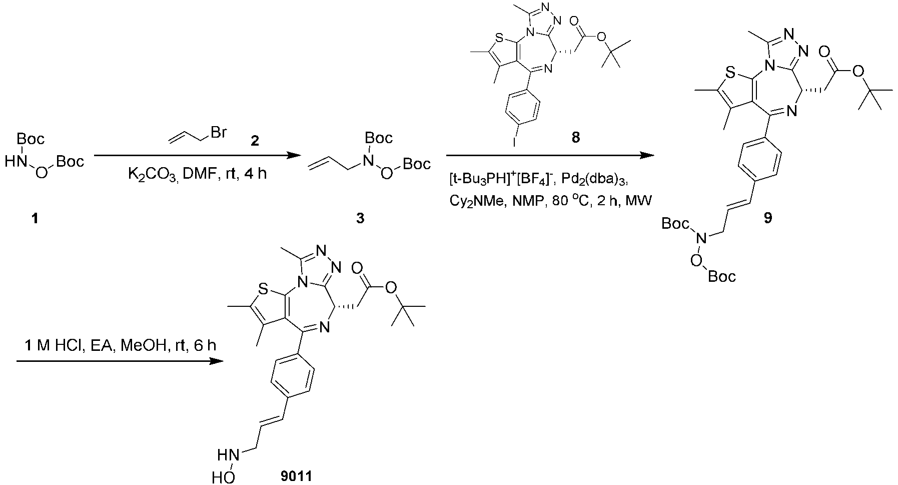

Compound 4 (1.00 g, 0.44 mmol), compound 5 (0.60 g, 0.66 mmol), Pd₂(dba)₃ (0.20 g, 0.04 mmol), SPhos (0.09 g, 0.04 mmol), and Cs₂CO₃ (2.10 g, 1.31 mmol) were mixed in toluene (10 mL). The mixture was stirred at 110 °C for 16 h under N₂ atmosphere. The solvent was removed under vacuum. The residue was purified by flash column chromatography (PE/EA = 1/1) to give compound 6 (1.3 g, 98.5%) as a yellow oil.

Compound 6 (1.30 g, 2.16 mmol) and HCl (26 mL, 1 M in THF) were mixed in THF (50 mL). The mixture was stirred at room temperature for 1 h. The solvent was removed under vacuum and the residue was diluted with water (30 mL). The solution was adjusted to pH 7 with a saturated NaHCO₃ solution and extracted with EtOAc (30 mL × 4). The combined organic layer was dried over Na₂SO₄, filtered, concentrated, and purified by chromatography (MeOH/ DCM = 5%) to give compound 7 (770.0 mg, 81.6%) as a yellow solid.

To a solution of t-BuONO (75 mg, 0.74 mmol) in CH₃CN (10 mL) was added KI (76 mg, 0.46 mmol) at room temperature. The mixture was stirred and heated at 65 °C for 2 h. Then, a solution of compound 7 (100 mg, 0.23 mmol) in CH₃CN (10 mL) was added. The mixture was stirred at 65 °C for 18 h. The reaction was quenched with water (15 mL). The mixture was extracted with EtOAc (20 mL × 3). The organic layer was washed with brine (10 mL × 2), dried over Na₂SO₄, concentrated, and purified by FCC (MeOH/DCM = 5%) to give compound 8 (103 mg, 82%) as a yellow solid.

To a solution of compound 1 (1.4 g, 0.006 mol) and compound 2 (1.31 g, 0.011 mol) in DMF (10 mL) was added K₂CO₃ (1.08 g, 0.008 mol). The mixture was stirred at room temperature for 4 h. The solution was diluted with water. The aqueous phase was extracted with ethyl acetate. The organic layer was washed with brine, dried over MgSO₄, and concentrated under reduced pressure to give a residue. The residue was purified by FCC (PE/EtOAc = 95/5) to give compound 3 (1 g, 55% yield) as a colorless oil.

Compound 3 (90 mg, 0.164 mmol), compound 8 (90 mg, 0.328 mmol), Cy₂NMe (46 mg, 0.131 mmol), tri-*tert-*butylphosphine tetrafluoroborate (116 mg, 0.492 mmol), and Pd₂(dba)₃ (36 mg, 0.025 mmol) were dissolved in NMP (3 mL) under microwave irradiation, and the solution was stirred at 80 °C for 2 h. The solution was filtered and the filtrate was collected. The solvent was removed under reduced pressure. The residue was extracted with EA (30 mL). The crude product was purified by FCC (DCM/MeOH = 20/1) to give compound 9 (30 mg, 13.2% yield) as a yellow solid.

Compound 9 (30 mg, 0.043 mmol) was dissolved in a mixed solution of HCl in EA/MeOH (1 mL/1 mL, 1 N). The mixture was stirred at room temperature for 6 h. The solvent was removed under reduced pressure. The crude product was purified by prep-HPLC (column: Phenomenex luna C18 250 mm × 100 mm × 10 µm; mobile phase: [H₂O (0.1% FA)-ACN]; B%: 10%-55%, 40 min) to give 9011 (10 mg, 43.16% yield) as a yellow solid.
**LCMS:** [M+H] ⁺ = 494.2;
**¹H NMR:** (400 MHz, d₄-MeOD) δ 7.458 (d, J = 8.4 Hz, 2H), 7.404 (d, J = 8.4 Hz, 2H), 6.641 (d, J = 16.0 Hz, 1H), 6.433 (dt, J = 15.8, 6.4 Hz, 1H), 4.542 (m, 1H), 3.628 (d, J = 6.4 Hz, 2H), 3.412 (m, 2H), 2.698 (s, 3H), 2.455 (s, 3H), 1.704 (s, 3H), 1.496 (s, 9H).
**HPLC:** 95.15% purity;
**SFC:** Rt = 13.319 min.

### Example 1-2

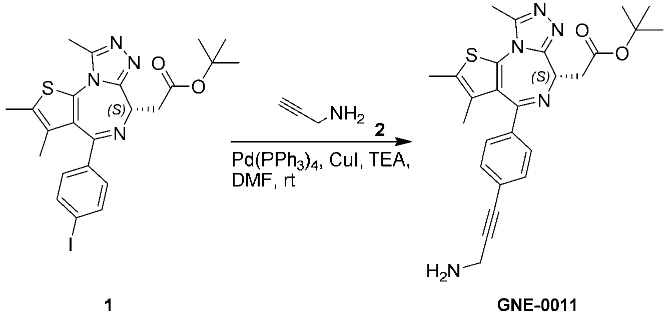

To a solution of compound 1 (500 mg, 0.912 mmol) in DMF (7 mL) were added compound 2 (75 mg, 1.367 mmol), CuI (17 mg, 0.091 mmol), Pd(PPh₃)₄ (105 mg, 0.09 mmol), and TEA (277 mg, 2.735 mmol). The resulting mixture was stirred at room temperature for 2 h under N₂ atmosphere. The mixture was filtered and concentrated under reduced pressure to give a residue. The crude product was purified directly by prep-HPLC (column: Phenomenex moon: C18 250 mm × 100 mm × 10 µm; mobile phase: [ACN-H₂O (0.1% FA)]; B%: 5%-45%, 40 min) to give GNE-0011 (280 mg, 61.3% yield) as a white solid.
**LCMS:** [M+H] ⁺ = 476.1;
**¹H NMR:** (400 MHz, MeOD) δ 7.469 - 7.417 (m, 4H), 4.555 (dd, J = 8.2, 6.2 Hz, 1H), 3.722 (s, 2H), 3.421 - 3.301 (m, 2H), 2.693 (s, 3H), 2.453 (s, 3H), 1.694 (s, 3H), 1.491 (s, 9H).
**HPLC:** 99.29% purity;
**SFC:** Rt = 2.395 min.

### Example 1-3

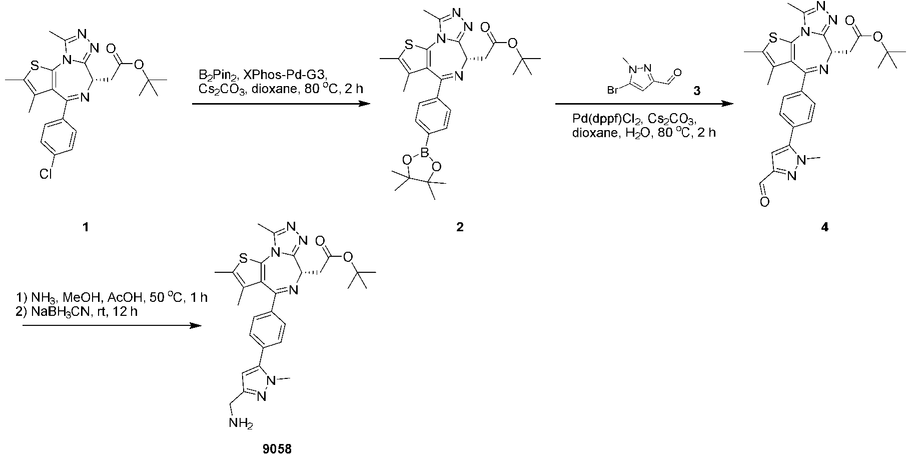

Compound 1 (3 g, 0.007 mol), B₂PiN₂ (3.35 g, 0.013 mol), Cs₂CO₃ (6.45 g, 0.020 mol), and XPhos-Pd-G3 (1.12 g, 0.001 mol) were dissolved in 1,4-dioxane (20 mL), and the solution was heated at 80 °C for 2 h under N₂ atmosphere. The solvent was removed under reduced pressure to give a residue. The residue was diluted with water (100 mL), extracted with DCM (10 mL × 3), washed with brine (100 mL × 3), dried over Na₂SO₄, filtered, and concentrated. Then, the crude product was purified by flash chromatography (MeOH/DCM = 7%) to give compound 2 (3.43 g, 5.6 mmol, 85% yield) as a yellow solid.

A solution of compound 2 (116 mg, 0.212 mmol), compound 3 (40 mg, 0.212 mmol), Cs₂CO₃ (138 mg, 0.423 mmol), and Pd(dppf)Cl₂ (16 mg, 0.021 mmol) in 1.4-dioxane/H₂O =5:1 (1 mL) was stirred at 80 °C for 2 h under N₂ atmosphere. The mixture was diluted with water (10 mL) and extracted with EA (100 mL × 3). The organic layer was washed with a saturated aqueous sodium chloride solution (50 mL), dried over Na₂SO₄, and concentrated under reduced pressure. The residue was purified by FCC (DCM/MeOH = 20/1) to give compound 4 (60 mg, 50.8% yield) as a yellow oil.

A solution of compound 4 (35 mg, 0.066 mmol), AcOH (0.2 mL), and NH₃·H₂O (5.62 mg, 0.330 mmol) in MeOH (2 mL) was stirred at 50 °C for 1 h. Then, NaBH₃CN (21 mg, 0.330 mmol) was added, and the resulting mixture was stirred at room temperature for 12 h. The reaction mixture was concentrated under reduced pressure. The crude product was purified by prep-HPLC (column: Phenomenex luna: C18 250 mm × 100 mm × 10 µm; mobile phase: [H₂O (0.1% FA)-ACN]; B%: 20%-60%, 40 min) to give 9058 (8 mg, 0.015 mmol, 22.3% yield) as a white solid.
**LCMS:** [M+H] ⁺ = 532.3.
**¹H NMR:** (400 MHz, MeOD) δ 8.541 (s, 0.6H), 7.597 (d, J = 8.2 Hz, 2H), 7.551 (d, J = 8.4 Hz, 2H), 6.472 (s, 1H), 4.595 (d, J = 8.4 Hz, 1H), 4.027 (s, 2H), 3.878 (s, 3H), 3.447 (t, J = 7.2 Hz, 2H), 2.709 (s, 3H), 2.470 (s, 3H), 1.747 (s, 3H), 1.504 (s, 9H).
**HPLC:** 98.95% purity.
**SFC:** Rt = 3.023 min.

### Example 1-4

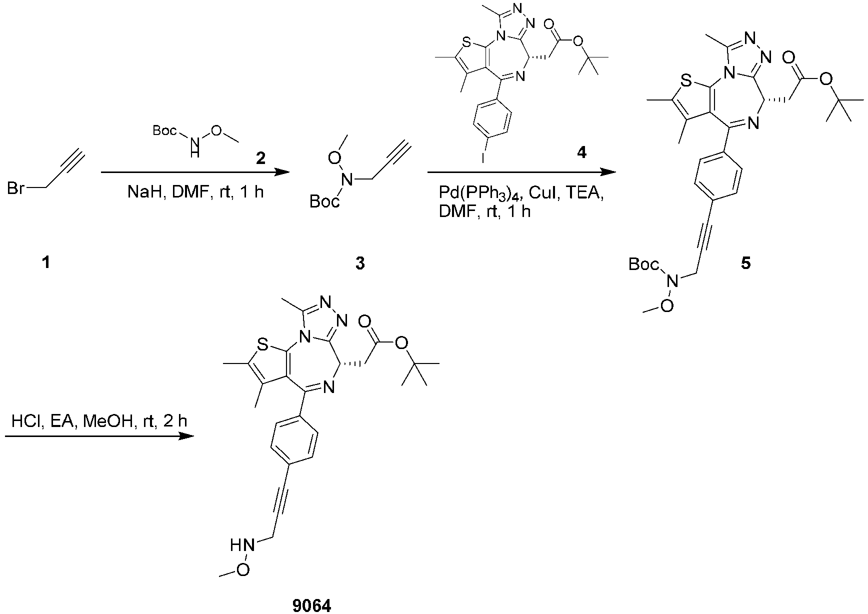

To a solution of compound 2 (500 mg, 3.397 mmol) in DMF (5 mL) was added NaH (150 mg, 3.737 mmol) at 0 °C under N₂ atmosphere. The mixture was stirred at 0 °C for 0.5 h. Then, compound 1 (444 mg, 3.737 mmol) was added dropwise. The mixture was stirred at room temperature for 1 h. Then, the mixture was diluted with water (30 mL), extracted with EtOAc (30 mL × 2), washed with brine (50 mL × 5), dried over Na₂SO₄, and concentrated under reduced pressure to give compound 3 (550 mg, crude) as a colorless oil, which was used in next step without further purification.

A solution of compound 4 (100 mg, 0.182 mmol), compound 3 (68 mg, 0.365 mmol), CuI (10 mg, 0.055 mmol), Pd(PPh₃)₄ (21 mg, 0.018 mmol), and TEA (55 mg, 0.547 mmol) in DMF (2 mL) was stirred at room temperature for 1 h. The solution was diluted with water (10 mL) and extracted with EA (10 mL × 3). The organic layer was washed with brine (10 mL × 5), dried over Na₂SO₄, and concentrated. The crude product was purified by FCC (eluent: MeOH in DCM = 0-10%) to give compound 5 (60 mg, 0.079 mmol, 43.39% yield) as a yellow solid.

To a solution of compound 5 (60 mg, 0.082 mmol) in MeOH (1 mL) was added a 2 N HCl/EA solution (1 mL). The mixture was stirred at room temperature for 2 h. Then, the solvent was removed by N₂. The residue was purified by prep-HPLC (Column: Phenomenex luna C18 250 mm × 100 mm × 10 µm; mobile phase: [water (0.05% FA)-ACN]; B%: 5%-50%, 30 min) to give 9064 (13.94 mg, 0.028 mmol, 9.2% yield, 96.08% purity) as a white solid.
**LCMS:** [M+H] ⁺ = 506.3;
**¹H NMR:** (400 MHz, CD₃OD) δ 7.434 (dd, J = 8.4 Hz, 4.0 Hz, 4H), 4.570 - 4.535 (m, 1H), 3.833 (s, 2H), 3.574 (s, 3H), 3.441 - 3.408 (m, 2H), 2.693 (s, 3H), 2.452 (s, 3H), 1.696 (s, 3H), 1.491 (s, 9H).
**HPLC:** 96.08% purity;
**SFC:** Rt = 5.559 min.

### Example 1-5

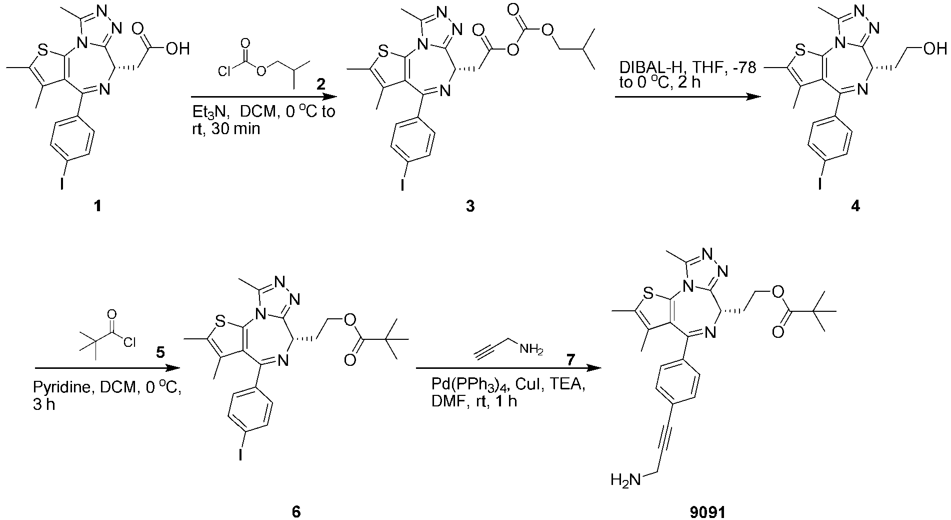

To a solution of compound 1 (150 mg, 0.305 mmol) in DCM (5 mL) was added triethylamine (46 mg, 0.457 mmol) at 0 °C, followed by the dropwise addition of compound 2 (46 mg, 0.335 mmol). The resulting solution was heated to room temperature and stirred for 30 min. The mixture was diluted with DCM (20 mL), washed with brine (20 mL), dried over anhydrous Na₂SO₄, and concentrated under vacuum to give compound 3 (160 mg, crude) as a yellow oil. To a solution of compound 3 (160 mg, 0.270 mmol) in THF (3 mL) was added DIBAL-H (0.81 mmol) dropwise at - 78 °C under N₂ atmosphere. The reaction mixture was stirred at -78 °C for 0.5 h, and then heated to 0°C and stirred for 1.5 h. A saturated aqueous MeOH solution (3 mL) was added. The solvent was removed under reduced pressure. The residue was diluted with water (10 mL) and extracted with EA (10 mL × 3). The organic layer was dried over Na₂SO₄ and concentrated under reduced pressure. The residue was purified by FCC (DCM/MeOH = 10/1) to give compound 4 (120 mg, 65.0% yield) as a yellow oil.

To a solution of compound 4 (120 mg, 0.251 mmol) in DCM (5 mL) was added pyridine (30 mg, 0.376 mmol) and compound 5 (45 mg, 0.376 mmol) dropwise at 0 °C under N₂ atmosphere. The reaction mixture was stirred at 0 °C for 3 h and quenched with H₂O (10 mL). The solution was extracted with DCM (10 mL × 2). The organic layer was dried over Na₂SO₄ and concentrated under reduced pressure. The residue was purified by FCC (DCM/MeOH = 20:1) to give compound 6 (70 mg, 44.6% yield) as a yellow oil.

A solution of compound 6 (70 mg, 0.125 mmol), compound 7 (21 mg, 0.374 mmol), Pd(PPh₃)₄ (29 mg, 0.025 mmol), TEA (38 mg, 0.374 mmol), and CuI (5 mg, 0.025 mmol) in DMF (1 mL) was stirred at room temperature for 1 h under N₂ atmosphere. The mixture was filtered and the filtrate was collected. The crude was purified by prep-HPLC (column: Phenomenex luna C18 250 mm × 100 mm × 10 µm; mobile phase: [H₂O (0.1% FA)-ACN]; B%: 5%-40%, 40 min) to give 9091 (9 mg, 14.1% yield) as a yellow solid.
**LCMS:** [M+H] ⁺ = 490.2;
**¹H NMR:** (400 MHz, MeOD) δ 7.490 (s, 4H), 4.692 - 4.623 (m, 1H), 4.432 - 4.365 (m, 1H), 4.192 (dd, J = 9.8, 4.8 Hz, 1H), 3.862 (s, 2H), 2.893 - 2.726 (m, 2H), 2.693 (s, 3H), 2.437 (s, 3H), 1.670 (s, 3H), 1.088 (s, 9H).
**HPLC:** 99.2% purity;
**SFC:** Rt = 2.755 min.

### Example 1-6

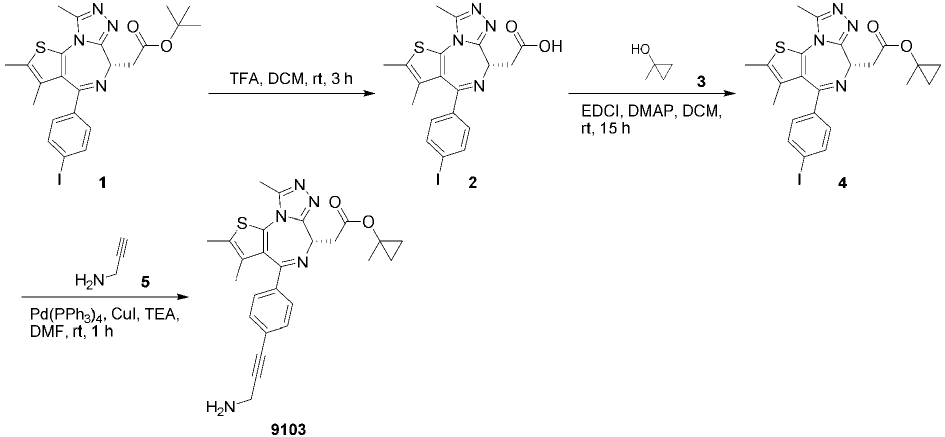

Compound 1 (300 mg, 0.547 mmol) was dissolved in DCM/TFA (2:1, 3 mL), and the solution was stirred at room temperature for 3 h under N₂ atmosphere. The mixture was diluted with water (10 mL) and extracted with DCM (10 mL × 3). The organic layer was collected, dried over Na₂SO₄, and concentrated to give compound 2 (250 mg, 0.46 mmol, 83.55% yield) as a brown solid.

A solution of compound 2 (300 mg, 0.609 mmol), EDCI (155 mg, 0.810 mmol), and DMAP (6 mg, 0.049 mmol) in DCM (3 mL) was stirred at room temperature under N₂ atmosphere. After 10 min, compound 3 (51 mg, 0.701 mmol) was added dropwise. The reaction mixture was stirred at room temperature for 15 h. The mixture was diluted with water (10 mL) and extracted with DCM (10 mL × 3). The organic layer was collected, dried over Na₂SO₄, and concentrated. Then, the crude product was purified by flash chromatography (PE/EA = 70:30) to give compound 4 (100 mg, 0.16 mmol, 27% yield) as a yellow solid.

A solution of compound 4 (90 mg, 0.165 mmol), compound 5 (11 mg, 0.198 mmol), Pd(PPh₃)₄ (38 mg, 0.033 mmol), CuI (6 mg, 0.032 mmol), and TEA (50 mg, 0.494 mmol) in DMF (1 mL) was stirred at room temperature for 1 h under N₂ atmosphere. The mixture was diluted with MeOH, filtered, and purified by prep-HPLC (column: Phenomenex moon C18 250 mm × 100 mm × 10 µm; mobile phase: [H₂O (0.1% FA)-ACN]; B%: 20%-60%, 30 min) to give 9103 (15 mg, 0.03 mmol, 18.3% yield) as a white solid.
**LCMS:** [M+H] ⁺ = 474.2;
**¹H NMR:** (400 MHz, CDCl₃) δ 7.391 (t, J = 10.2 Hz, 4H), 4.584 (t, J = 7.2 Hz, 1H), 3.939 (s, 2H), 3.550 (d, J = 6.8 Hz, 2H), 2.665 (s, 3H), 2.400 (s, 3H), 1.654 (s, 3H), 1.559 (s, 3H), 0.914 (s, 2H), 0.656 (s, 2H).
**HPLC:** 99.55% purity;
**SFC:** Rt = 7.007 min.

### Example 1-7

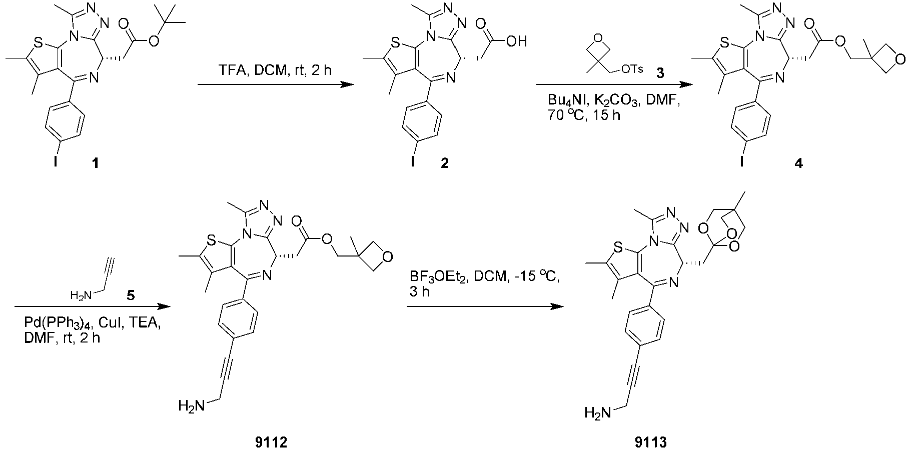

A solution of compound 1 (250 mg, 0.456 mmol) in DCM/TFA (2:1, 3 mL) was stirred at room temperature for 2 h under N₂ atmosphere. The mixture was diluted with water (10 mL) and extracted with DCM (10 mL × 3). The organic layer was collected, dried over Na₂SO₄, and concentrated to give compound 2 (200 mg, crude) as a yellow oil.

A solution of compound 2 (200 mg, 0.406 mmol), compound 3 (51 mg, 0.701 mmol), Bu₄NI (15 mg, 0.041 mmol), and K₂CO₃ (112 mg, 0.812 mmol) in DMF (3 mL) was stirred at 70 °C for 15 h under N₂ atmosphere. The mixture was diluted with water (10 mL) and extracted with EA (10 mL × 3). The organic layer was collected, dried over Na₂SO₄, and concentrated. Then, the crude product was purified by flash chromatography (DCM/MeOH = 10:1) to give compound 4 (100 mg, 0.156 mmol, 38.4% yield) as a yellow oil.

A solution of compound 4 (90 mg, 0.156 mmol), compound 5 (17 mg, 0.312 mmol), Pd(PPh₃)₄ (36 mg, 0.031 mmol), CuI (6 mg, 0.031 mmol), and TEA (50 mg, 0.46 mmol) in DMF (1 mL) was stirred at room temperature for 2 h under N₂ atmosphere. The mixture was diluted with MeOH (3 mL) and purified by prep-HPLC (column: Phenomenex moon C18 250 mm × 100 mm × 10 µm; mobile phase: [H₂O (0.1% FA)-ACN]; B%: 10%-50%, 40 min) to give 9112 (15 mg, 14.6% yield) as a yellow solid.
**LCMS:** [M+H] ⁺= 504.1;
**¹H NMR:** (400 MHz, MeOD) δ 8.525 (s, 0.8H), 7.520 - 7.438 (m, 4H), 4.675 (t, J = 7.2 Hz, 1H), 4.502 (dd, J = 14.4, 6.0 Hz, 2H), 4.345 (dd, J = 6.0, 2.0 Hz, 2H), 4.249 (s, 2H), 3.915 (s, 2H), 3.591 (d, J = 7.4 Hz, 2H), 2.691 (s, 3H), 2.450 (s, 3H), 1.685 (s, 3H), 1.323 (s, 3H).
**HPLC:** 98.7% purity;
**SFC:** Rt = 5.801 min.

To a solution of 9112 (50 mg, 0.099 mmol) in anhydrous DCM (2 mL) was added a solution of BF₃·Et₂O (0.1 mL, 46.5%) in anhydrous DCM (1 mL) dropwise at -10 °C under N₂ atmosphere. The reaction mixture was stirred at - 10°C for 3 h. Then, the reaction was quenched with trimethylamine. The mixture was concentrated, dissolved with MeOH, and purified by prep-HPLC (column: Phenomenex luna C18 250 mm × 100 mm × 10 µm; mobile phase: [H₂O (0.1% TFA)-ACN]; B%: 5%-40%, 40 min) to give 9113 as a TFA salt. Then, HCl (2 drops, 1 M) was added to the solution to give M019113 (15 mg, 28.5% yield) as a yellow solid.
**LCMS:** [M+H] ⁺= 504.1;
**¹H NMR:** (400 MHz, MeOD) δ 7.565 (s, 4H), 4.865 (d, J = 6.4 Hz, 1H), 4.171 - 4.052 (m, 4H), 3.653 - 3.545 (m, 2H), 3.492 - 3.413 (m, 4H), 2.930 (s, 3H), 2.507 (s, 3H), 1.726 (s, 3H), 0.911 (s, 3H).
**HPLC:** 95.1% purity;
**SFC:** Rt = 3.066 min.

### Example 1-8

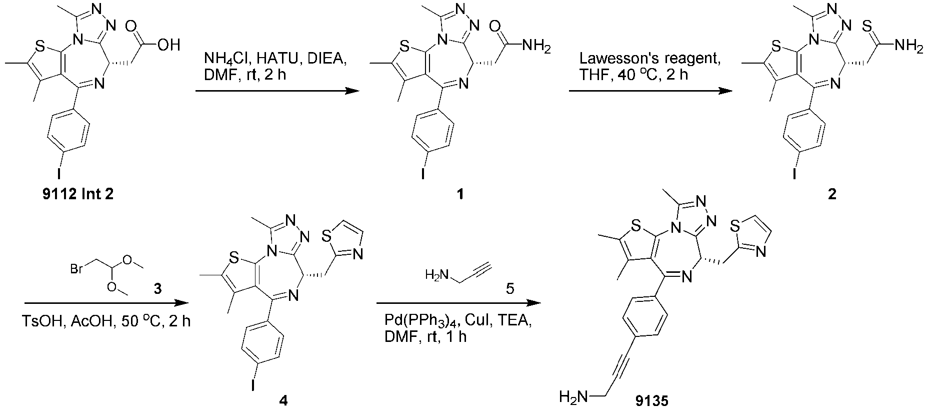

A solution of 9112 Int 2 (400 mg, 0.813 mmol), NH₄Cl (130 mg, 2.438 mmol), HATU (618 mg, 1.625 mmol), and DIEA (525 mg, 4.063 mmol) in DMF (10 mL) was stirred at room temperature for 2 h. The mixture was diluted with water (10 mL) and extracted with EA (10 mL × 3). The combined organic layer was dried over Na₂SO₄ and concentrated. The crude product was purified by FCC (DCM/MeOH = 10:1) to give compound 1 (300 mg, 0.550 mmol, 67.63% yield) as a yellow solid.

A solution of compound 1 (300 mg, 0.611 mmol) and Lawesson's reagent (494 mg, 1.221 mmol) in THF (10 mL) was stirred and heated at 40 °C for 2 h. Then, the mixture was diluted with water (10 mL) and extracted with EtOAc (10 mL × 3). The combined organic layer was dried over Na₂SO₄ and concentrated. The crude product was purified by FCC (DCM/MeOH = 10:1) to give compound 2 (250 mg, 0.443 mmol, 72.62% yield) as a yellow solid.

A solution of compound 2 (250 mg, 0.493 mmol), compound 3 (125 mg, 0.739 mmol), and TsOH (17 mg, 0.099 mmol) in AcOH (5 mL) was stirred and heated at 50 °C for 2 h. Then, the mixture was diluted with water (5 mL) and extracted with EtOAc (5 mL × 3). The combined organic layer was dried over Na₂SO₄ and concentrated. The crude product was purified by FCC (DCM/MeOH = 10:1) to give compound 4 (150 mg, 0.254 mmol, 51.55% yield) as a yellow solid.

A solution of compound 4 (30 mg, 0.057 mmol), compound 5 (9 mg, 0.170 mmol), Pd(PPh₃)₄ (13 mg, 0.011 mmol), CuI (2 mg, 0.011 mmol), and TEA (17 mg, 0.170 mmol) in DMF (3 mL) was stirred at room temperature for 2 h under N₂ atmosphere. The mixture was diluted with MeOH, filtered, and purified by prep-HPLC (column: Phenomenex moon C18 250 mm × 100 mm × 10 µm; mobile phase: [H₂O (0.1% FA)-ACN]; B%: 20%- 60%, 30 min) to give 9135 (5 mg, 0.010 mmol, 95% yield) as a white solid.
**LCMS:** [M+H] ⁺ = 459.1;
**¹H NMR:** (400 MHz, MeOD) δ 7.723 (d, J = 3.4 Hz, 1H), 7.639 (d, J = 3.4 Hz, 1H), 7.446 (d, J = 6.6 Hz, 4H), 4.569 (t, J = 6.8 Hz, 1H), 4.064 (dd, J = 13.6, 10.4 Hz, 2H), 3.384 (s, 2H), 2.605 (s, 3H), 2.399 (s, 3H), 1.597 (s, 3H). **HPLC:** 95.86% purity;
**SFC:** Rt = 3.550 min.

### Example 1-9

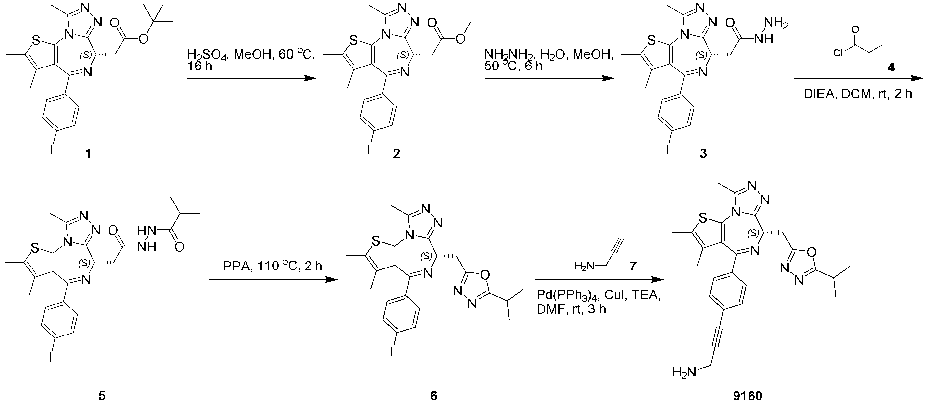

A solution of compound 1 (1 g, 0.002 mol) in MeOH/H₂SO₄ = 20:1 (20 mL) was stirred at 60 °C for 16 h under N₂ atmosphere. The mixture was evaporated and the residue was purified by flash chromatography (DCM/MeOH = 98/2) to give compound 2 (0.89 g, 83.33% yield) as a yellow solid.

To a solution of compound 2 (890 mg, 1.758 mmol) in MeOH (20 mL) was added NH₂NH₂·H₂O (879 mg, 17.576 mmol) dropwise. The reaction mixture was stirred and heated at 50 °C for 5 h. The solution was evaporated and the residue was purified by flash chromatography (DCM/MeOH = 95/5) to give compound 3 (800 mg, 85.39% yield) as a yellow solid.

To a solution of compound 3 (800 mg, 1.580 mmol) and DIEA (408 mg, 3.160 mmol) in DCM (50 mL) was added compound 4 (168 mg, 1.580 mmol) dropwise at room temperature. The reaction mixture was stirred at room temperature for 2 h. The solution was evaporated and the residue was purified by flash chromatography (DCM/MeOH = 96/4) to give compound 5 (700 mg, 73.02% yield) as a yellow solid.

A solution of compound 5 (700 mg, 1.214 mmol) in PPA (60 mL) was heated to 110 °C and stirred at 110 °C for 2 h. The reaction was quenched with H₂O (50 mL) at 25 °C, diluted with EA (300 mL), washed with water and brine, dried over Na₂SO₄, filtered, and concentrated. The mixture was evaporated and the residue was purified by flash chromatography (DCM/MeOH = 95/5) to give compound 6 (500 mg, 70.05% yield) as a yellow solid.

A solution of compound 6 [50 mg, 0.089 mmol], compound 7 (6 mg, 0.107 mmol), Pd(PPh₃)₄ (21 mg, 0.018 mmol), TEA (18 mg, 0.179 mmol), and copper(I) iodide in DMF (10 mL) was stirred at room temperature for 3 h. The mixture was directly purified by prep-HPLC (column: Phenomenex luna C18 250 mm × 100 mm × 10 µm; mobile phase: [H₂O (0.1% FA)-ACN]; B%: 10%-50%, 40 min) to give 9160 (25 mg, 54.64% yield) as a white solid.
**LCMS:** [M+H] ⁺ = 486.2;
**¹H NMR:** (400 MHz, MeOD) δ 7.481 - 7.460 (d, J = 8.4 Hz, 2H), 7.421 - 7.400 (d, J = 8.4 Hz, 2H), 4.819 - 4.783 (m, 1H), 4.056 - 3.999 (m, 2H), 3.887 (s, 2H), 3.298 - 3.203 (m, 1H), 2.692 (s, 3H), 2.453 (s, 3H), 1.679 (s, 3H), 1.402 - 1.379 (m, 3H).
**HPLC:** 97.45% purity.

### Example 1-10

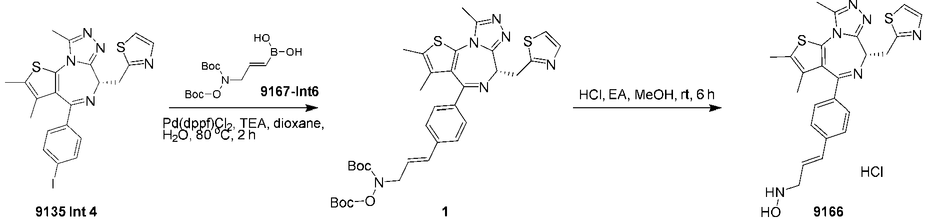

A solution of 9135 Int 4 (1.5 g, 0.003 mol), 9167-Int6 (1.79 g, 0.006 mol), Pd(dppf)Cl₂ (0.41 g, 0.5 mmol), and TEA (0.85 g, 0.008 mol) in 1,4-dioxane/H₂O = 4/1 (15 mL) was heated to 80 °C and stirred at 80 °C for 2 h under N₂ atmosphere. The mixture was diluted with H₂O (10 mL), extracted with EtOAc (20 mL × 3), washed with brine (20 mL), and dried over anhydrous sodium sulfate. The organic layer was concentrated under reduced pressure to give a crude product. The residue was purified by flash chromatography column (eluent: MeOH in DCM = 0-5%) to give compound 6 (600 mg, 0.80 mmol, 30% yield) as a yellow solid.

A solution of compound 1 (600 mg, 0.884 mmol) in HCl in EA/MeOH (10 mL, 2 M) was stirred at room temperature for 6 h under N₂ atmosphere. The solvent was removed under N₂ flow to give a crude product. The residue was purified by reverse column (eluent: ACN in H₂O = 5-30%, 0.1% TFA) to give 9166 (300 mg, 0.598 mmol, 68% yield) as a yellow solid.
**LCMS:** [M+H] ⁺ = 477.10.
¹H NMR: (400 MHz, MeOD) δ 7.755 (d, J = 3.6 Hz, 1H), 7.562 (d, J = 3.6 Hz, 5H), 7.525 (d, J = 8.4 Hz, 2H), 7.475 (d, J = 8.4 Hz, 2H), 6.931 (d, J = 16.0 Hz, 1H), 6.421 - 6.305 (m, 1H), 4.661 (dd, J = 8.8, 5.2 Hz, 1H), 4.196 (ddd, J = 20.4, 15.2, 7.0 Hz, 2H), 4.018 (d, J = 7.2 Hz, 2H), 2.711 (s, 3H), 2.439 (s, 3H), 1.659 (s, 3H).
**HPLC:** 95.6% purity.

### Example 1-11

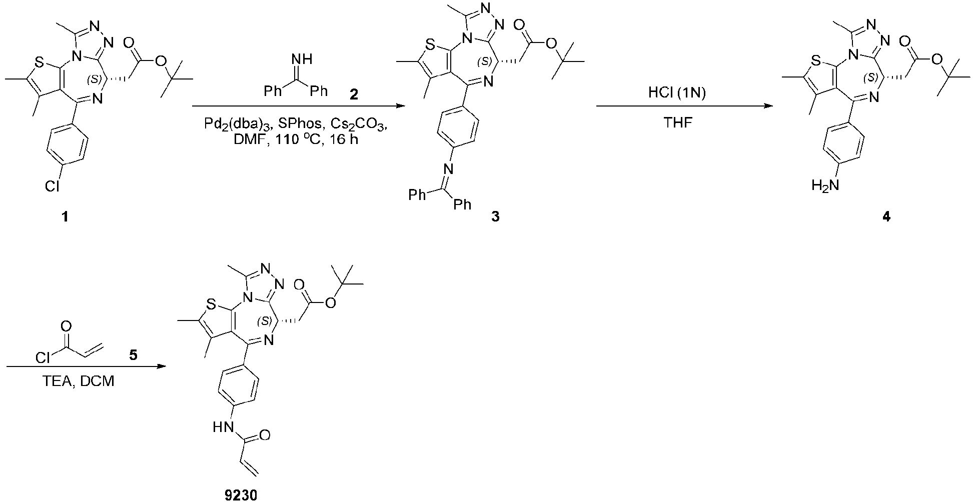

To a solution of compound 1 (300 mg, 0.657 mmol) in 1,4-dioxane (10 mL) was added compound 2 (178 mg, 0.985 mmol), Cs₂CO₃ (642 mg, 1.97) and XPhos Pd G3 (55.6 mg, 0.0657 mmol) at 100 °C. The reaction mixture was stirred at 100 °C under N₂ for 8 h. The mixture was concentrated under reduced pressure. The residue was purified by silica gel chromatography eluting with EtOAc in petroleum from 0% to 50% to give compound 3 (300 mg, 72 %yield) as a yellow solid. LC-MS: (ESI) m/z [M+H] ⁺ 602.2.

To a stirred solution of compound 3 (300 mg, 0.499 mmol) in THF (5.0 mL) was added 1 M HCl (1.0 mL) dropwise at 25 °C. The resulting mixture was stirred for 30 min at 25°C. The reaction was concentrated under reduced pressure and was purified by reversed phase chromatograph (ACN-Water(0.1% NH₃HCO₃); From 5% to 10%) to give compound 4 (150 mg, 69% yield) as a white solid. LC-MS: (ESI) m/z [M+H] ⁺ 438.2.

To a solution of compound 4 (60.0 mg, 0.137 mmol) in DCM (6.0 mL) was added TEA (41.6 mg, 0.411 mmol) and prop-2-enoyl chloride (24.8 mg, 0.274 mmol) at 25 °C. The reaction mixture was stirred at 25 °C for 10 min. The mixture was concentrated under reduced pressure, and the residue was purified by Prep-HPLC (with CH₃CN from 45% to 75% in 10 min) to give compound 9230 (13.4 mg, 19% yield) as a white solid.

¹H NMR (400 MHz, DMSO-*d₆*) δ 10.40 (s, 1H), 7.71 (d, *J =* 8.4 Hz, 2H), 7.39 (d, *J =* 8.4 Hz, 2H), 6.50 - 6.40 (m, 1H), 6.33 - 6.24 (m, 1H), 5.82 - 5.73 (m, 1H), 4.42 - 4.36 (m, 1H), 3.33 - 3.28 (m, 2H), 2.60 (s, 3H), 2.42 (s, 3H), 1.67 (s, 3H), 1.43 (s, 9H). LC-MS: m/z [M+H] ⁺ 492.2.

### Example 1-12

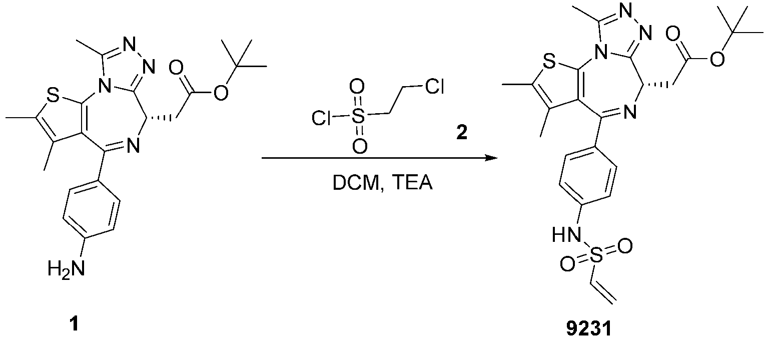

To a solution of compound 1 (60.0 mg, 0.137 mmol) in DCM (6.0 mL) was added TEA (41.6 mg, 0.411 mmol) and 2-chloroethanesulfonyl chloride (44.7 mg, 0.274 mmol) at 25 °C. The reaction mixture was stirred at 25 °C for 10 min. The mixture was concentrated under reduced pressure, and the residue was purified by Prep-HPLC (with CH₃CN from 45% to 75% in 10 min) to give compound 9231 as a white solid.

¹H NMR (400 MHz, DMSO-*d₆*) δ 10.40 (s, 1H), 7.71 (d, *J* = 8.4 Hz, 2H), 7.39 (d, *J* = 8.4 Hz, 2H), 6.51 - 6.38 (m, 1H), 6.33 - 6.22 (m, 1H), 5.82 - 5.74 (m, 1H), 4.44 - 4.34 (m, 1H), 3.33 - 3.28 (m, 2H), 2.60 (s, 3H), 2.43 (s, 3H), 1.67 (s, 3H), 1.43 (s, 9H). LC-MS: m/z [M+H] ⁺ 528.2.

### Example 1-13

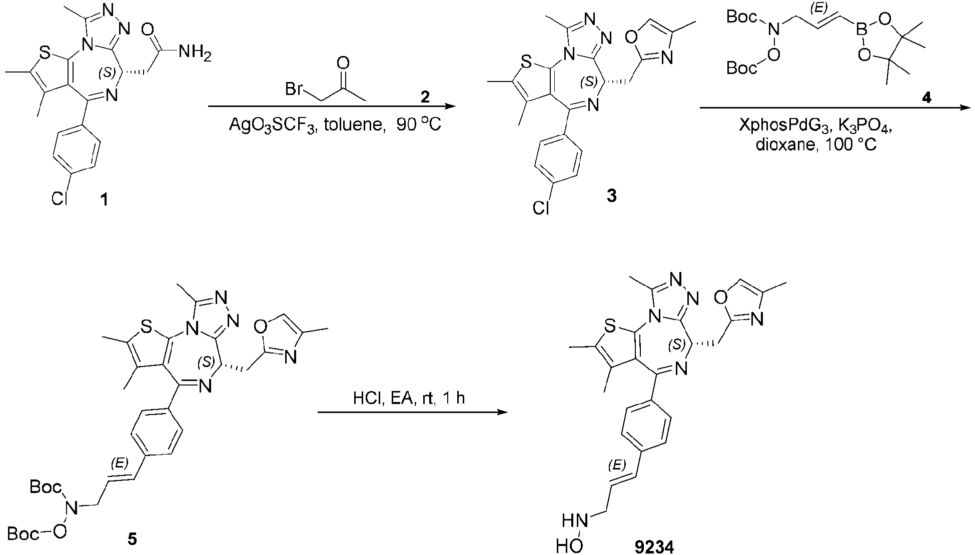

To a solution of compound 1 (2500 mg, 6.25 mmol) in toluene (10 mL) was added 1-bromopropan-2-one (4281 mg, 31.25 mmol) and CF₃SO₃Ag (3212 mg, 12.50 mmol) at 25 °C. After addition, the mixture was stirred at 90 °C for 2 h. The reaction mixture was purified by prep-HPLC (with CH₃CN from 10% to 70% in 10 min) to give compound 3(100 mg, 3% yield) as a yellow solid. LC-MS: (ESI) m/z [M+H]⁺ 438.1.

The mixture of compound 3 (100 mg, 0.22 mmol), X phos Pd G3 (19 mg, 0.02 mmol), compound 4 (137 mg, 0.34 mmol) and K₃PO₄ (145 mg, 0.68 mmol) in dioxane (1 mL) and H₂O (0.1 mL) was stirred at 25 °C under N₂. The mixture was warmed to 100 °C and stirred at that temperature for 1 h. The reaction mixture was cooled to 25 °C, diluted with water (10 mL) and extracted with EtOAc (20 mL × 2). The combined organic phases were dried over Na₂SO₄ and filtered. The filtrate was concentrated under reduced pressure. The residue was purified by silica gel chromatography eluting with EtOAc/petroleum ether with EtOAc from 0 to 80% in 15 min to give compound 5 (50 mg, 29% yield) as a yellow solid. LC-MS: (ESI) m/z [M+H]⁺ 675.2.

To a solution of compound 5 (50 mg, 0.07 mmol) in EA (1 mL) was added HCl in EA (1 mL) at 25 °C. After addition, the mixture was stirred at 25 °C for 1 h. The reaction mixture purified by prep-HPLC (with CH₃CN from 10% to 70% in 10 min) to afford compound 9234 (4.2 mg, 11% yield) as a white solid.

¹H NMR (400 MHz, DMSO-*d₆*) δ 11.80 (s, 2H), 10.97 (s, 1H), 7.75 (s, 1H), 7.55 - 7.39 (m, 4H), 6.86 - 6.82 (m, 1H), 6.42 - 6.40 (m, 1H), 4.77 - 4.473(m, 1H), 3.88 - 3.82 (m, 4H), 2.68 (s, 3H), 2.49 (s, 3H), 2.03 (s, 3H), 1.61 (s, 3H). LC-MS: (ESI) m/z [M+H]⁺ 475.1.

### Example1-14

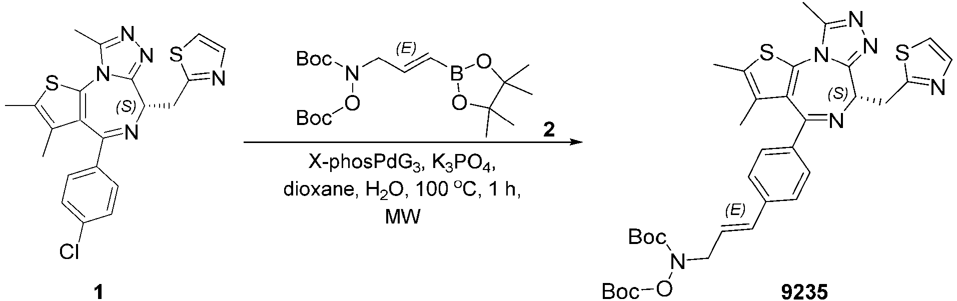

A mixture of compound 1 (3.6 g, 8.2 mmol), compound 2 (4.9 g, 12.3 mmol), X phos Pd G₃ (690 mg, 0.8 mmol) and K₃PO₄ (5.22 g, 24.6 mmol) in 1,4-dioxane/H₂O(44 mL) was heated at 100 °C for 1h with use Microwave. LCMS showed the starting material was consumed and desired product was formed. The mixture was diluted with H₂O (50 mL), extracted with EtOAc (50 mL × 3), washed with brine (50 mL), dried over anhydrous sodium sulfate. The organic layer was concentrated under reduced pressure to give the crude product. The residue was purified by flash chromatography column (eluent MeOH in DCM = 0 - 10%) to afford compound 9235 (2.5 g, 3.7 mmol, 45% yield) as a yellow solid.

¹H NMR (400 MHz, DMSO-*d₆*) δ 7.71 - 7.70 (m, 1H), 7.63 - 7.62 (m, 1H), 7.50 - 7.40 (m, 4H), 6.63 - 6.59 (m, 1H), 6.37 - 6.32 (m, 1H), 4.54 - 4.53 (m, 1H), 4.30 (s, 2H), 4.08 - 4.06 (m, 2H), 2.60 (s, 3H), 2.39 (s, 3H), 1.58 (s, 3H), 1.41 - 1.38 (m, 18H), 1.75 - 1.50 (m, 6H). LC-MS: (ESI) m/z [M+H]⁺ 677.2.

### Example1-15

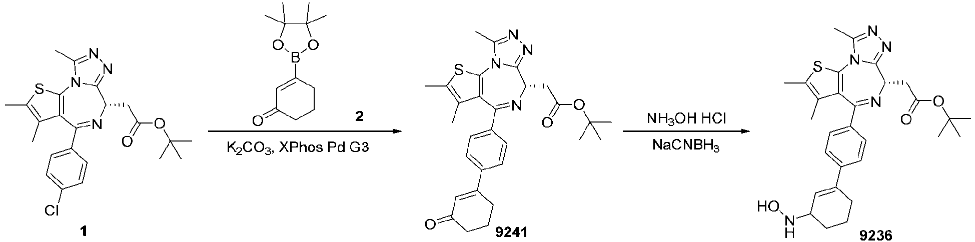

To a solution of compound 1 (400 mg, 0.875 mmol) and compound 2 (292 mg, 1.31 mmol) in 1,4-dioxane (10 mL) and H₂O (1 mL) was added K₂CO₃ (363 mg, 2.63 mmol) and Xphos Pd G3 (74.1 mg, 0.0875 mmol) at 25 °C. The reaction mixture was stirred at 100 °C under N₂ for 6 h. The mixture was concentrated under reduced pressure. The residue was purified by silica gel chromatography eluting with THF in petroleum from 0% to 65% to give compound 9241 (410 mg, 86%yield) as a yellow solid.

¹H NMR (400 MHz, DMSO-*d₆*) δ 7.74 (d, *J =* 8.4 Hz, 2H), 7.48 (d, *J =* 8.4 Hz, 2H), 6.42 (s, 1H), 4.50 - 4.40 (m, 1H), 3.35 - 3.30 (m, 2H), 2.81 - 2.71 (m, 2H), 2.63 (s, 3H), 2.44 (s, 3H), 2.41 - 2.33 (m, 2H), 2.12 - 2.00 (m, 2H), 1.64 (s, 3H), 1.44 (s, 9H). LC-MS: (ESI) m/z [M+H] ⁺ 517.2.

To a solution of compound 9241 (60.0 mg, 0.116 mmol) in EtOH (6.0 mL) was added Hydroxylamine hydrochloride (12.1 mg, 0.174 mmol) and TEA (17.6 mg, 0.174 mmol). After stirred at 80 °C for 3 h, Sodium cyanoborohydride (36.5 mg, 0.581 mmol) was added at 25 °C. Then the mixture was stirred at 25 °C for 12 h. The mixture was quenched with water (20 mL) and then extracted with ethyl acetate (30 mL x 2). The combined organic phases were dried over sodium sulfate and filtered. The mixture was concentrated under reduced pressure, and the residue was purified by Prep-HPLC (with CH3CN from 30% to 40% in 8 min) to give compound 9236 (3.80 mg, 6.0% yield) as a white solid.

¹H NMR (400 MHz, DMSO-*d₆*) δ 10.86 (s, 1H), 7.37 (d, *J* = 8.4 Hz, 2H), 7.06 (d, *J* = 8.4 Hz, 2H), 6.46 (s, 1H), 5.31 (s, 1H), 4.17 (s, 1H), 3.26 - 3.17 (m, 1H), 3.09 - 3.96 (m, 1H), 2.78 - 2.69 (m, 1H), 2.49 - 2.41 (m, 7H), 2.34 (s, 3H), 1.87 (s, 3H), 1.81 - 1.72 (m, 2H), 1.42 (s, 9H). LC-MS: (ESI) m/z [M+H] ⁺ 543.2.

### Example1-16

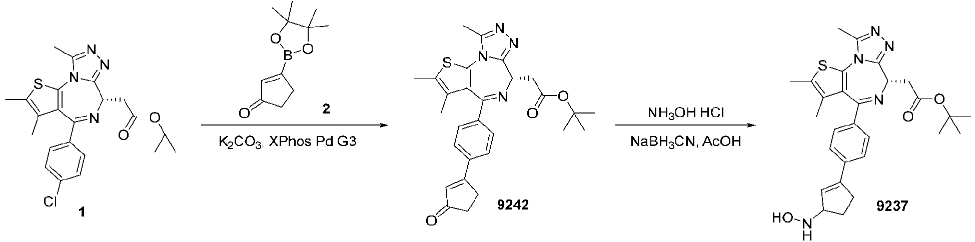

To a solution of compound 1 (400 mg, 0.875 mmol) and compound 2 (273 mg, 1.31 mmol) in 1,4-dioxane (10 mL) and H₂O (1 mL) was added K₂CO₃ (363 mg, 2.63 mmol) and Xphos Pd G3 (74.1 mg, 0.0875 mmol) at 25 °C. The reaction mixture was stirred at 100 °C under N₂ for 6 h. The mixture was concentrated under reduced pressure. The residue was purified by silica gel chromatography eluting with THF in petroleum from 0% to 45% to give compound 9242 (390 mg, 84% yield) as a yellow solid.

¹H NMR (400 MHz, DMSO-*d₆*) δ 7.87 (d, *J =* 8.4 Hz, 2H), 7.51 (d, *J =* 8.4 Hz, 2H), 6.80 (s, 1H), 4.52 - 4.38 (m, 1H), 3.37 - 3.35 (m, 2H), 3.08 - 2.97 (m, 2H), 2.62 (s, 3H), 2.49 - 2.45 (m, 2H), 2.43 (s, 3H), 1.64 (s, 3H), 1.44 (s, 9H). LC-MS: (ESI) m/z [M+H] ⁺ 503.2.

To a solution of compound 9242 (80.0 mg, 0.159 mmol) in EtOH (6.0 mL) was added Hydroxylamine hydrochloride (22.1 mg, 0.318 mmol) and TEA (48.3 mg, 0.477 mmol). After stirred at 80 °C for 3 h, NaCNBH₃ (50.0 mg, 0.796 mmol) was added at 25 °C. Then the mixture was stirred at 25 °C for 12 h. The mixture was quenched with water (20 mL) and then extracted with ethyl acetate (30 mL x 2). The combined organic phases were dried over sodium sulfate and filtered. The mixture was concentrated under reduced pressure, and the residue was purified by Prep-HPLC (with CH₃CN from 30% to 40% in 8 min) to give compound 9237 (14.0 mg, 17% yield) as a white solid.

¹H NMR (400 MHz, DMSO-*d₆*) δ 10.48 (s, 1H), 7.45 (d, *J* = 8.4 Hz, 2H), 7.07 (d, *J* = 8.4 Hz, 2H), 6.72 (s, 1H), 5.31 (d, *J* = 8.0 Hz, 1H), 4.23 - 4.12 (m, 1H), 3.31 - 3.24 (m, 1H), 3.05 (dd, *J* = 16.4, 7.2 Hz, 1H), 2.81 - 2.76 (m, 2H), 2.76 - 2.69 (m, 1H), 2.67 - 2.60 (m, 2H), 2.42 (s, 3H), 2.35 (s, 3H), 1.88 (s, 3H), 1.41 (s, 9H). LC-MS: (ESI) m/z [M+H] ⁺ 520.2.

### Example 1-17

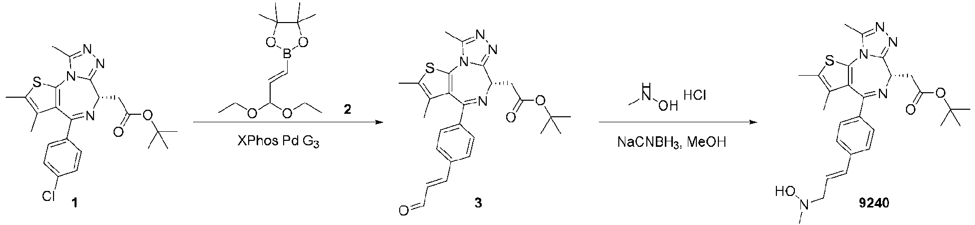

To a solution of compound 1 (400 mg, 0.875 mmol) and compound 2 (359 mg, 1.40 mmol) in 1,4-dioxane (10 mL) and H₂O (1 mL) was added K₃PO₄ (557 mg, 2.63 mmol) and Xphos Pd G3 (74.1 mg, 0.0875 mmol) at 25°C. The reaction mixture was stirred at 100 °C under N2 for 1 h. The mixture was concentrated under reduced pressure. The residue was purified by silica gel chromatography eluting with THF in petroleum from 0% to 65% to give compound 3 (350 mg, 76% yield) as a yellow solid. LC-MS: (ESI) m/z [M+H] ⁺ 477.2.

To a solution of compound 3 (80.0 mg, 0.168 mmol) in MeOH (6.0 mL) was added *N*-methylhydroxylamine hydrochloride (28.1 mg, 0.336 mmol) and TEA (34.0 mg, 0.336 mmol). After stirred at 25 °C for 30 min, NaBH₃CN (106 mg, 1.68 mmol) was added at 25 °C. Then the mixture was stirred at 25 °C for 12 h. The mixture was quenched with water (20 mL) and then extracted with ethyl acetate (30 mL x 2). The combined organic phases were dried over sodium sulfate and filtered. The mixture was concentrated under reduced pressure, and the residue was purified by Prep-HPLC (with CH3CN from 30% to 40% in 8 min) to give compound 9240 (14.0 mg, 16% yield) as a white solid. ¹H NMR (400 MHz, DMSO-*d₆*) δ 7.95 (s, 1H), 7.51 - 7.44 (m, 2H), 7.42 - 7.33 (m, 2H), 6.62 - 6.50 (m, 1H), 6.47 - 6.38 (m, 1H), 4.48 - 4.31 (m, 1H), 3.34 - 3.30 (m, 4H), 2.60 (s, 3H), 2.50 (s, 3H), 2.42 (s, 3H), 1.65 (s, 3H), 1.43 (s, 9H). LC-MS: (ESI) m/z [M+H] ⁺ 508.2.

### Example1-18

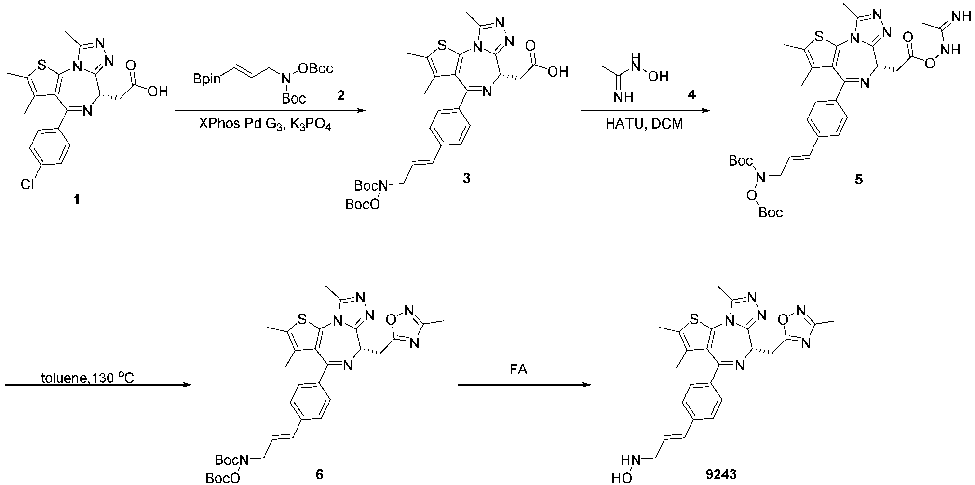

A mixture of compound 1 (500 mg, 1.24 mmol), tert-butyl {[tert-butyl(formyl)-$l^{3}-oxidanyl][(2E)-3-(dihydroxyboranyl)prop-2-en-1-yl]amino}oxy formate (750 mg, 1.87 mmol), X phos Pd G₃ (105 mg, 0.12 mmol) and K₃PO₄ (1323 mg, 6.23 mmol) in 1,4-dioxane/H₂O (11 mL) was heated at 100 °C for 1h. LCMS showed the starting material was consumed and desired product was formed. The mixture was diluted with H₂O (50 mL), extracted with EtOAc (50 mL × 3), washed with brine (50 mL), dried over anhydrous sodium sulfate. The organic layer was concentrated under reduced pressure to give the crude product. The residue was purified by flash chromatography column (eluent MeOH in DCM = 0 - 10%) to afford compound 3 (350 mg, 39% yield) as a yellow solid. LC-MS: (ESI) m/z [M+H]⁺ 638.3.

To a solution of compound 3 (450 mg, 0.70 mmol) in DCM (10 mL) was added compound 4 (78 mg, 1.0551 mmol), HATU (401 mg, 1.05 mmol) and DIEA (272 mg, 2.11 mmol) at 25 °C. After addition, the mixture was stirred at 25 °C for 1 h. The reaction mixture was concentrated under reduced pressure. The residue was purified by silica gel chromatography eluting with EtOAc/petroleum ether with EtOAc from 0 to 90% in 15 min to give compound 5 (400 mg, 73%yield) as a yellow solid. LC-MS: (ESI) m/z [M+H]⁺ 694.3.
compound 5 (400 mg, 0.57 mmol) in toluene (10 mL) was stirred at 130 °C for 1 h. The reaction mixture was concentrated under reduced pressure. The residue was purified by silica gel chromatography eluting with EtOAc/petroleum ether with EtOAc from 0 to 90% in 15 min to give compound 6 (250 mg, 61% yield) as a yellow solid. LC-MS: (ESI) m/z [M+H]⁺ 676.2.
compound 6 (130 mg, 0.19 mmol) in FA (2 mL) was stirred at 25 °C for 1 h. The reaction mixture was concentrated under reduced pressure. The reaction mixture was purified by prep-HPLC (with CH₃CN from 0% to 100% in 10 min) to afford compound 9243_(10 mg, 10% yield) as a white solid.

¹H NMR (400 MHz, DMSO-*d₆*) δ 7.46 - 7.44 (m, 2H), 7.34 - 7.32 (m, 2H), 6.54 - 6.50 (m, 1H), 6.44 - 6.37 (m, 1H), 4.73 - 4.69 (m, 1H), 4.01 - 3.94 (m, 2H), 3.51 - 3.49 (m, 2H), 2.62 (s, 3H), 2.42 (s, 3H), 2.31 (s, 3H), 1.63 (s, 3H). LC-MS: (ESI) m/z [M+H]⁺ 476.2.

### Example1-19

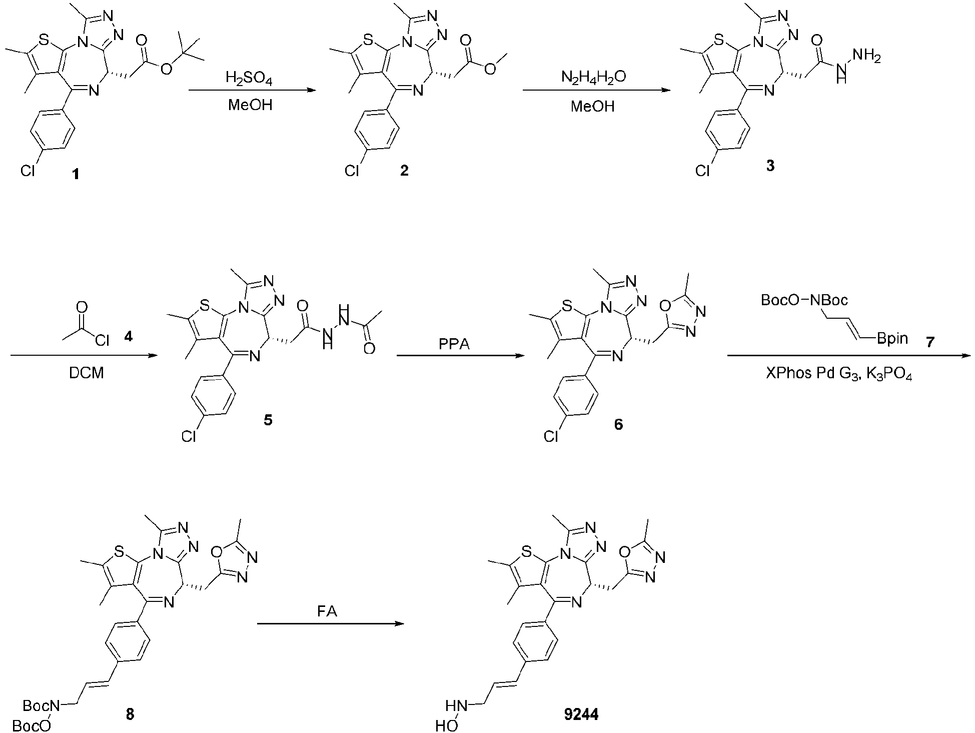

To a solution of compound 1 (1 g, 2.10 mmol) in MeOH (20 mL) was added H₂SO₄ (20 mL) at 25 °C. After addition, the mixture was stirred at 60 °C for 16 h. The reaction mixture was diluted with water (50 mL) and extracted with EtOAc (100 mL × 2). The combined organic phases were dried over Na₂SO₄ and filtered. The filtrate was concentrated under reduced pressure to give compound 2 (800 mg, 79% yield) as a yellow solid LC-MS: (ESI) m/z [M+H]⁺ 415.0.

To a solution of compound 2 (1000 mg, 2.41 mmol) in MeOH (20 mL) was added N₂H₄.H₂O (772 mg, 24.10 mmol) at 25 °C. After addition, the mixture was stirred at 50 °C for 2 h. The reaction mixture was diluted with water (30 mL) and extracted with EtOAc (50 mL × 2). The combined organic phases were dried over Na₂SO₄ and filtered. The filtrate was concentrated under reduced pressure. The residue was purified by silica gel chromatography eluting with EtOAc/petroleum ether with EtOAc from 0 to 90% in 15 min to give compound 3 (800 mg, 76% yield) as a yellow solid. LC-MS: (ESI) m/z [M+H]⁺ 415.1.

To a solution of compound 3 (500 mg, 1.20 mmol) in DCM (10 mL) was added acetyl chloride(104 mg, 1.32 mmol) and DIEA (311 mg, 2.41 mmol) at 25 °C. After addition, the mixture was stirred at 25 °C for 1 h. The reaction mixture was diluted with water (10 mL) and extracted with DCM (20 mL × 2). The combined organic phases were dried over Na₂SO₄ and filtered. The filtrate was concentrated under reduced pressure. The residue was purified by silica gel chromatography eluting with EtOAc/petroleum ether with EtOAc from 0 to 90% in 15 min to give compound 5 (300 mg, 54% yield) as a yellow solid. LC-MS: (ESI) m/z [M+H]⁺ 457.1.

Compound 5 (300 mg, 0.61 mmol) in PPA (5 mL) was stirred at 110 °C for 1 h. The reaction mixture was diluted with water (10 mL) and extracted with EtOAc (20 mL × 2). The combined organic phases were dried over Na₂SO₄ and filtered. The filtrate was concentrated under reduced pressure. The residue was purified by silica gel chromatography eluting with EtOAc/petroleum ether with EtOAc from 0 to 90% in 15 min to give compound 6 (150 mg, 52% yield) as a yellow solid. LC-MS: (ESI) m/z [M+H]⁺ 439.1.

The mixture of compound 6 (200 mg, 0.45 mmol), X phos Pd G₃(38 mg, 0.04 mmol), compound 7 (310 mg, 0.77 mmol) and K₃PO₄ (290 mg, 1.36 mmol) in Dioxane (2 mL) and H₂O(0.2 mL) was stirred at 25 °C under N₂. The mixture was warmed to 100 °C and stirred at that temperature for 1 h. The reaction mixture was cooled to 25 °C, diluted with water (10 mL) and extracted with EtOAc (20 mL × 2). The combined organic phases were dried over Na₂SO₄ and filtered. The filtrate was concentrated under reduced pressure. The residue was purified by silica gel chromatography eluting with EtOAc/petroleum ether with EtOAc from 0 to 80% in 15 min to give compound 8 (150 mg, 46% yield) as a yellow solid. LC-MS: (ESI) m/z [M+H]⁺ 676.3.
compound 8(150 mg, 0.22 mmol) in FA (5 mL) was stirred at 25 °C for 10 min. The reaction mixture was concentrated under reduced pressure. The reaction mixture was purified by prep-HPLC (with CH₃CN from 0% to 100% in 10 min) to afford compound 9244 (6 mg, 5% yield) as a white solid.

¹H NMR (400 MHz, DMSO-*d₆*) δ 7.46 - 7.44 (m, 2H), 7.35 - 7.33 (m, 2H), 6.58 - 6.54 (m, 1H), 6.44 - 6.39 (m, 1H), 4.68 - 4.64 (m, 1H), 3.94 - 3.90 (m, 2H), 3.55 - 3.52 (m, 2H), 2.62 (s, 3H), 2.49 (s, 3H), 2.42 (s, 3H), 1.63 (s, 3H). LC-MS: (ESI) m/z [M+H]⁺ 476.1.

### Example1-20

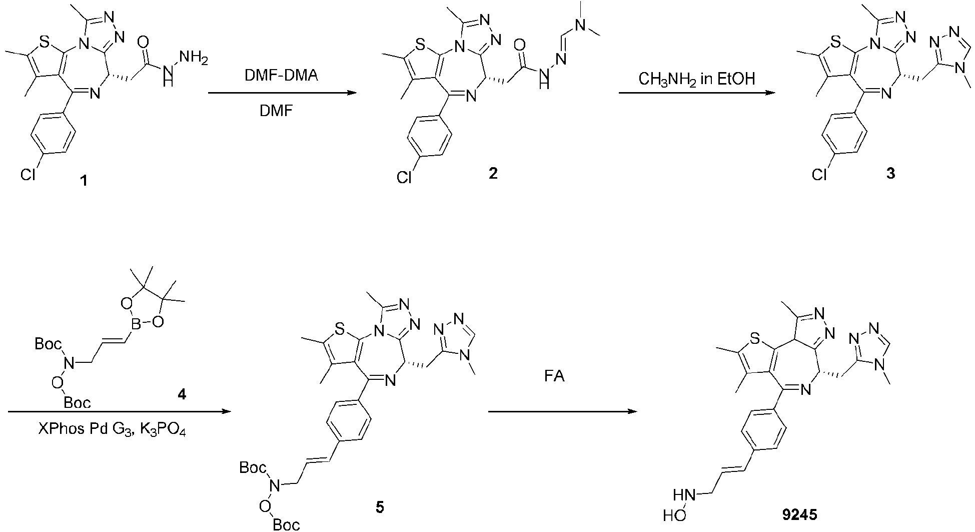

To a solution of compound 1 (800 mg, 1.92 mmol) in DMF (10 mL) was added DMF-DMA (1148 mg, 9.64 mmol) at 25 °C. After addition, the mixture was stirred at 110 °C for 2 h. The reaction mixture was concentrated under reduced pressure to give compound 2 (800 mg, 79% yield) as a yellow solid.

LC-MS: (ESI) m/z [M+H]⁺ 470.2.

To a solution of compound 2 (700 mg, 1.48 mmol) in EtOH (10 mL) was added methanamine (231 mg, 7.44 mmol) at 25 °C. After addition, the mixture was stirred at 110 °C for 1 h. The reaction mixture was concentrated under reduced pressure. The residue was purified by prep-HPLC (with CH₃CN from 0% to 100% in 10 min) to afford compound 3 (300 mg, 43% yield) as a white solid.

LC-MS: (ESI) m/z [M+H]⁺ 438.1.

The mixture of compound 3 (120 mg, 0.27 mmol), X phos Pd G₃ (23 mg, 0.02 mmol), compound 4 (186 mg, 0.46 mmol) and K₃PO₄ (174 mg, 0.82 mmol) in Dioxane (2 mL) and H₂O (0.2 mL) was stirred at 25 °C under N₂. The mixture was warmed to 100 °C and stirred at that temperature for 1 h. The reaction mixture was cooled to 25 °C, diluted with water (10 mL) and extracted with EtOAc (20 mL × 2). The combined organic phases were dried over Na₂SO₄ and filtered. The filtrate was concentrated under reduced pressure. The residue was purified by silica gel chromatography eluting with EtOAc/petroleum ether with EtOAc from 0 to 80% in 15 min to afford compound 5 (100 mg, 51% yield) as a yellow solid.

LC-MS: (ESI) m/z [M+H]⁺ 675.2.

Compound 5 (120 mg, 0.17 mmol) in FA (2 mL) was stirred at 25 °C for 1 h. The reaction mixture was concentrated under reduced pressure. The reaction mixture was purified by prep-HPLC (with CH₃CN from 0% to 90% in 10 min) to compound 9245 (10 mg, 11% yield) as a white solid.

¹H NMR (400 MHz, DMSO-*d₆*) δ 8.41 (s, 1H), 7.46 - 7.44 (m, 2H), 7.35 - 7.33 (m, 2H), 6.59 - 6.55 (m, 1H), 6.43 - 6.39 (m, 1H), 4.71 - 4.67 (m, 1H), 3.94 - 3.90 (m, 1H), 3.80 (s, 3H), 3.77 - 3.74 (m, 1H), 3.52 - 3.50 (m, 2H), 2.60 (s, 3H), 2.41 (s, 3H), 1.63 (s, 3H).). LC-MS: (ESI) m/z [M+H]⁺ 475.1.

### Example1-21

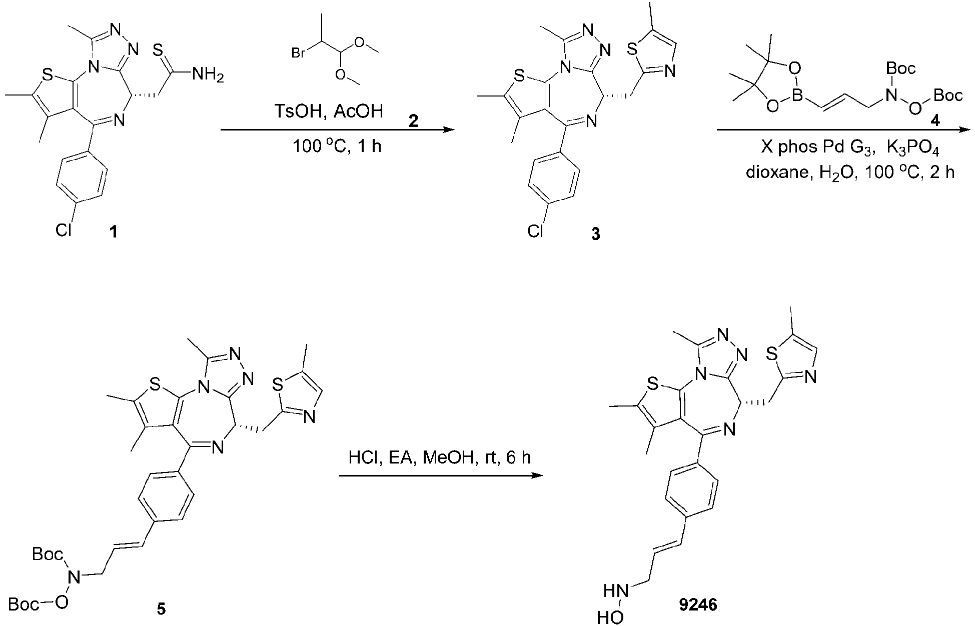

To a solution of compound 1 (300 mg, 0.72 mmol) in AcOH (5 mL) was added TsOH.H₂O (411 mg, 2.16 mmol) and compound 2 (330 mg, 1.80 mmol) at 25 °C. After addition, the mixture was stirred at 100 °C for 1 h. The reaction mixture was diluted with water (10 mL) and extracted with EtOAc (20 mL × 2). The combined organic phases were dried over Na₂SO₄ and filtered. The filtrate was concentrated under reduced pressure. The residue was purified by silica gel chromatography eluting with EtOAc/petroleum ether from 0 to 90% in 15 min to give compound 3 (200 mg, 54% yield) as a yellow solid.

LC-MS: (ESI) m/z [M+H]⁺ 454.1.

The mixture of compound 3 (200 mg, 0.44 mmol), X phos Pd G3(37 mg, 0.04 mmol), compound 4 (265 mg, 0.66 mmol) and K₃PO₄ (280 mg, 1.32 mmol) in Dioxane (5 mL) and H₂O (0.5 mL) was stirred at 25 °C under N₂. The mixture was warmed to 100 °C and stirred at that temperature for 2 h. The reaction mixture was cooled to 25 °C, diluted with water (10 mL) and extracted with EtOAc (20 mL × 2). The combined organic phases were dried over Na₂SO₄ and filtered. The filtrate was concentrated under reduced pressure. The residue was purified by silica gel chromatography eluting with EtOAc/petroleum ether with EtOAc from 0 to 80% in 15 min to give compound 5 (150 mg, 44% yield) as a yellow solid.

LC-MS: (ESI) m/z [M+H]⁺ 691.2.

Compound 5 (100 mg, 0.14 mmol) in FA(2 mL) was stirred at 25 °C for 1 h. The reaction mixture was concentrated under reduced pressure. The reaction mixture was purified by prep-HPLC (with CH₃CN from 0% to 100% in 10 min) to afford compound 9246 (10 mg, 13% yield) as a white solid.

¹H NMR (400 MHz, DMSO-*d₆*) δ 7.45 - 7.36 (m, 5H), 6.59 - 6.55 (m, 1H), 6.45 - 6.38 (m, 1H), 4.52 - 4.81 (m, 1H), 4.00 - 3.97 (m, 2H), 3.51 - 3.42 (m, 2H), 2.61 (s, 3H), 2.41 (s, 3H), 2.40 (s, 3H), 1.62 (s, 3H). LC-MS: (ESI) m/z [M+H]⁺ 491.9.

### Example1-22

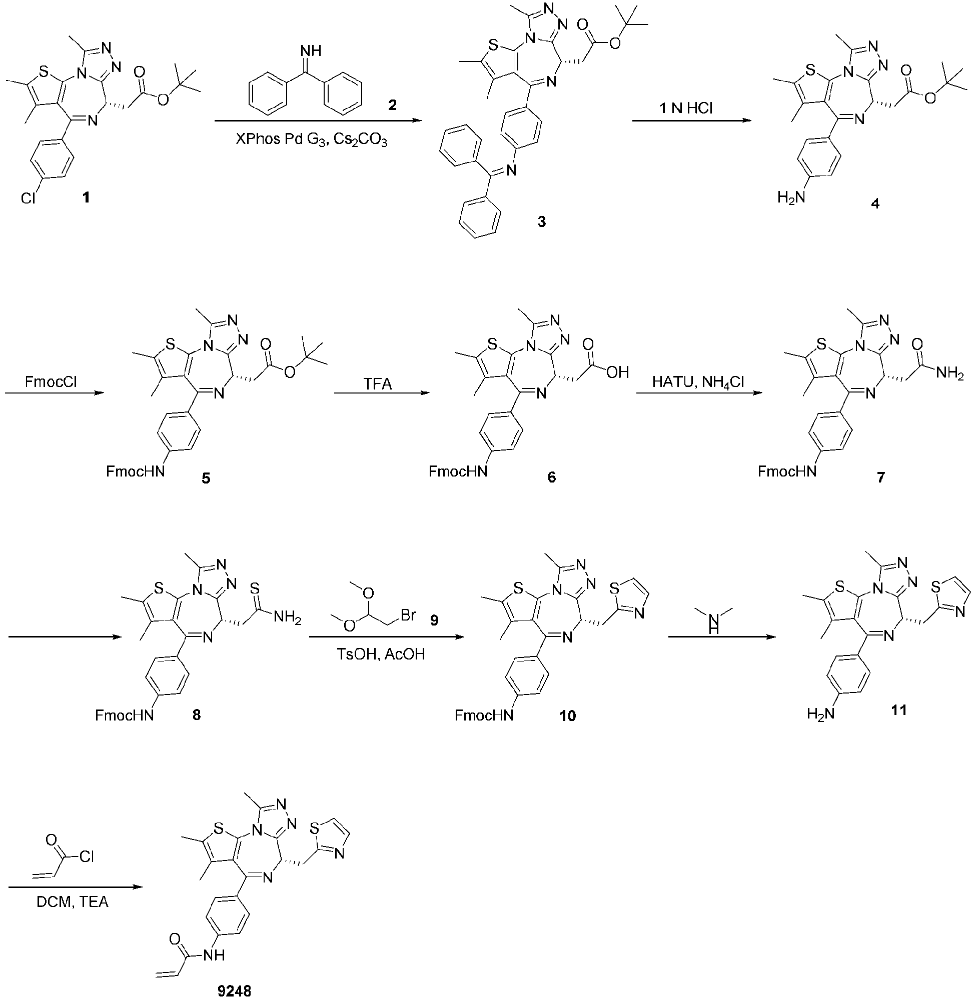

To a solution of compound 1 (300 mg, 0.657 mmol) in 1,4-dioxane (10 mL) was added compound 2 (178 mg, 0.985 mmol), Cs₂CO₃ (642 mg, 1.97) and Xphos Pd G3 (55.6 mg, 0.0657 mmol) at 100 °C. The reaction mixture was stirred at 100 °C under N₂ for 8 h. The mixture was concentrated under reduced pressure. The residue was purified by silica gel chromatography eluting with EtOAc in petroleum from 0% to 50% to give compound 3 (300 mg, 72 %yield) as a yellow solid.

LC-MS: (ESI) m/z [M+H] ⁺ 602.2.

To a stirred solution of compound 3 (1.10 g, 1.80 mmol) in THF (10 mL) was added 1 M HCl (2.0mL) dropwise at 25 °C. The resulting mixture was stirred for 30 min at 25 °C. The mixture was quenched with NaHCO₃ solution (30 mL) and then extracted with ethyl acetate (30 mL x 2). The combined organic phase was dried over sodium sulfate and filtered. The filtrate was concentrated under reduced pressure. The residue was purified by silica gel chromatography eluting with THF in petroleum from 0% to 80% to give compound 4 (0.730 g, 89% yield) as a white solid.

LC-MS: (ESI) m/z [M+H] ⁺ 438.2.

To a solution of compound 4 (730 mg, 1.67 mmol) in 1,4-dioxane (8.0 mL) and NaHCO₃ solution (1 M, 8.0 mL) was added 9*H*-fluoren-9-ylmethyl chloroformate (518 mg, 2.00 mmol) at 25°C. The reaction mixture was stirred at 25°C for 16 h. The mixture was quenched with water (10 mL) and then extracted with ethyl acetate (30 mL x 2). The combined organic phase was dried over sodium sulfate and filtered. The residue was purified by silica gel chromatography eluting with THF in petroleum from 0% to 20% to give compound 5 (730 mg, 65% yield) as a yellow solid.

LC-MS: (ESI) m/z [M+H] ⁺ 660.3.

To a solution of compound 5 (730 mg, 1.11 mmol) in DCM (6.0 mL) was added TFA (2.0 mL) at 25°C. The reaction mixture was stirred at 25 °C for 2 h. The filtrate was concentrated under reduced pressure to give compound 6 (660 mg, crude) as a yellow oil, which was used directly in the next step without further purification.

LC-MS: (ESI) m/z [M+H] ⁺ 604.2.

To a solution of compound 6 (667 mg, 1.11 mmol) in DCM (5.0 mL) was added HATU (462 mg, 1.22 mmol), DIPEA (857 mg, 6.63 mmol) and NH₄Cl (177 mg, 3.32 mmol) at 25 °C. The reaction mixture was stirred at 25 °C for 2 h. The reaction mixture was added to water (20 mL), The aqueous phase was extracted with ethyl acetate (30 mL X 2), The combined organic phases were washed with an aqueous solution of NaCl (20 mL x 3) and dried over Na₂SO₄, filtered, and concentrated under reduce pressure. The residue was purified by silica gel chromatography eluting with MeOH in DCM from 0 % to 6% to give compound 7 (609 mg, 87% yield) as a yellow solid.

LC-MS: (ESI) m/z [M+H] ⁺ 603.2.

A mixture of compound 7 (609 mg, 1.01 mmol) and Lawesson's Reagent (CAS:19172-47-5, 450 mg, 1.11 mmol) in THF (10 mL) was stirred and heated at 100 °C for 1 hours. The mixture was diluted with water (30 mL), extracted with EA (30 mL x 3). The combined organic layer was dried over Na₂SO₄ and concentrated. The crude product was purified by FCC (DCM/MeOH = 10:1) to give compound 8(500 mg, 78 % yield) as a yellow solid.

LC-MS: (ESI) m/z [M+ H] ⁺ 619.2.

A mixture of compound 8 (500 mg, 0.808 mmol), compound 9 (273 mg, 1.62 mmol) and TsOH (347 mg, 2.02 mmol) in AcOH (8.0 mL) was stirred and heated at 60 °C for 1 h with use Microwave. Then the mixture was diluted with water (20 mL), extracted with EtOAc (20 mL x 3). The combined organic layer was dried over Na₂SO₄ and concentrated. The crude product was purified by FCC (DCM/MeOH = 10:1) to give compound 10(330 mg, 62% yield) as a white solid.

LC-MS: (ESI) m/z [M+ H] ⁺ 643.2.

To a solution of compound 10 (330 mg, 0.513 mol) in DCM (1.5mL) was added dimethylamine (2 M, 1.5 mL) at 25 °C. The mixture was stirred at 25 °C for 1 h. The mixture was concentrated under reduced pressure. The residue was purified by silica gel chromatography eluting with MeOH in PE from 0% to 6% to give compound 11(200 mg, 88% yield) as a yellow solid.

LC-MS: (ESI) m/z [M+H] ⁺ 421.1.

To a solution of compound 12 (20.0 mg, 0.0476 mmol) in DCM (2.0 mL) was added prop-2-enoyl chloride (6.46 mg, 0.0714 mmol) and TEA (14.5 mg, 0.143 mmol) at 25 °C. The reaction mixture was stirred at 25 °C for 10 min. The mixture was concentrated. The residue was purified by Prep-HPLC (Chromatographic columns:-Xbridge-C18 150 x 19 mm,5um Mobile Phase : ACN-H₂O, 45-75 % MeCN in 0.1% FA/water, 30 mL/min) to give compound 9248 (4.10 mg, 18% yield) as a white solid.

¹H NMR (400 MHz, DMSO-*d₆*) δ 10.39 (s, 1H), 7.76 - 7.69 (m, 3H), 7.66 - 7.61 (m, 1H), 7.43 (d, *J* = 8.8 Hz, 2H), 6.51 - 6.40 (m, 1H), 6.27 (dd, *J* = 17.2, 2.0 Hz, 1H), 5.78 (dd, *J* = 10.0, 2.0 Hz, 1H), 4.56 - 4.45 (m 1H), 4.16 - 3.96 (m, 2H), 2.61 (s, 3H), 2.41 (s, 3H), 1.64 (s, 3H). LC-MS: (ESI) m/z [M+H] ⁺ 475.1.

### Example1-23

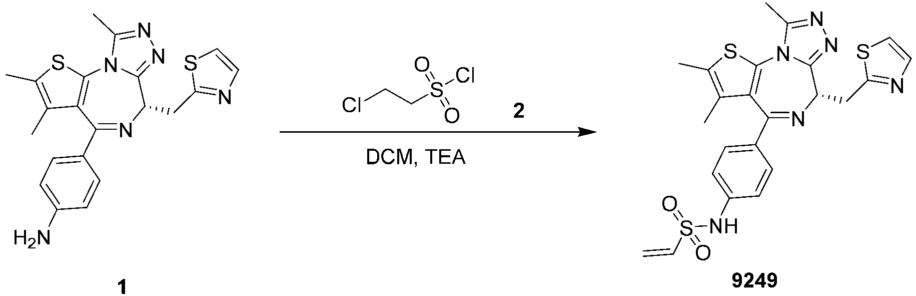

To a solution of compound 1 (30.0 mg, 0.0587 mmol) in DCM (2.0 mL) was added 2-chloroethanesulfonyl chloride (14.6 mg, 0.0881 mmol) and TEA (17.8 mg, 0.176 mmol) at 25 °C. The reaction mixture was stirred at 25 °C for 10 min. LCMS showed the starting material was consumed and desired product was formed. The mixture was concentrated. The residue was purified by Prep-HPLC (Chromatographic columns:-Xbridge-C18 150 × 19 mm,5um Mobile Phase: ACN-H₂O, 45-75 % MeCN in 0.1% FA/water, 30 mL/min) to give compound 9249 (4.10 mg, 13% yield) as a white solid.

¹H NMR (400 MHz, DMSO-*d₆*) δ 7.71 (d, *J =* 1.2 Hz, 1H), 7.61 (d, *J* = 1.2 Hz, 1H), 7.35 (d, *J =* 2.4 Hz, 2H), 7.10 (d, *J* = 2.4 Hz, 2H), 6.71 (dd, *J* = 6.4, 2.4 Hz, 1H), 6.10 (d, *J=* 16.4 Hz, 1H), 5.97 (d, *J =* 9.6 Hz, 1H), 4.52 - 4.46 (m, 1H), 4.06 - 3.99 (m, 2H), 2.59 (s, 3H), 2.40 (s, 3H), 1.62 (s, 3H). LC-MS: (ESI) m/z [M+H] ⁺ 511.1.

### Example1-24

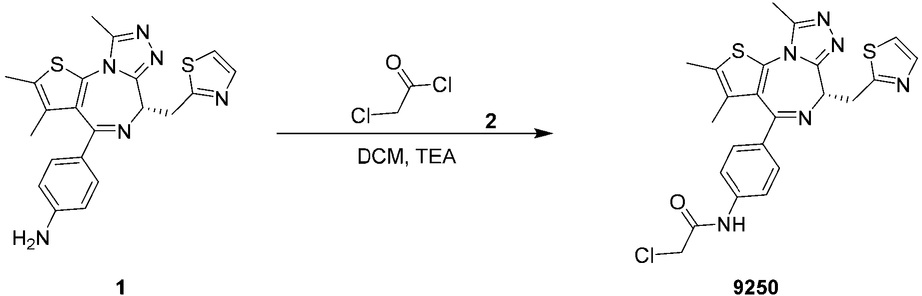

To a solution of compound 1 (20.0 mg, 0.0476 mmol) in DCM (2.0 mL) was added 2-chloroacetyl chloride (8.06 mg, 0.0714 mmol) and TEA (14.5 mg, 0.143 mmol) at 25 °C. The reaction mixture was stirred at 25 °C for 10 min. LCMS showed the starting material was consumed and desired product was formed. The mixture was concentrated. The residue was purified by Prep-HPLC (Chromatographic columns:-Xbridge-C18 150 × 19 mm,5um Mobile Phase : ACN-H₂O, 45-75 % MeCN in 0.1% FA/water, 30 mL/min) to give compound 9250 (4.20 mg, 18% yield) as a white solid.

¹H NMR (400 MHz, DMSO-*d₆*) δ 10.55 (s, 1H), 7.72 (s, 1H), 7.72 - 7.63 (m, 3H), 7.43 (d, *J* = 8.8 Hz, 2H), 4.55 - 4.50 (m, 1H), 4.27 (s, 2H), 4.06 - 4.00 (m, 2H), 2.51 (s, 3H), 2.40 (s, 3H), 1.62 (s, 3H). LC-MS: (ESI) m/z [M+H] ⁺ 497.1.

### Example1-25

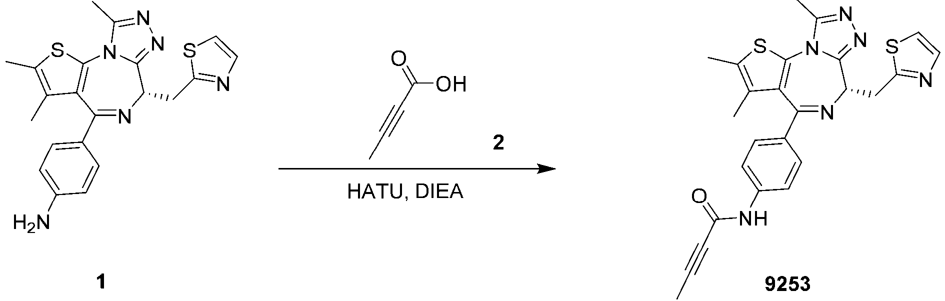

To a solution of compound 1 (20.0 mg, 0.0476 mmol) in DMF(2.0 mL) was added but-2-ynoic acid (6.00 mg, 0.0714 mmol), DIPEA (18.5 mg, 0.143 mmol) and HATU (27.2 mg, 0.0714 mmol) at 25 °C. The reaction mixture was stirred at 25 °C for 1 h. LCMS showed the starting material was consumed and desired product was formed. The mixture was quenched with water (20 mL) and then extracted with ethyl acetate (20 mL x 2). The combined organic phases were washed with an aqueous solution of NaCl (10 mL x 2) and dried over Na2SO4, filtered, and concentrated under reduce pressure. The residue was purified by Prep-HPLC (Chromatographic columns:-Xbridge-C18 150 x 19 mm,5um Mobile Phase: ACN-H₂O, 45-75 % MeCN in 0.1% FA/water, 30 mL/min) to give compound 9253 (5.70 mg, 24% yield) as a white solid.

¹H NMR (400 MHz, DMSO-*d₆*) δ 10.82 (s, 1H), 7.72 (s, 1H), 7.71 - 7.62 (m, 3H), 7.40 (d, *J* = 8.8 Hz, 2H), 4.54 - 4.51 (m, 1H), 4.07 - 4.00 (m, 2H), 2.51 (s, 3H), 2.40 (s, 3H), 2.04 (s, 3H), 1.62 (s, 3H). LC-MS: (ESI) m/z [M+H] ⁺ 487.1.

### Example1-26

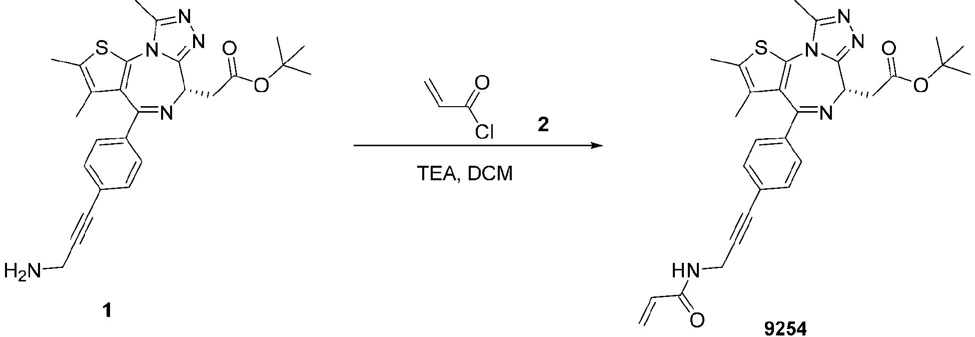

To a solution of compound 1 (20.0 mg, 0.0421 mmol) in DCM (3.0 mL) was added prop-2-enoyl chloride (5.72 mg, 0.0631 mmol) and TEA (12.8 mg, 0.126 mmol) at 25 °C. Then the mixture was stirred at 25 °C for 10 min. LCMS showed the starting material was consumed and desired product was detected. The mixture was quenched with water (20 mL) and then extracted with ethyl acetate (20 mL x 2). The combined organic phases were dried over sodium sulfate and filtered. The filtrate was concentrated under reduced pressure. The residue was purified by Prep-HPLC (Chromatographic columns:-Xbridge-C18 150 × 19 mm,5um Mobile Phase: ACN-H₂O, 45-75 % MeCN in 0.1% FA/water, 30 mL/min) to give compound 9254 (7.60 mg, 34% yield) as a white solid.

¹H NMR (400 MHz, DMSO-*d₆*) δ 8.66 (t, *J* = 5.2 Hz, 1H), 7.47 (d, *J =* 8.4 Hz, 2H), 7.41 (d, *J =* 8.4 Hz, 2H), 6.26 - 6.11 (m, 2H), 5.63 (d, *J =* 2.4 Hz, 1H), 4.44 - 4.40 (m, 1H), 2.22 (d, *J =* 5.3 Hz, 2H), 2.31 - 2.26 (m, 2H), 2.60 (s, 3H), 2.41 (s, 3H), 1.62 (s, 3H), 1.42 (s, 9H). LC-MS: (ESI) m/z [M+H] ⁺ 530.2.

### Example1-27

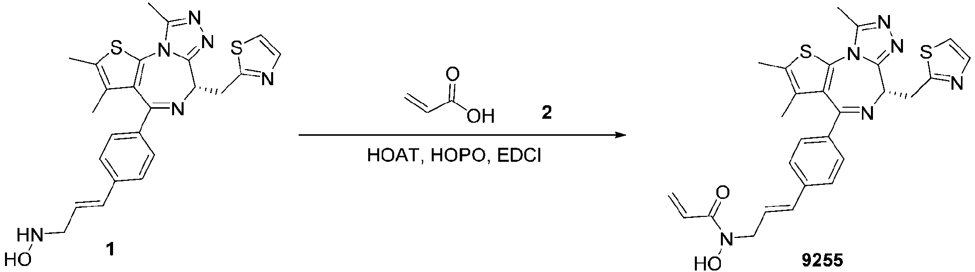

To a solution of compound 1 (100 mg, 0.210 mmol) in DMF (3.0 mL) was added prop-2-enoic acid (30.2 mg, 0.420 mmol), HOAt (3.43 mg, 0.0251 mmol), HOPO (23.3 mg, 0.210 mmol) and N-Ethylmorpholine (72.5 mg, 0.629 mmol) at 25 °C. After stirred for 5 min, EDCI (52.3 mg, 0.273 mmol) was added at 25 °C. Then the mixture was stirred at 25 °C for 55 min. LCMS showed the starting material was consumed and desired product was detected. The mixture was quenched with water (20 mL) and then extracted with ethyl acetate (20 mL x 2). The combined organic phases were dried over sodium sulfate and filtered. The filtrate was concentrated under reduced pressure. The residue was purified by Prep-HPLC (Chromatographic columns:-Xbridge-C18 150 × 19 mm,5um Mobile Phase : ACN-H₂O, 45-75 % MeCN in 0.1% FA/water, 30 mL/min) to give compound 9255 (8.50 mg, 8% yield) as a white solid.

¹H NMR (400 MHz, DMSO-*d₆*) δ 9.69 (d, *J* = 7.6 Hz, 1H), 7.73 - 7.72 (m, 3H), 7.67 - 7.64 (m, 2H), 7.52 - 7.50 (m, 2H), 7.45 - 7.42 (m, 1H), 7.41 - 7.39 (m, 1H), 6.92 (dd, *J* = 7.6, 15.6 Hz, 1H), 4.60 - 4.53 (m, 2H), 4.10 - 4.07 (m, 3H), 2.62 (s, 3H), 2.41 (s, 3H), 1.61 (s, 3H). LC-MS: (ESI) m/z [M+H] ⁺ 531.1.

### Example1-28

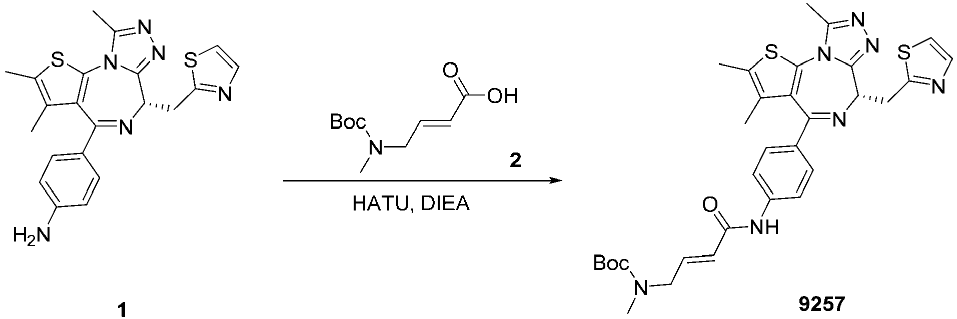

To a solution of compound 1 (50.2.0 mg, 0.232 mmol), DIPEA (59.9 mg, 0.232 mmol) and HATU (88.2 mg, 0.232 mmol) at 25 °C. The reaction mixture was stirred at 25 °C for 16 h. LCMS showed the starting material was consumed and desired product was formed. The mixture was quenched with water (20 mL) and then extracted with ethyl acetate (20 mL × 2). The combined organic phases were washed with an aqueous solution of NaCl (10 mL × 2) and dried over Na₂SO₄, filtered, and concentrated under reduce pressure. The residue was purified by Prep-HPLC (Chromatographic columns:-Xbridge-C18 150 × 19 mm,5um Mobile Phase: ACN-H₂O, 45-75 % MeCN in 0.1% FA/water, 30 mL/min) to give compound 9257 (10.5 mg, 22% yield) as a white solid.

¹H NMR (400 MHz, DMSO-*d₆*) δ 10.17 (d, *J =* 24.0 Hz, 1H), 7.72 (d, *J =* 3.6 Hz, 1H), 7.65 - 7.62 (m, 3H), 7.42 - 7.39 (m, 2H), 6.94 (br, 0.4 H), 6.32 (br, 0.6 H), 5.12 - 4.95 (m, 1H), 4.50 - 4.49 (m, 1H), 4.09 - 3.99 (m, 2H), 3.25 (s, 1H), 3.07 - 2.95 (m, 4H), 2.51 (s, 3H), 2.40 (s, 3H), 1.62 (s, 3H), 1.42 (s, 9H). LC-MS: (ESI) m/z [M+H] ⁺ 618.2.

### Example1-29

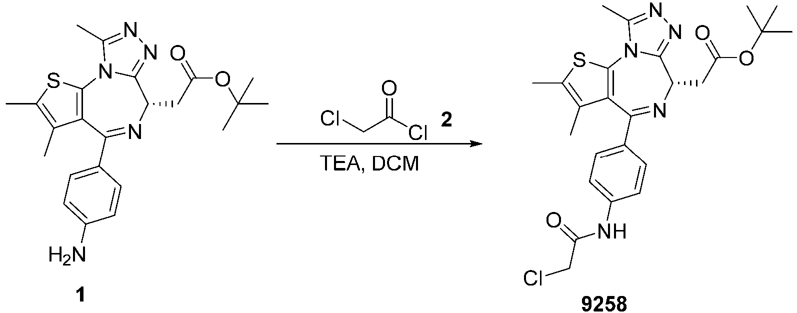

To a solution of compound 1 (80 mg, 0.1828 mol)and TEA (73.99 mg, 0.7312 mmol) in DCM (2 mL) was drop-wised added 2-chloroacetyl chloride (30.97 mg, 0.2742 mmol) at 0 °C. The mixture was stirred at 25°C for 7h. LCMS showed that raw material was consumed and the desired product mass was detected. The mixture was concentrated to dryness. The residue was purified by Prep-HPLC (Chromatographic columns:WELCH Xtimate C18 21.2*250mm 10 um, Mobile Phase: ACN-H₂O(0.1%FA),Gradient:50-50) to give compound 9258 (30.8 mg, 32% yield) as a white solid.

¹H NMR (400 MHz, DMSO-*d₆*) δ 10.52(s, 1H), 7.63 (d, *J =* 9.2 Hz, 2H), 7.41 (d, *J =* 8.4 Hz, 2H), 4.40 - 4.36 (m, 1H), 4.27 (s, 2H), 3.33 - 3.29 (m, 2H), 2.51 (s, 3H), 2.42 (s, 3H), 1.66 (s, 3H), 1.46 (s, 9H). LC-MS: (ESI) m/z [M+H] + 514.1.

### Example1-30

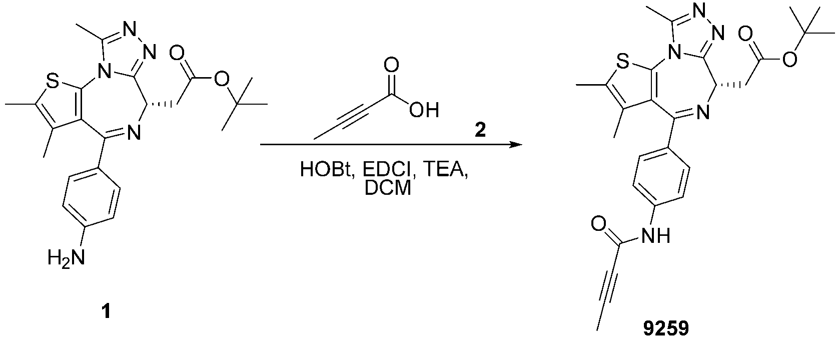

To a solution of compound 1 (55 mg, 0.1257 mol), but-2-ynoic acid (15.85 mg, 0.18855 mmol) and pyridine (149.14 mg, 1.8855 mmol) in DCM (2 mL) was drop-wised added POCl₃ (38.55 mg, 0.2514 mmol) at 0 °C. The mixture was stirred at 25 °C for 1 h. LCMS showed that raw material was consumed and the desired product mass was detected. The mixture was concentrated to dryness. The residue was purified by Prep-HPLC (Chromatographic columns: WELCH Xtimate C18 21.2*250mm 10um, Mobile Phase: ACN-H₂O (0.1%FA)Gradient:45-55) to give compound 9259 (15.6 mg, 24% yield) as a white solid.

¹H NMR (400 MHz, DMSO-*d₆*) δ 10.83 (s, 1H), 7.63 (d, *J =* 8.8 Hz, 2H), 7.37 (d, *J* = 8.8 Hz, 2H), 4.42 - 4.37 (m, 1H), 3.33 - 3.29 (m, 2H), 2.51 (s, 3H), 2.42 (s, 3H), 2.05 (s, 3H), 1.65 (s, 3H), 1.45 (s, 9H). LC-MS: (ESI) m/z [M+H] + 504.2.

### Example1-31

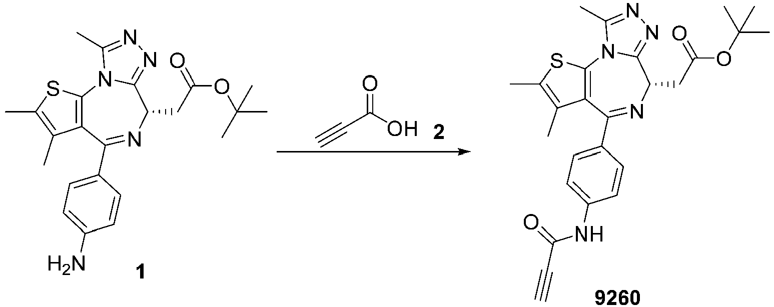

To a solution of compound 1 (60 mg, 0.1371 mol), prop-2-ynoic acid (14.41 mg, 0.2056 mmol) and pyridine (162.67 mg, 2.0564 mmol) in DCM (2 mL) was drop-wised added POCl₃ (42.04 mg, 0.2742 mmol) at 0 °C. The mixture was stirred at 25 °C for 0.5 h. LCMS showed that raw material was consumed and the desired product mass was detected. The mixture was concentrated to dryness. The residue was purified by Prep-HPLC (Chromatographic columns: WELCH Xtimate C18 21.2*250mm 10um, Mobile Phase: ACN-H₂O (0.1% FA) Gradient: 47-53) to give compound 9260 (14.2 mg, 21% yield) as a yellow solid.

¹H NMR (400 MHz, DMSO-*d₆*) δ 11.02 (s, 1H), 7.63 (d, *J=* 8.8 Hz, 2H), 7.38 (d, *J* = 8.4 Hz, 2H), 4.46 (s, 1H), 4.41 - 4.36 (m, 1H), 3.33 - 3.28 (m, 2H), 2.50 (s, 3H), 2.42 (s, 3H), 1.65 (s, 3H), 1.48 (s, 9H). LC-MS: (ESI) m/z [M+H] + 490.2.

### Example1-32

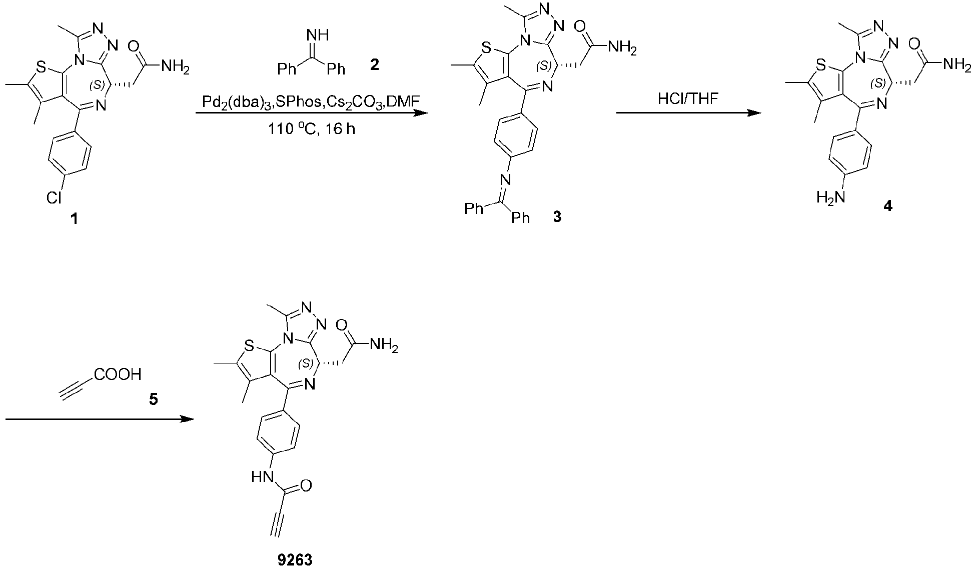

To a solution of compound 1 (500 mg, 1.2503 mmol) in DMF (5 mL) was added compound 2 (226.60 mg, 1.2503 mmol), Pd₂(dba)₃ (114.49 mg, 0.12503 mmol), SPhos (102.77 mg, 0.25006 mmol) and CAESIUM CARBONATE (1222.12 mg, 3.75 mmol). The reaction mixture was stirred at 110 °C for 12 h. LCMS showed desired product was formed. The reaction mixture was quenched with H₂O(10 mL), extracted with EtOAc(10 mL*3), the organic layer was dried over anhydrous Na₂SO₄, filtered and concentrated in vacuo, the residue was purified by silica chromatography eluting with MeOH in DCM from 0% to 5% to give compound 3 (60 mg, 3%) as a yellow oil. LC-MS: (ESI) m/z [M+H]⁺ 545.2.

Compound 3 (60 mg, 0.1102 mmol) was dissolved in THF (1 mL) and 2M/HCl (1 mL). The reaction mixture was stirred at 25 °C for 1 h. LCMS showed desired product was formed. The reaction mixture was quenched with NaHCO₃ solution (5 mL), extracted with EtOAc(10 mL*3), the organic layer was dried over anhydrous Na₂SO₄, filtered and concentrated in vacuo to give compound 4 (40 mg, 29%) as a yellow oil, which would be used directly without any further purification.

LC-MS: (ESI) m/z [M+H]⁺ 381.1.

To a solution of compound 4 (40 mg, 0.1051 mmol), propiolic acid (11.04 mg, 0.15765 mmol), pyridine (16.63 mg, 0.2102 mmol) in DCM (3 mL) was added POCl₃ (32.23 mg, 0.2102 mmol) at 0 °C. The reaction mixture was stirred at 25 °C for 30 min. LCMS showed desired product was formed. The reaction mixture was concentrated in vacuo, the residue was purified by Pre-HPLC (Chromatographic columns:-Xbridge-C18 150 x 19 mm,5um Mobile Phase:ACN-H₂O(0.1%FA))(0-100%) to give compound 9263 (4.6 mg,10%) as a white.

¹H NMR (400 MHz, DMSO-*d₆*-25 °C) δ 11.03 (s, 1H), 7.63 (d, *J* = 8.8 Hz, 3H), 7.39 (d, *J* = 8.6 Hz, 2H), 6.97 (s, 1H), 4.46 (d, *J* = 1.8 Hz, 2H), 3.21 (d, *J* = 7.2 Hz, 2H), 2.59 (s, 3H), 2.41 (s, 3H), 1.64 (s, 3H).

¹H NMR (400 MHz, DMSO-*d*₆-80 °C) δ 10.72 (s, 1H), 7.61 (d, *J* = 8.6 Hz, 2H), 7.39 (d, *J* = 8.6 Hz, 2H), 6.67 (s, 2H), 4.47 (t, *J* = 6.8 Hz, 1H), 4.22 (s, 1H), 3.33 - 3.17 (m, 2H), 2.58 (s, 3H), 2.41 (s, 3H), 1.66 (s, 3H). LC-MS: (ESI) m/z[M+H] + 433.1.

### Example1-33

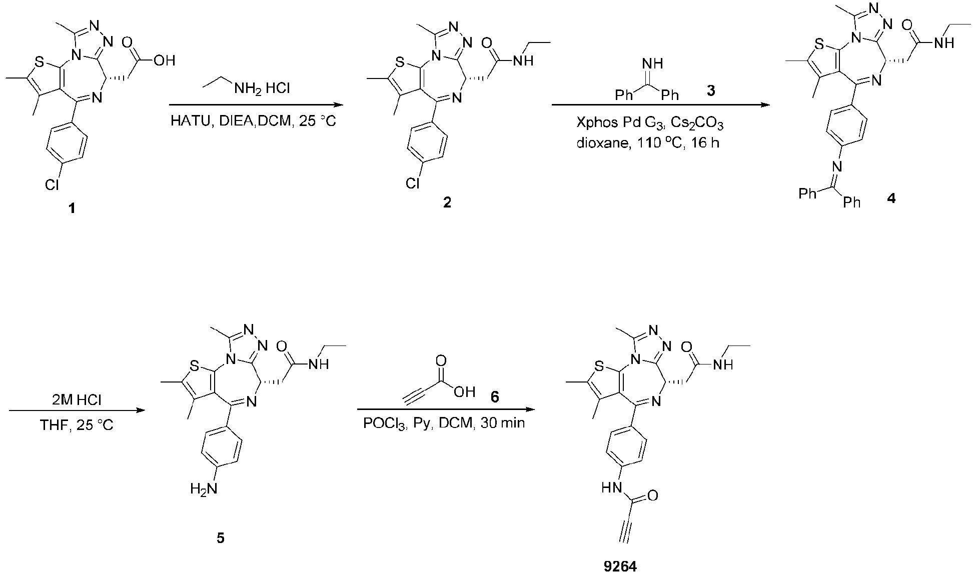

To a solution of compound 1 (300 mg, 0.748 mmol) in DCM (5.0 mL) was added ethylamine hydrochloride (183 mg, 2.245 mmol), DIEA (483 mg, 3.742 mmol) and HATU (341 mg, 0.898 mmol). The reaction mixture was stirred at 25 °C for 1 h. The reaction mixture was quenched with water (10 mL), extracted with DCM (10 mL × 3), the organic phases were dried over anhydrous Na₂SO₄, filtered, and concentrated in vacuo, the residue was purified by silica gel chromatography eluting with MeOH in DCM from 0% to 5% to afford compound 2 (320 mg, 95% yield) as a white solid.

LC-MS: (ESI) *m*/*z* [M+H]⁺ 428.1.

To a solution of compound 2 (340 mg, 0.795 mmol) in dioxane (3.0 mL) were added compound 3 (216 mg, 1.19 mmol), Cs₂CO₃ (776 mg, 2.38 mmol) and Xphos Pd G3 (67 mg, 0.0794 mmol). The reaction mixture was stirred at 110 °C for 16 h under N₂ atmosphere in seal tube. The mixture was quenched with water (10 mL) and extracted with EtOAc (10 mL × 3). The combined organic phases were dried over anhydrous Na₂SO₄, filtered, and concentrated in vacuo. The residue was purified by silica gel chromatography eluting with MeOH in DCM from 0 to 5% to afford compound 4 (280 mg, 61% yield) as a yellow solid.

LC-MS: (ESI) *m*/*z* [M+H]⁺ 573.2.

To a solution of compound 4 (280 mg) in THF (2.0 mL) was added 2M HCl (2.0 mL). The reaction mixture was stirred at 25 °C for 1 h. The mixture was quenched with NaHCO₃ solution (5 mL) and then extracted with DCM (10 mL × 3). The combined organic phases were dried over anhydrous Na₂SO₄ and filtered. The filtrate was concentrated in vacuo. The residue was purified by silica gel chromatography eluting with EtOAc in petroleum from 0% to 80% to afford compound 5 (120 mg, 60% yield) as a yellow solid.

LC-MS: (ESI) *m*/*z* [M+H]⁺ 409.2.

To a solution of compound 5 (120 mg, 0.294 mmol) in DCM (2.0 mL) were added prop-2-ynoic acid (31 mg, 0.441 mmol), pyridine (35 mg, 0.441 mmol) and POCl₃ (90 mg, 0.587 mmol) at 0 °C. The reaction mixture was stirred at 25 °C for 30 min. The mixture was in vacuo. The residue was purified by Prep-HPLC (Chromatographic columns:-Xbridge-C18 150 × 19 mm,5um Mobile Phase : ACN-H₂O, 23-55 % MeCN in 0.1% FA/water, 30 mL/min) to afford compound 6 (28.5 mg, 21% yield) as a yellow solid.

¹H NMR (400 MHz, DMSO-*d₆*) δ 11.01 (s, 1H), 8.19 (t, *J* = 5.2 Hz, 1H), 7.64 (d, *J* = 8.4 Hz, 2H), 7.38 (d, *J* = 8.4 Hz, 2H), 4.53 - 4.38 (m, 2H), 3.21 - 3.09 (m, 4H), 2.59 (s, 3H), 2.41 (s, 3H), 1.64 (s, 3H), 1.06 (t, *J* = 7.2 Hz, 3H). LC-MS: (ESI) *m*/*z* [M+H]⁺ 461.2.

### Example1-34

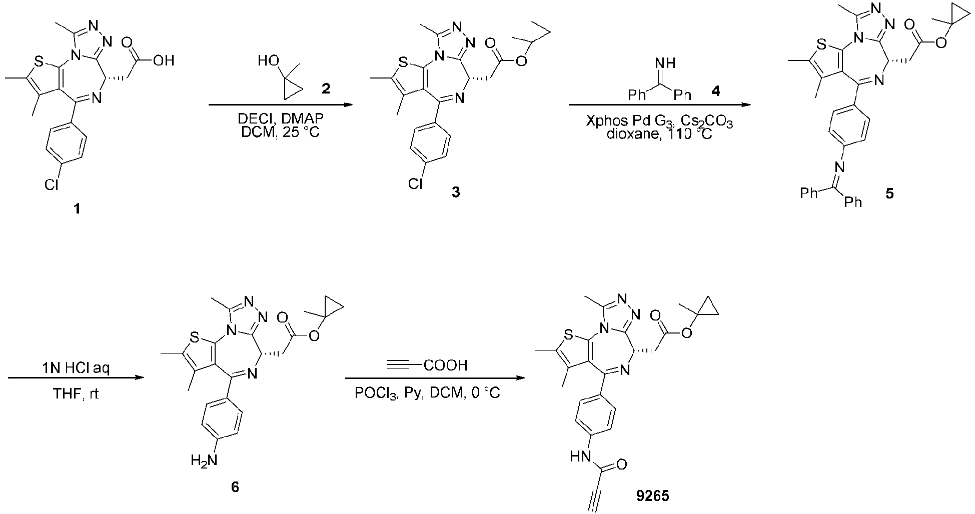

To a solution of compound 1 (400 mg, 0.998 mmol) in DCM (5.0 mL) were added EDCI (248 mg, 1.30 mmol), DMAP (61 mg, 0.499 mmol) and compound 2 (142 mg, 1.99 mmol). The reaction mixture was stirred at 25 °C for 16 h. The mixture was concentrated in vacuo. The residue was purified by silica gel chromatography eluting with 0 - 70% EtOAc in Petroleum ether to afford compound 3 (360 mg, yield: 79%) as a white solid.

LC-MS: (ESI) *m*/*z* [M+H]⁺ 455.1.

To a solution of compound 3 (200 mg, 0.440 mmol) in dioxane (2.0 mL) were added compound 4 (119 mg, 0.660 mmol), Xphos Pd G3 (37 mg, 0.0440 mmol) and Cs₂CO₃ (286 mg, 0.879 mmol) in seal tube. The reaction mixture was stirred at 110 °C under N₂ atmosphere for 16 h. The mixture was concentrated in vacuo. The residue was purified by silica gel chromatography eluting with 0 - 60% EtOAc in Petroleum ether to afford compound 5 (160 mg, yield: 36%) as a yellow oil.

LC-MS: (ESI) *m*/*z* [M+H]⁺ 600.2.

To a solution of compound 5 (160 mg, 0.267 mmol) in THF (2.0 mL) was added 1N HCl aq. (2.0 mL). The reaction mixture was stirred at 25 °C for 1 h. The mixture was quenched with NaHCO₃ solution (5 mL) and then extracted with DCM (10 mL × 3). The combined organic phases were dried over anhydrous Na₂SO₄ and filtered. The filtrate was concentrated in vacuo. The residue was purified by silica gel chromatography eluting with EtOAc in petroleum from 0% to 65% to afford compound 6 (60 mg, 51% yield) as a yellow solid.

LC-MS: (ESI) *m*/*z* [M+H]⁺ 436.1.

To a solution of compound 6 (50 mg, 0.115 mmol) in DCM (2.0 mL) were added prop-2-ynoic acid (12 mg, 0.172 mmol), pyridine (13 mg, 0.172 mmol) and POCl₃ (35 mg, 0.230 mmol) at 0 °C. The mixture was stirred at 0 °C for 30 min. The mixture was concentrated in vacuo. The residue was purified by Prep-HPLC (Chromatographic columns:-WELCH Xtimate C18 21.2*250mm 10um; Mobile Phase : ACN-H₂O, 40-50% MeCN in 0.1% NH₃/water, 30 mL/min) to afford compound 9265 (21.7 mg, 38% yield) as a white solid.

¹H NMR (400 MHz, DMSO-*d₆*) δ 11.01 (s, 1H), 7.64 (d, *J* = 8.8 Hz, 2H), 7.37 (d, *J* = 8.4 Hz, 2H), 4.49 - 4.38 (m, 2H), 3.34 - 3.33 (m, 2H), 2.63 - 2.57 (m, 3H), 2.42 (s, 3H), 1.65 (s, 3H), 1.50 - 1.47 (m, 3H), 0.82 - 0.76 (m, 2H), 0.69 - 0.63 (m, 2H), LC-MS: (ESI) *m*/*z* [M+H]⁺ 488.1.

### Example 2-1

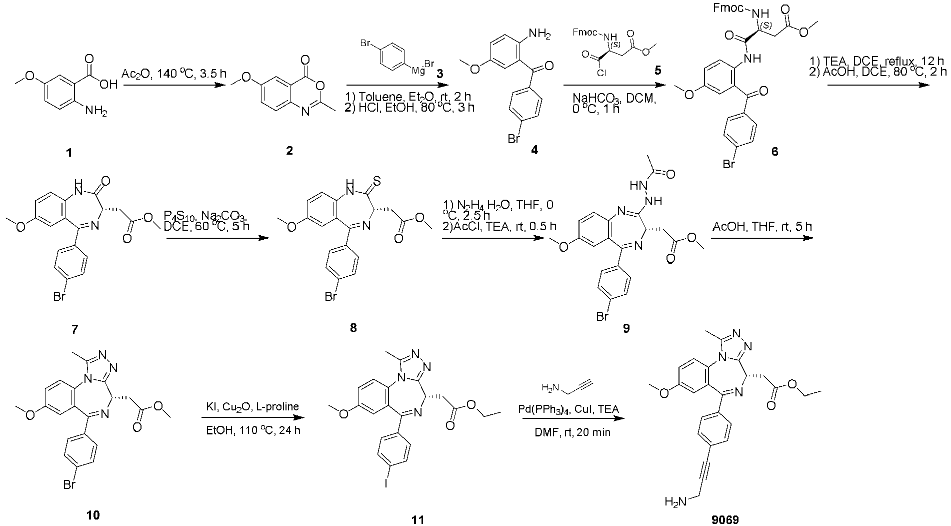

A solution of compound 1 (25.0 g, 0.15 mol) in Ac₂O (140 mL) was stirred at 140 °C for 3.5 h. The resulting mixture was concentrated under reduced pressure, and the residue was ground with Et₂O to give compound 2 (23.0 g, crude) as a brown solid.

To a solution of compound 2 (20.0 g, 104.60 mmol) in PhMe/Et₂O (200 mL + 100 mL) was added a solution of compound 3 (83.6 mL, 83.60 mmol, 1 M) in THF dropwise at 0 °C under N₂ atmosphere. The reaction mixture was stirred at room temperature for 2 h. The reaction mixture was diluted with 1 M HCl and extracted with EA (100 mL × 2). The combined organic phases were concentrated under reduced pressure to give a residue, which was dissolved in a solution of ethanol (30 mL) and concentrated hydrochloric acid (30 mL). The solution was heated to 80 °C and stirred at 80 °C for 3 h. The reaction mixture was cooled to room temperature and concentrated under vacuum, and the residue was partitioned between DCM and a 4 N NaOH solution. The aqueous layer was re-extracted with DCM (100 mL × 2). The combined organic phases were dried over anhydrous Na₂SO₄, filtered, and concentrated. The crude product was purified by silica gel column chromatography to give compound 4 (6.0 g, 17.78% yield) as a yellow oil.

Compound 4 (12.0 g, 39.20 mmol), compound 5 (16.8 g, 43.10 mmol), and NaHCO₃ (6.6 g, 78.40 mmol) were dissolved in DCM (120 mL), and the solution was stirred at 0 °C for 1 h. The resulting mixture was extracted with DCM (100 mL × 2). The organic layer was collected, washed twice with saturated brine, and dried over Na₂SO₄. The organic layer was concentrated under reduced pressure to give compound 6 (24.0 g, crude) as a yellow oil.

A solution of compound 6 (24.0 g, 36.40 mmol) and TEA (14.7 g, 145.60 mmol) in DCE (40 mL) was stirred and heated to reflux for 3 h. The resulting mixture was concentrated to dryness. The crude product was dissolved in DCE (240 mL), and then AcOH (23.7 mL) was added. The mixture was stirred at 80 °C for 2 h. The resulting mixture was extracted with DCM (100 mL × 3). The organic layer was collected, washed twice with saturated brine, and dried over Na₂SO₄. The organic layer was concentrated under reduced pressure to give a residue. The residue was purified by column chromatography (SiO₂, DCM/MeOH = 98/2) to give compound 7 (9.0 g, 20.50 mmol, 56.3% yield) as an off-white solid.

Compound 7 (6.0 g, 14.40 mmol), P₄S₁₀ (5.8 g, 25.90 mmol), and sodium carbonate (2.8 g, 25.90 mmol) were dissolved in DCE (60 mL), and the solution was stirred at 60 °C for 5 h. The resulting mixture was extracted with DCM (100 mL × 2). The organic layer was collected, washed twice with saturated brine and a NaHCO₃ solution, respectively, and dried over Na₂SO₄. The organic layer was concentrated under reduced pressure to give a residue. The residue was purified by column chromatography (SiO₂, DCM = 100%) to give compound 8 (3.0 g, 6.60 mmol, 45.8% yield) as a yellow solid.

To a solution of compound 8 (1.2 g, 2.80 mmol) in THF (12 mL) was added hydrazine hydrate (0.4 g, 8.40 mmol), and the mixture was stirred at 0 °C for 2.5 h. To the solution was added TEA (0.9 g, 8.40 mmol), and the reaction mixture was cooled to 0 °C. Then, acetyl chloride (0.7 g, 8.40 mmol) was added, and the mixture was stirred at 0 °C for 30 min. The reaction mixture was diluted with water and DCM and separated. The aqueous layer was re-extracted with DCM (10 mL × 3). The combined organic fraction was dried over anhydrous Na₂SO₄, filtered, and concentrated under vacuum to give compound 9 (1.0 g, 1.90 mmol, 67.9% yield) as a yellow solid.

Compound 9 (1.0 g, 1.90 mmol), THF (10 mL), and AcOH (10 mL) were mixed, and the mixture was stirred at room temperature for 5 h. The reaction mixture was diluted with water and DCM and separated. The aqueous layer was re-extracted with DCM (20 mL × 3). The combined organic fraction was dried over anhydrous Na₂SO₄. The organic layer was concentrated under reduced pressure to give a residue. The residue was purified by column chromatography (SiO₂, DCM/MeOH = 96/4) to give compound 10 (700 g, 1.40 mmol, 66.7% yield) as a white solid.

Compound 10 (100 mg, 0.22 mmol), potassium iodide (109 mg, 0.66 mmol), Cu₂O (3 mg, 0.02 mmol), and pyrrolidine-2-carboxylic acid (5 mg, 0.04 mmol) were mixed in EtOH (1.5 mL). The mixture was heated and stirred at 110 °C for 24 h under N₂ atmosphere. The mixture was extracted with ethyl acetate (10 mL × 3) and evaporated under vacuum. The residue was purified by column chromatography (DCM/MeOH = 50/1) to give compound 11 (65 mg, 53% yield) as a yellow solid.

Compound 11 (30 mg, 0.06 mmol), prop-2-yn-1-amine (4 mg, 0.07 mmol), Pd(PPh₃)₄ (13 mg, 0.01 mmol), CuI (2 mg, 0.01 mmol), and triethylamine (18 mg, 0.17 mmol) were mixed in DMF (1 mL). The mixture was stirred at room temperature for 20 min under N₂ atmosphere. The mixture was diluted with MeOH, filtered and purified by prep-HPLC (column: Phenomenex luna C18 250 mm × 100 mm × 10 µm; mobile phase: [H₂O (0.1% FA)-ACN]; B%: 15%-55%, 40 min) to give 9069 (5.8 mg, 23% yield) as a white solid.
**LCMS:** [M+H] ⁺ = 444.1;
**¹H NMR:** (400 MHz, MeOD) δ 7.744 (d, J = 9.0 Hz, 1H), 7.535 (d, J = 8.4 Hz, 2H), 7.483 (d, J = 8.4 Hz, 2H), 7.386 (dd, J = 9.0, 2.8 Hz, 1H), 6.905 (d, J = 2.8 Hz, 1H), 4.636 - 4.581 (m, 1H), 4.196 (q, J = 7.2 Hz, 2H), 3.911 (s, 2H), 3.827 (s, 3H), 3.501 (dd, J = 7.2, 4.4 Hz, 2H), 2.631 (s, 3H), 1.287 (t, J = 7.2 Hz, 3H).
**HPLC:** 98.98% purity;
**SFC:** Rt = 3.932 min.

### Example 2-2

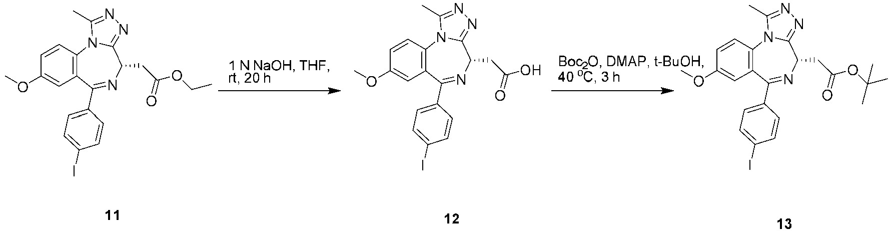

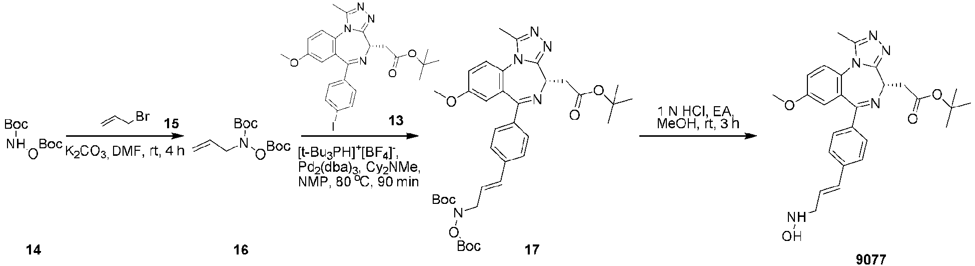

Compound 11 (400 mg, 0.796 mmol) and an aqueous NaOH solution (10 mL, 2 N) were dissolved in THF (10 mL), and the mixture was stirred at room temperature for 20 h. The solution was adjusted to about pH 5 with a 2 N HCl solution and extracted with DCM (20 mL × 3). The organic layer was dried over Na₂SO₄ and concentrated to give compound 12 (300 mg, 0.615 mmol, 90% yield).

To a solution of compound 12 (300 mg, 0.615 mmol) in t-BuOH (3 mL) was added Boc₂O (781 mg, 3.584 mmol) and DMAP (18 mg, 0.123 mmol). The mixture was stirred at 40 °C for 3 h. The reaction liquid was diluted with water (20 mL) and extracted with EA (20 mL × 3). The resulting organic layer was dried over Na₂SO₄ and concentrated to give a crude product. The crude product was purified by FCC (DCM/MeOH = 50:1) to give compound 13 (260 mg, 0.382 mmol, 53.3% yield) as a yellow solid.

To a solution of compound 14 (1 g, 4.292 mmol) in DMF (10 mL) was added potassium carbonate (0.77 g, 5.579 mmol) and compound 15 (0.94 g, 7.836 mmol). The mixture was stirred at room temperature for 4 h. The solution was diluted with water (100 mL) and extracted with ethyl acetate (100 mL × 3). The organic layer was washed with a saturated aqueous sodium chloride solution (50 mL × 5), dried over Na₂SO₄, and concentrated under reduced pressure. The residue was purified by FCC (eluent: PE/EA = 95/5) to give compound 16 (0.6 g, 46.5% yield) as a colorless oil.

Compound 16 (36 mg, 0.132 mmol), compound 13 (60 mg, 0.110 mmol), [*t*-Bu₃PH]⁺[BF₄]⁻ (64 mg, 0.220 mmol), Pd₂(dba)₃ (20 mg, 0.022 mmol), and Cy₂NMe (43 mg, 0.220 mmol) were dissolved in NMP (1 mL). The mixture was stirred under microwave irradiation at 80 °C for 90 min under N₂ atmosphere. Then, the mixture was filtered, concentrated, and diluted with water (10 mL). The resulting solution was extracted with EtOAc (10 mL × 3). The combined organic layer was dried over anhydrous Na₂SO₄ and concentrated. The residue was subjected to flash silica gel chromatography (eluent: DCM/MeOH = 95/5) to give compound 17 (25 mg, 31.2% yield) as a white solid.

To a solution of compound 17 (25 mg, 0.036 mmol) in MeOH (1 mL) was added a solution of HCl in EA (2 N, 1 mL). The solution was stirred at room temperature for 3 h. The solution was diluted with MeOH (2 mL) and dried with nitrogen. The crude was dissolved in MeOH (2 mL) and purified by prep-HPLC (column: Phenomenex luna C18 250 mm × 100 mm × 10 µm; mobile phase: [H₂O (0.1% FA)-ACN]; B%: 5%-40%, 40 min) to give 9077 (5.9 mg, 32.1% yield) as a white solid.
**LCMS:** [M+H] ⁺ = 490.2;
**¹H NMR:** (400 MHz, MeOD) δ 8.529 (s, 0.79 H), 7.721 (d, *J =* 9.2 Hz, 1H), 7.476 (q, *J* = 8.4 Hz, 4H), 7.380 (dd, *J* = 9.2, 2.8 Hz, 1H), 6.934 (d, *J* = 2.8 Hz, 1H), 6.649 (d, *J* = 16.0 Hz, 1H), 6.439 (dt, *J* = 16.0, 6.4 Hz, 1H), 4.543 (dd, *J* = 8.4, 6.0 Hz, 1H), 3.821 (s, 3H), 3.635 (d, *J* = 6.4 Hz, 2H), 3.502 - 3.364 (m, 2H), 2.636 (s, 3H), 1.486 (s, 9H). **HPLC:** 99.37% purity;
**SFC:** Rt = 9.747 min.

### Example 2-3

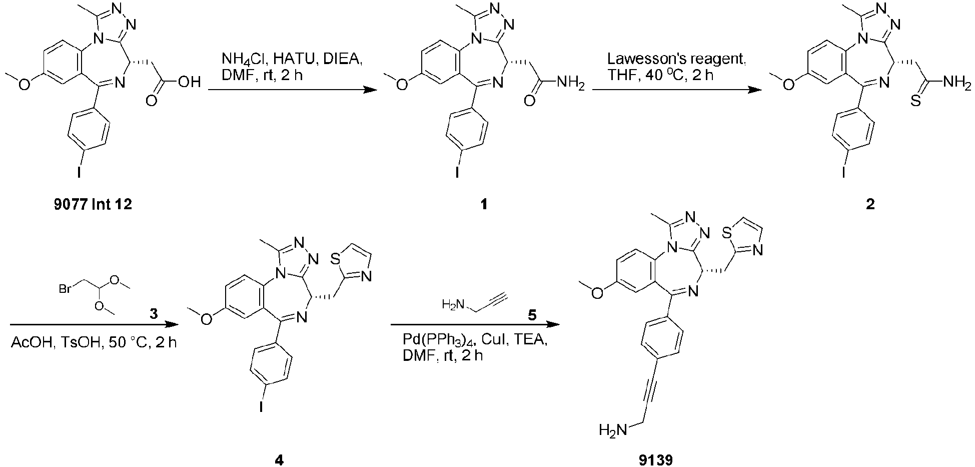

To a solution of 9077 Int 12 (1.0 g, 0.002 mol) in DMF (10 mL) were added DIEA (0.78 g, 0.006 mol), HATU (1.14 g, 0.003 mmol), and NH₄Cl (0.16 g, 0.003 mmol). The resulting mixture was stirred at room temperature for 2 h. The crude product was purified directly by flash silica gel column chromatography (DCM/MeOH = 5/1) to give compound 1 (0.8 g, 80%) as a yellow solid.

Compound 1 (0.8 g, 0.002 mol) and Lawesson's reagent (2 mg, 0.004 mol) were mixed in THF (10 mL), and the mixture was stirred at 40 °C for 3 h. The solvent was removed under reduced pressure. The resulting crude product was purified directly by flash silica gel column chromatography (DCM/MeOH = 8/1) to give compound 2 (0.6 g, 75%) as a yellow oil.

Compound 2 (500 mg, 0.993 mmol), compound 3 (336 mg, 1.987 mmol), and TsOH (34 mg, 0.199 mmol) were dissolved in AcOH (10 mL), and the mixture was heated and stirred at 50 °C for 2 h. The crude product was purified directly by flash silica gel column chromatography (DCM/MeOH = 10/1) to give compound 4 (400 mg, 75.6%) as a yellow oil.

To a solution of compound 4 (200 mg, 0.379 mmol) in DMF (5 mL) were added Pd(PPh₃)₄ (88 mg, 0.076 mmol), copper(I) iodide (14 mg, 0.076 mmol), compound 5 (31 mg, 0.569 mmol), and TEA (115 mg, 1.138 mmol). The reaction mixture was stirred at room temperature for 2 h under N₂ atmosphere. The solution was filtered and purified directly by prep-HPLC (column: Phenomenex luna C18 250 mm × 100 mm × 10 µm; mobile phase: [H₂O (0.1% FA)-ACN]; B%: 0%-540%, 40 min) to give 9139 (25 mg, 9.91%) as a brown solid.
**LCMS:** [M+H] ⁺ = 455.2;
**¹H NMR:** (400 MHz, d₄-MeOD) δ 7.749 - 7.726 (m, 2H), 7.586 - 7.494 (m, 5H), 7.389 (d, J = 3.6 Hz, 1H), 6.876 (s,
1H), 4.646 (s, 1H), 4.085 (m, 4H), 3.808 (s, 3H), 2.646 (s, 3H).
**HPLC:** 95.13% purity.

### Example 2-4

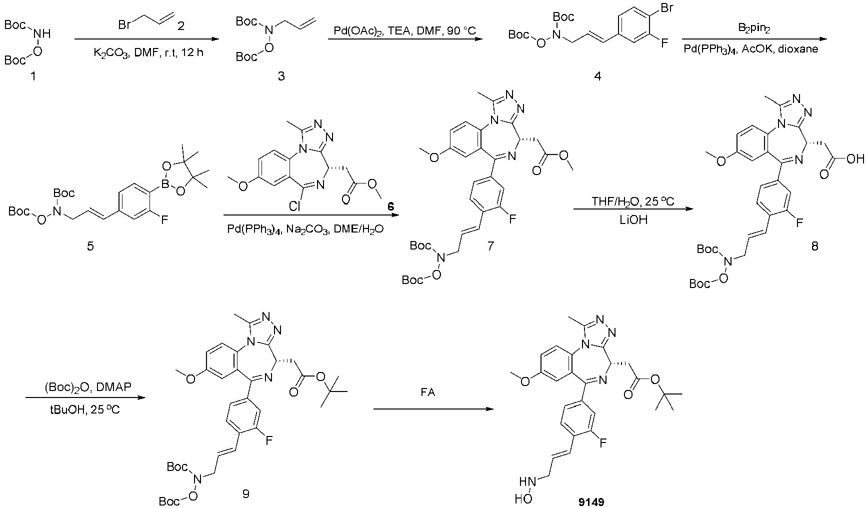

To a solution of compound 1 (15.0 g, 63.8 mmol) in DMF (100 mL) were added K₂CO₃ (10.9 g, 76.5 mmol) and compound 2 (15.4 g, 128 mmol). The reaction mixture was stirred at 20°C for 12 h. The mixture was diluted with water (100 mL) and extracted with EtOAc (150 mL × 2). The organic layer was washed with a saturated NaCl solution (200 mL), dried over anhydrous Na₂SO₄, filtered, and concentrated under reduced pressure. The residue was purified by flash column chromatography (120 g of silica gel column, PE/EtOAc and EtOAc, from 0% to 10%) to give compound 3 (17.1 g, 88% yield) as a colorless oil. LC-MS: (ESI) m/z [m+Na]+ 296.4.

To a solution of compound 3 (6.00 g, 21.8 mmol) and 1-bromo-2-fluoro-4-iodobenzene (6.56 g, 21.78 mmol) in DMF (25 mL) were added Pd(OAc)₂ (726 mg, 4.36 mmol) and TEA (6.61 g, 65.4 mmol). The reaction mixture was stirred at 90 °C for 5 h under N₂ atmosphere. The mixture was diluted with DCM (100 mL), washed with brine, dried over anhydrous Na₂SO₄, filtered, and concentrated under reduced pressure. The residue was purified by flash column chromatography (120 g of silica gel column, PE/EtOAc and EtOAc, from 0% to 20%) to give compound 4 (3.6 g, 33% yield) as a yellow gum.

To a solution of compound 4 (400 mg, 0.892 mmol) and bis(pinacolato)diboron (453 mg, 1.78 mmol) in dioxane (13 mL) were added Pd(PPh₃)₄ (103 mg, 0.0892 mmol) and potassium acetate (263 mg, 2.68 mmol). The reaction mixture was stirred at 100 °C for 4 h to give a reaction liquid containing compound 5. The reaction liquid was used in the next step without further purification. LC-MS: (ESI) m/z [m+Na]⁺ 516.4.

To a solution of compound 6 (100 mg, 0.298 mmol) and compound 5 (229 mg, 0.597 mmol) in dioxane (12 mL) and H₂O (0.2 mL) were added Pd(PPh₃)₄ (34.5 mg, 0.0298 mmol) and Na₂CO₃ (94.9 mg, 0.896 mmol). The reaction mixture was stirred at 100 °C for 5 h under N₂ atmosphere. The mixture was concentrated under reduced pressure. The residue was purified by flash column chromatography (25 g of silica gel column, DCM/MeOH and MeOH, from 0% to 5%) to give compound 7 (120 mg, 42% yield) as a yellow solid. LC-MS: (ESI) m/z [m+H]⁺ 666.4.

To a solution of compound 7 (120 mg, 0.180 mmol) in THF (5 mL) and H₂O (2 mL) was added LiOH (43.0 mg, 1.80 mmol). The reaction mixture was stirred at 25 °C for 12 h. The mixture was added to EA (10 mL), and the mixture was extracted with water (40 mL). Then, the aqueous layer was acidified with an aqueous HCl solution (1 M) until pH = 6-7. The mixture was washed with EtOAc (40 mL × 3). The organic layer was dried over anhydrous Na₂SO₄. The mixture was purified by concentration under reduced pressure to give compound 8 (70 mg, 54% yield) as a yellow solid. LC-MS: (ESI) m/z [m+H]⁺ 552.0.

To a solution of compound 8 (70 mg, 0.107 mmol) and Boc₂O (117 mg, 0.536 mmol) in t-BuOH (3 mL) was added 4-DMAP (13.1 mg, 0.107 mmol). The reaction mixture was stirred at 25 °C for 3 h. The mixture was purified by concentration under reduced pressure. The mixture was purified by TLC (DCM/MeOH=20/1) to give compound 9 (45 mg, 53% yield) as a yellow solid.

A solution of compound 9 (30 mg, 0.0423 mmol) in FA (2 mL) was stirred at 25 °C for 2 h. The mixture was subjected to Prep HPLC (column: -Xbridge-C18 150 × 19 mm, 5 µm, mobile phase: ACN/H₂O (0.1% FA), gradient: 25%-35%) to give compound 9149 (4.3 mg, yield 20%) as a white solid.

¹H NMR (400 MHz, DMSO) δ 7.81 (d, J = 8.8 Hz, 1H), 7.45 (t, J = 8.0 Hz, 1H), 7.40 - 7.33 (m, 2H), 7.25 (d, J = 12.0 Hz, 1H), 6.79 (d, J = 2.8 Hz, 1H), 6.61 - 6.43 (m, 2H), 4.47 - 4.40 (m, 1H), 3.76 (s, 3H), 3.50 (d, J = 5.6 Hz, 3H), 3.33 - 3.30 (m, 2H), 2.53 (s, 3H), 1.43 (s, 9H). ¹⁹F NMR (376 MHz, DMSO) δ -113.33. LC-MS: (ESI) m/z [M+H]⁺ 508.2.

### Example 2-5

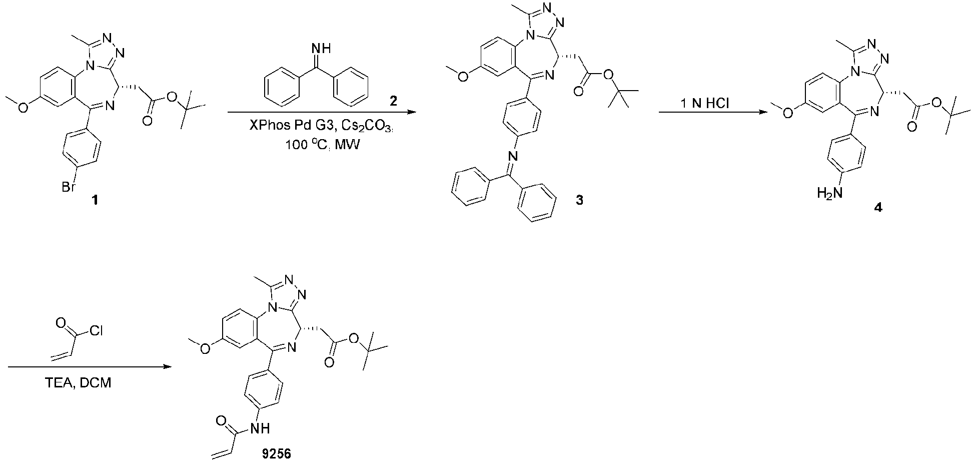

To a solution of compound 1 (200 mg, 0.603mmol) in DMF (4 mL) was added compound 2(109 mg, 0.603 mmol), Cs₂CO₃ (197 mg, 0.603 mmol), PD₂(DBA)₃ (18.4 mg, 0.0201 mmol) and SPhos (16.5 mg, 0.0402 mmol) at 100 °C. The reaction mixture was irradiated in a microwave reactor at 100 °C for 1 h. The reaction mixture was added to water (20 mL). The aqueous phase was extracted with ethyl acetate (50 mL). The combined organic phases were washed with an aqueous solution of NaCl (15 mL x 2) and dried over Na₂SO₄, filtered, and concentrated under reduce pressure. The residue was purified by silica gel chromatography eluting with EtOAc in petroleum from 0% to 50% to give compound 3 (110 mg, 44%yield) as a yellow solid.

LC-MS: (ESI) m/z [M+H] ⁺ 589.2.

To a stirred solution of compound 3 (110 mg, 0.184 mmol) in THF (5.0 mL) was added 1 N HCl (1.0 mL) dropwise at 25 °C. The resulting mixture was stirred for 30 min at 25 °C. The mixture was quenched with NaHCO₃ solution (20 mL) and then extracted with ethyl acetate (20 mL x 2). The combined organic phases were dried over sodium sulfate and filtered. The filtrate was concentrated under reduced pressure. The residue was purified by silica gel chromatography eluting with THF in petroleum from 0% to 80% to give compound 4 (61.0 mg, 75% yield) as a yellow solid.

LC-MS: (ESI) m/z [M+H] ⁺ 434.2.

To a solution of compound 4 (61.0 mg, 0.141 mmol) in DCM (4.0 mL) was added prop-2-enoyl chloride (19.1 mg, 0.211 mmol) and TEA (42.7 mg, 0.422 mmol) at 25 °C. The reaction mixture was stirred at 25 °C for 10 min. The mixture was quenched with water (20 mL) and then extracted with DCM (20 mL x 2). The combined organic phases were dried over sodium sulfate and filtered. The residue was purified by Prep-HPLC (Chromatographic columns:-Xbridge-C18 150 x 19 mm,5um Mobile Phase : ACN-H₂O, 45-75 % MeCN in 0.1% FA/water, 30 mL/min) to give compound 9256 (31.6 mg, 45% yield) as a white solid.

¹H NMR (400 MHz, DMSO-*d₆*) δ 10.37 (s, 1H), 7.79 (d, *J* = 8.8 Hz, 1H), 7.71 (d, *J* = 8.8 Hz, 2H), 7.49 (d, *J* = 8.8 Hz, 2H), 7.38 (dd, *J* = 8.8, 2.8 Hz, 1H), 6.90 (d, *J* = 2.8 Hz, 1H), 6.50 - 6.40 (m, 1H), 6.28 (dd, *J* = 17.2, 2.0 Hz, 1H), 5.78 (dd, *J* = 10.0, 2.0 Hz, 1H), 4.44 - 4.33 (m, 1H), 3.80 (s, 3H), 3.34 - 3.24 (m, 2H), 2.54 (s, 3H), 1.43 (s, 9H). LC-MS: (ESI) m/z [M+H] ⁺ 488.2.

### Example 3-1

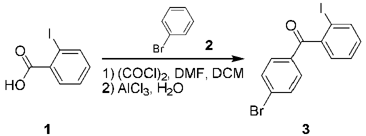

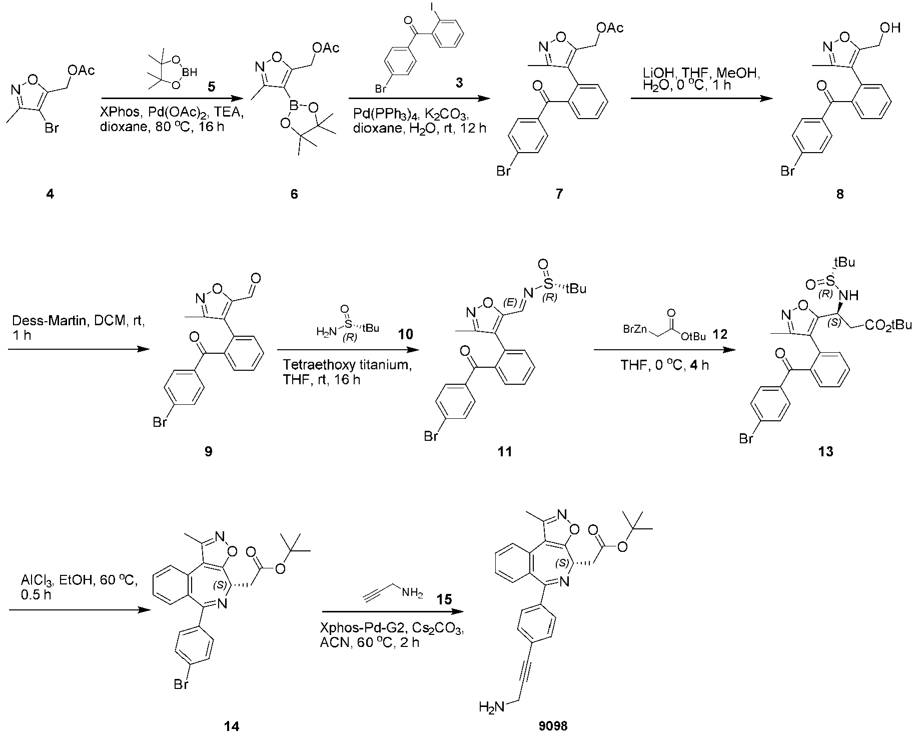

To a solution of compound 1 (25 g, 100 mmol) in DCM (50 mL) was added oxalyl chloride (19.2 g, 151 mmol). The mixture was stirred at room temperature for 5 min, and then DMF (3 drops) was added. The resulting mixture was stirred at room temperature for 2 h. The solvent was removed under reduced pressure, and the resulting residue was redispersed in compound 2 (150 mL). Then, anhydrous AlCl₃ (20.2 g, 151.2 mmol) was added at 0 °C. The mixture was stirred at room temperature for 16 h. The reaction was quenched with ice-cold water, and the reaction liquid was extracted with CHCl₃ (200 mL × 3). The organic layer was dried over Na₂SO₄, and concentrated. The residue was purified by FCC (PE/EA = 20/1) to give compound 3 (20 g, 41% yield) as a white solid.

Compound 4 (10 g, 42.70 mmol), compound 5 (8.2 g, 64.05 mmol), XPhos (2.04 g, 4.27 mmol), Pd(OAc)₂ (0.48 g, 2.14 mmol), and TEA (12.96 g, 128.10 mmol) were mixed in dioxane (50 mL). The mixture was stirred and heated at 80 °C for 16 h under N₂ atmosphere. After the reaction was completed, the reaction solution was cooled to room temperature and filtered. The filtrate was concentrated to give compound 6 (20 g, crude) as a brown oil.

Compound 6 (3 g, 0.011 mmol), compound 3 (4.56 g, 0.012 mmol), Pd(PPh₃)₄ (1.24 g, 0.001 mmol), and K₂CO₃ (4.44 g, 0.032 mmol) were mixed in dioxane/H₂O (5:1, 60 mL). The mixture was stirred at room temperature for 12 h under N₂ atmosphere. The reaction liquid was diluted with EtOAc, filtered, extracted, and concentrated. The crude product was purified by FCC (EA/PE = 10%) to give compound 7 (1.6 g, 32.7%) as a yellow solid.

To compound 7 (1.6 g 0.004 mmol) in THF/MeOH/H₂O (10 mL/10 mL/5 mL) was added LiOH·H₂O (0.28 g, 0.012 mmol) at 0 °C. The mixture solution was stirred at 0 °C for 1 h. Then, the reaction was quenched with water, and the reaction liquid was extracted with EtOAc. The combined organic phases were washed with brine, dried over Na₂SO₄, filtered, and concentrated. The solvent was removed under reduced pressure to give compound 8 (1.4 g, crude) as a yellow solid.

A solution of compound 8 (700 mg, 1.881 mmol) and Dess-Martin periodinane (1595 mg, 3.761 mmol) in DCM (10 mL) was stirred at room temperature for 1 h. The solvent was removed under vacuum, and the residue was purified by FCC (EA/PE = 10%) to give compound 9 (600 mg, 77.57%) as a yellow oil.

To a solution of compound 9 (600 mg, 1.621 mmol) in THF (6 mL) were added tetraethyl titanate (739 mg, 3.242 mmol) and compound 10 (216 mg, 1.783 mmol). The resulting mixture was stirred at room temperature for 16 h. Then, the mixture was diluted with brine (10 mL) and ethyl acetate (10 mL). The resulting mixture was stirred vigorously at room temperature for 1.5 h. The mixture was filtered, and the organic layer was isolated. The aqueous solution was extracted with EtOAc. The combined organic layer was dried over Na₂SO₄, filtered, and concentrated. The residue was purified by FCC (EA/PE = 10%) to give compound 11 (480 mg, 50%) as a yellow solid.

To a solution of compound 11 (400 mg, 0.845 mmol) in THF (5 mL) was added a solution of compound 12 (330 mg, 1.268 mmol) in THF (5 mL) at 0 °C over 10 min. The resulting mixture was stirred at 0 °C for 4 h. The reaction was quenched with saturated aqueous NH₄Cl and EA solutions. The organic layer was isolated, and the aqueous phase was extracted with EA. The combined organic layer was washed with brine, dried over Na₂SO₄, filtered, and concentrated under reduced pressure. The residue was purified by FCC (typically gradient elution, 5% MTBE:95% hexane to 50% MTBE:50% hexane) to give compound 13 (100 mg, 20%) as a white solid.

Compound 13 (100 mg, 0.169 mmol) and AlCl₃ (34 mg, 0.254 mmol) were dissolved in EtOH (10 mL). The solution was heated at 60 °C for 30 min under N₂ atmosphere. Then, the solution was cooled to room temperature. The reaction was quenched with a NaHCO₃ solution, and the reaction liquid was extracted with EA. The combined organic layer was washed with brine, dried over Na₂SO₄, filtered, and concentrated under reduced pressure. The residue was purified by FCC (EA/PE = 10%) to give compound 14 (50 mg, 56.78% yield, 90% purity) as a yellow solid. Compound 14 (50 mg, 0.107 mmol), compound 15 (12 mg, 0.214 mmol), XPhos-Pd-G3 (17 mg, 0.021 mmol), and Cs₂CO₃ (70 mg, 0.214 mmol) were mixed in ACN (3 mL). The mixture was heated and stirred at 60 °C for 2 h under N₂ atmosphere. The solvent was removed under reduced pressure. The residue was extracted with EA (10 mL × 3). The solvent was removed. The residue was purified by prep-HPLC (column: Phenomenex luna C18 250 mm × 100 mm × 10 µm; mobile phase: [H₂O (0.1% FA)-ACN]; B%: 10%-55%, 40 min) to give 9098 (10.4 mg, 20.3% yield) as a white solid.
**LCMS:** [M+H] ⁺ = 442.1;
**¹H NMR:** (400 MHz, MeOD) δ 8.543 (s, 0.12H), 7.810 (d, J = 7.6 Hz, 1H), 7.712 (d, J = 4.0 Hz, 1H), 7.436 - 7.372 (m, 4H), 7.306 (d, J = 8.4 Hz, 2H), 4.385 (s, 1H), 3.648 (s, 2H), 3.319 (s, 2H), 2.564 (s, 3H), 1.484 (s, 9H). **HPLC:** 97.9% purity.

### Example 3-2

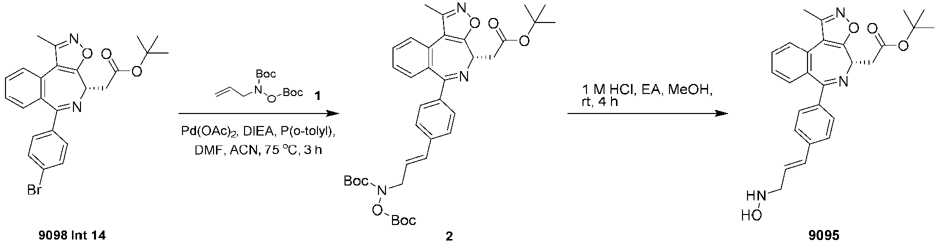

9098 Int 14 (50 mg, 0.107 mmol), compound 1 (44 mg, 0.161 mmol), Pd(OAc)₂ (12 mg, 0.054 mmol), P(o-tolyl) (33 mg, 0.107 mmol), and DIEA (60 mg, 0.110 mmol) were mixed in DMF (1 mL) and ACN (1 mL). The mixture was stirred under microwave irradiation at 75 °C for 3 h under N₂ atmosphere. Then, the mixture was filtered, concentrated, and diluted with water (10 mL). The resulting solution was extracted with EtOAc (10 mL × 3). The combined organic layer was dried over anhydrous Na₂SO₄ and concentrated. The residue was purified by FCC (eluent: PE/EtOAc = 90/10) to give compound 2 (30 mg, 42% yield) as a colorless oil.

A solution of compound 2 (30 mg, 0.045 mmol) in 1 M HCl in MeOH/EA (3 mL/3 mL) was stirred at room temperature for 4 h. The solution was diluted with MeOH (2 mL) and dried under N₂ flow. The crude product was dissolved with MeOH (2 mL) and purified by prep-HPLC (column: Phenomenex luna C18 250 mm × 100 mm × 10 µm; mobile phase: [H₂O (0.1% TFA)-ACN]; B%: 5%-45%, 40 min) to give 9095 (14.4 mg, 70% yield) as a yellow solid.
**LCMS:** [M+H] ⁺ = 460.1;
**¹H NMR:** (400 MHz, MeOD) δ 7.828 (d, J = 7.6 Hz, 1H), 7.729 (dd, J = 8.8, 4.0 Hz, 1H), 7.486 (d, J = 8.4 Hz, 2H), 7.445 - 7.402 (m, 2H), 7.369 (t, J = 7.6 Hz, 2H), 6.926 (d, J = 15.6 Hz, 1H), 6.612 - 6.263 (m, 1H), 4.412 (s, 1H), 4.011 (d, J = 7.2 Hz, 2H), 3.316 (s, 2H), 2.572 (s, 3H), 1.491 (s, 9H).
**HPLC:** 96.9% purity.

### Example 4-1

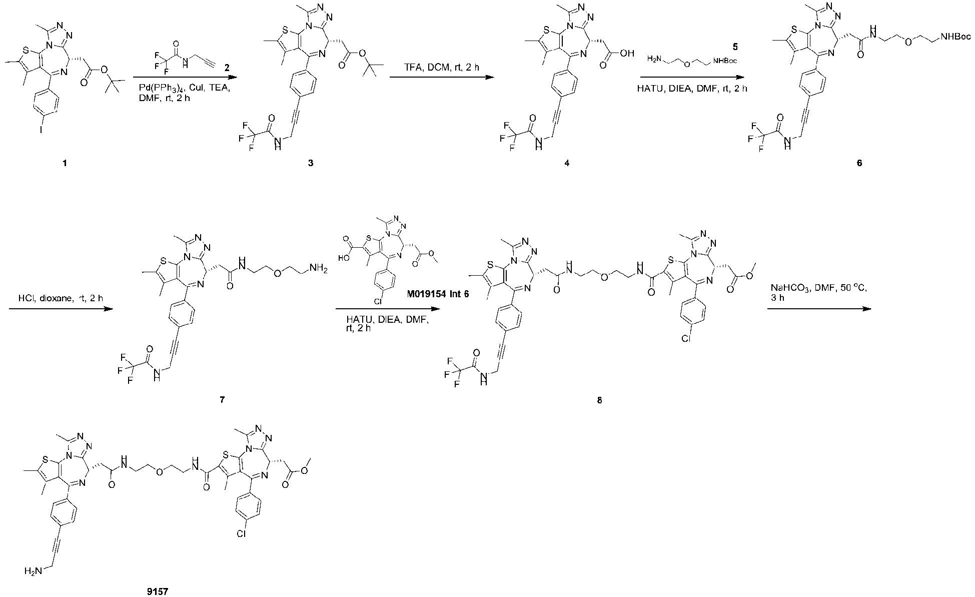

Compound 1 (500 mg, 0.912 mmol), compound 2 (207 mg, 1.367 mmol), Pd(PPh₃)₄ (211 mg, 0.182 mmol), CuI (35 mg, 0.182 mmol), and TEA (277 mg, 2.735 mmol) were dissolved in DMF (5 mL). The mixture was stirred at room temperature for 2 h. The mixture was diluted with water (30 mL) and extracted with EA (30 mL × 3). The combined organic layer was dried over Na₂SO₄ and concentrated. The crude product was purified by FCC (DCM/MeOH = 10:1) to give compound 3 (400 mg, 0.629 mmol, 69.08% yield) as a brown solid.

To a solution of compound 3 (400 mg, 0.699 mmol) in DCM (3 mL) was added TFA (3 mL). The reaction mixture was stirred at room temperature for 2 h. Then, the solvent was removed under reduced pressure to give a crude product. The crude product was purified by FCC (DCM/MeOH = 10:1) to give compound 4 (280 mg, 0.489 mmol, 69.85% yield) as a brown solid.

Compound 4 (280 mg, 0.543 mmol), compound 5 (133 mg, 0.652 mmol), HATU (310 mg, 0.815 mmol), and DIEA (211 mg, 1.629 mmol) were dissolved in DMF (5 mL). The solution was stirred at room temperature for 2 h. Then, the mixture was diluted with water (30 mL) and extracted with EtOAc (30 mL × 3). The combined organic layer was dried over Na₂SO₄ and concentrated. The crude product was purified by FCC (DCM/MeOH = 10:1) to give compound 6 (180 mg, 0.231 mmol, 42.49%yield) as a yellow solid.

Compound 6 (180 mg, 0.257 mmol) was dissolved in 1,4-dioxane (2 mL), and then a solution of HCl in 1,4-dioxane (2 mL, 1 M) was added. The reaction mixture was stirred at room temperature for 2 h. The solvent was removed under reduced pressure. The crude product was purified by FCC (DCM/MeOH = 10:1) to give compound 7 (150 mg, 0.224 mmol, 87.49% yield) as a yellow solid.

Compound 7 (150 mg, 0.249 mmol), 9154 Int 6 (111 mg, 0.249 mmol), HATU (114 mg, 0.299 mmol), and DIEA (97 mg, 0.748 mmol) were dissolved in DMF (5 mL). The solution was stirred at room temperature for 2 h. Then, the mixture was diluted with water (25 mL) and extracted with EtOAc (25 mL × 3). The combined organic layer was dried over Na₂SO₄ and concentrated. The crude product was purified by FCC (DCM/MeOH = 10:1) to give compound 8 (120 mg, 0.111 mmol, 44.44% yield) as a white solid.

Compound 8 (100 mg, 0.097 mmol) and NaHCO₃ (25 mg, 0.292 mmol) were dissolved in DMF (3 mL). The solution was heated at 50 °C for 3 h. The mixture was diluted with MeOH, filtered and purified by prep-HPLC (column: Phenomenex luna C18 250 mm × 100 mm × 10 µm; mobile phase: [H₂O (0.1% FA)-ACN]; B%: 20%-60%, 30 min) to give 9157 (24.4 mg, 0.0261 mmol, 27.01% yield) as a white solid.
**LCMS:** [M+H] ⁺ = 933.9;
**¹H NMR:** (400 MHz, *d₄*-MeOD) *δ* 7.427 - 7.325 (m, 8H), 4.610 (t, *J* = 7.2 Hz, 1H), 4.535 (t, *J* = 7.2 Hz, 1H), 4.038 (s, 2H), 3.729 (s, 3H), 3.729 - 3.430 (m, 12H), 2.683 (s, 3H), 2.612 (s, 3H), 2.431 (s, 3H), 2.009 (s, 3H), 1.617 (s, 3H).
**HPLC:** 99.28% purity.
**SFC:** Rt = 2.920 min.

The following compounds were prepared with reference to the above examples.

| | |
|---|---|
| 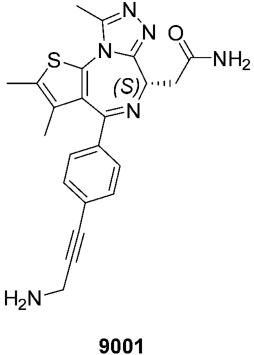 | **LCMS:** [M+H] ⁺ = 419.1; |
| | **¹H NMR:** ENB214152-029-2, (400 MHz, d₄-MeOD) δ 7.480 (s, 4H), 4.668 - 4.632 (m, 1H), 3.798 (s, 2H), 3.376 - 3.321 (m, 2H), 2.718 (s, 3H), 2.467 (s, 3H), 1.705 (s, 3H). |
| 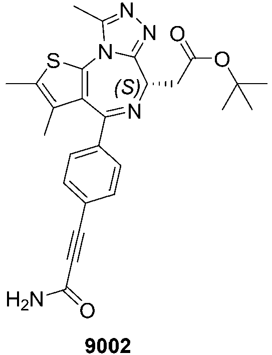 | **LCMS:** [M+H] ⁺ = 490.1; |
| | **¹H NMR:** ENB214035-092-1A, (400 MHz, CDCl₃) δ 7.553 - 7.495 (m, 4H), 5.840 (s, 1H), 5.646 (s, 1H), 4.611 (s, 1H), 3.567 (s, 2H), 2.747 (s, 3H), 2.426 (s, 3H), 1.682 (s, 3H), 1.503 (s, 9H). |
| 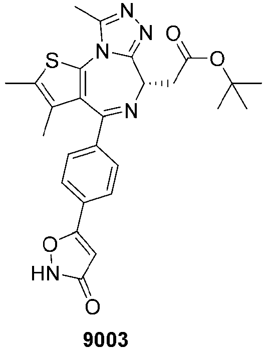 | **LCMS:** [M+H] ⁺ = 506.2; Rt = 1.074 min. |
| | **¹H NMR:** (400 MHz, d₄-MeOD) δ 7.789 - 7.768 (d, J = 8.4 Hz, 2H), 7.575 - 7.555 (d, J = 8 Hz, 2H), 6.203 (s, 1H), 4.596 (dd, J = 8.4, 6.2 Hz, 1H), 3.501 - 3.404 (m, 3H), 2.730 (s, 3H), 2.486 (s, 3H), 1.749 (s, 3H), 1.526 (s, 9H). |
| 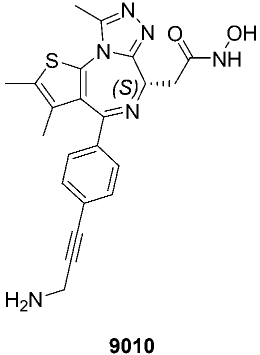 | **LCMS:** [M+H] ⁺ = 435.2. |
| | **¹H NMR:** (400 MHz, MeOD) δ 8.528 (s, 0.76H), 7.510 (s, 4H), 4.670 (dd, J = 9.6, 4.8 Hz, 1H), 3.949 (s, 2H), 3.337 - 3.329 (m, 1H), 3.206 - 3.169 (m, 1H), 2.716 (s, 3H), 2.468 (s, 3H), 1.696 (s, 3H). |
| 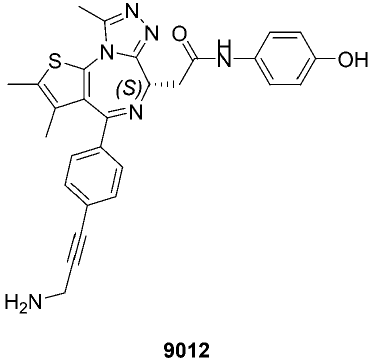 | **LCMS:** [M+H] ⁺ = 511.1; |
| | **¹H NMR:** (400 MHz, d₆-DMSO) |
| | δ 7.471 (s, 4H), 7.431 - 7.362 (m, 2H), 6.823 - 6.722 (m, 2H), 4.881 - 4.714 (m, 1H), 3.797 (s, 2H), 3.503 - 3.465 (m, 1H), 3.337 - 3.321 (m, 1H), 2.727 (s, 3H), 2.470 (s, 3H), 1.706 (s, 3H). **HPLC:** (214 nm 99.27% purity, 254 nm 99.25% purity) |
| 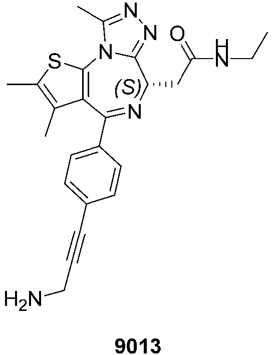 | **LCMS:** [M+H] ⁺ = 447.1; |
| | **¹H NMR:** (400 MHz, d₄-MeOD) δ 8.539 (s, 0.62H), 7.513 - 7.459 (m, 4H), 4.668 - 4.645 (m, 1H), 3.898 (s, 2H), 3.337 - 3.325 (m, 2H), 3,297 - 3.275 (m, 2H), 2.716 (s, 3H), 2.468 (s, 3H), 1.703 (s, 3H), 1.210 (t, J = 7.2 Hz, 3H). |
| 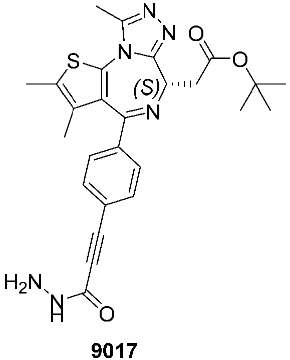 | **LCMS:** [M+H] ⁺ = 505.2; |
| | **¹H NMR:** (400 MHz, CDCl₃) δ 7.551 (dt, J = 26.0, 8.4 Hz, 4H), 4.609 - 4.574 (m, 1H), 3.569 (d, J = 7.6 Hz, 2H), 2.702 (s, 2H), 2.683 (d, 3H), 2.414 (s, 3H), 1.680 (d, 3H), 1.499 (t, 9H). |
| 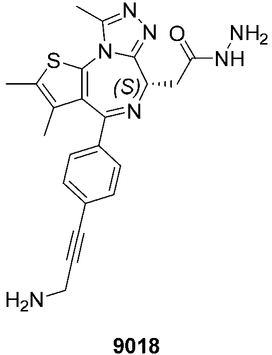 | **LCMS:** [M+H] ⁺ = 434.2; Rt = 0.59 min. |
| | **¹H NMR:** (400 MHz, d₆-DMSO) |
| | δ 9.321 (s, 1H), 8.182 (s, 1H), 7.462 - 7.401 (m, 4H), 4.522 (dd, J = 8.4, 5.6 Hz, 1H), 3.632 (d, J = 6.0 Hz, 3H), 3.105 (dd, J = 17.2, 11.6 Hz, 1H), 2.591 (s, 3H), 2.411 (s, 3H), 1.620 (s, 3H). |
| 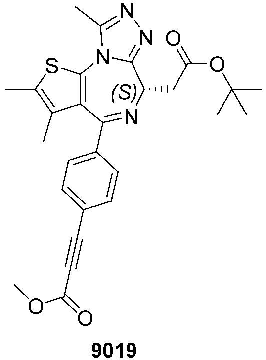 | **LCMS:** [M+H] ⁺ = 505.1; |
| | **¹H NMR:** (400 MHz, CDCl₃) |
| | δ 7.582 (d, J = 8.4 Hz, 2H), 7.494 (d, J = 8.2 Hz, 2H), 4.60 (t, 1H), 3.852 (s, 3H), 3.556 (d, J = 7.2 Hz, 2H), 2.750 (s, 3H), 2.424 (s, 3H), 1.682 (s, 3H), 1.502 (s, 9H). |
| 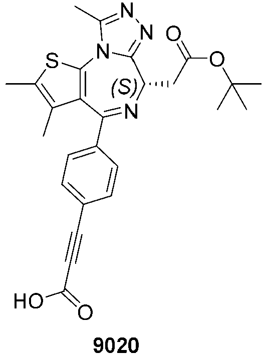 | **LCMS:** [M+H] ⁺ = 491.1; |
| | **¹H NMR:** (400 MHz, CDCl₃) |
| | δ 7.527 - 7.253 (m, 4H), 4.587 (s, 1H), 3.593 (s, 2H), 2.668 (s, 3H), 2.399 (s, 3H), 1.658 (s, 3H), 1.488 (s, 9H). |
| 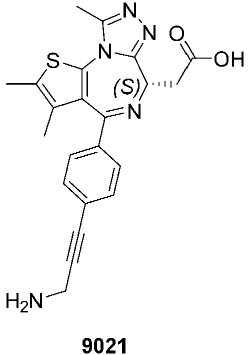 | **LCMS:** [M+H] ⁺ = 420.0; |
| | **¹H NMR:** (400 MHz, d₄-MeOD) |
| | δ 7.505 (m, 4H), 4.633 - 4.615 (m, 1H), 3.997 (s, 2H), 3.449 - 3.337 (m, 2H), 2.705 (s, 3H), 2.461 (s, 3H), 1.701 (s, 3H). |
| 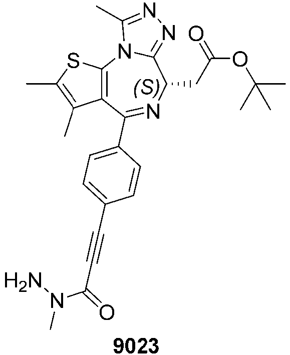 | **LCMS:** [M+H] ⁺ = 519.3; |
| | **¹H NMR:** (400 MHz, d₆-DMSO) |
| | δ 7.738 (d, J = 8.4 Hz, 2H), 7.750 (d, J = 8.0 Hz, 2H), 4.58 (m, 1H), 3.642 (s, 3H), 3.456 - 3.393 (m, 2H), 2.729 (s, 3H), 2.485 (s, 3H), 1.752 (s, 3H), 1.525 (s, 9H). |
| 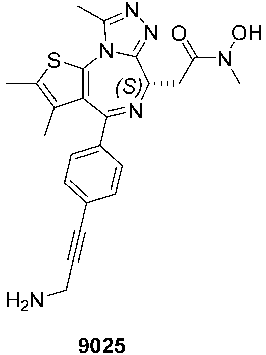 | **LCMS:** [M+H] ⁺ = 435.2; |
| | **¹H NMR:** (400 MHz, d₆-DMSO) |
| | δ 10.224 (s, 0.8H), 7.411 (q, J = 8.2 Hz, 4H), 4.516 (t, J = 6.8 Hz, 1H), 3.556 - 3.517 (m, 4H), 3.136 (s, 3H), 2.593 (s, 3H), 2.416 (s, 3H), 1.628 (s, 3H). |
| 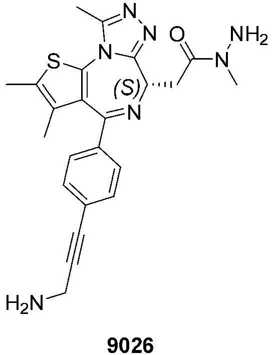 | **LCMS:** [M+H] ⁺ = 448.2; |
| | **¹H NMR:** (400 MHz, d₆-DMSO) |
| | δ 7.435 (s, 4 H), 4.645 (s, 1H), 3.975 (d, J = 8.4 Hz, 3H), 3.784 (s, 1H), 3.175(s, 3H), 2.638 (d, J = 8.0 Hz, 3H), 2.375 (d, J = 8.8 Hz, 3H), 1.594 (s, 3H). |
| 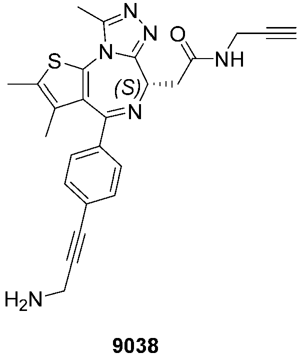 | **LCMS:** [M+H] ⁺ = 457.2; Rt = 0.709 min. |
| | **¹H NMR:** (400 MHz, d₄-MeOD) |
| | δ 7.508 (s, 4 H), 4.885 (d, J = 9.6 Hz, 1H), 4.685 - 4.601 (dd, J = 11.6 Hz, 1H), 4.045 (s, 1H), 3.904 (s, 2H), 3.532 - 3.502 (dd, J = 8.0 Hz, 1H), 3.335 (d, J = 7.6 Hz, 1H), 2.665 (s, 3H), 2.478 (d, J = 10.8 Hz, 1H), 2.215 (s, 3H), 1.705 (s, 3H). |
| 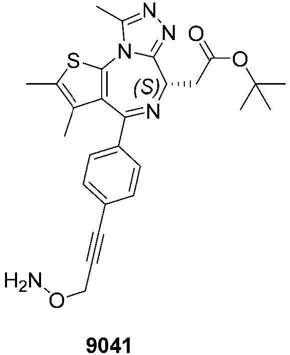 | **LCMS:** [M+H] ⁺ = 492.3; |
| | **¹H NMR:** (400 MHz, MeOD) |
| | δ 7.518 (d, J = 8.4 Hz, 2H), 7.464 (d, J = 8.4 Hz, 2H), 4.581 (dd, J = 8.4, 6.0 Hz, 1H), 4.520 (s, 2H), 3.468 - 3.432 (m, 2H), 2.720 (s, 3H), 2.479 (s, 3H), 1.726 (s, 3H), 1.517 (s, 9H). |
| 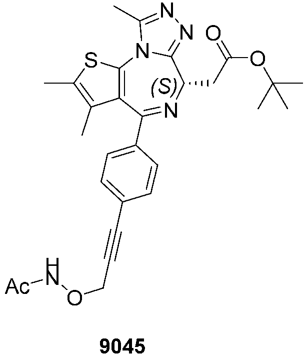 | **LCMS:** [M+H] ⁺ = 533.4; |
| | **¹H NMR:** (400 MHz, d₆-DMSO) |
| | δ 7.462 (q, J = 8.3 Hz, 4H), 4.582 (dd, J = 8.4, 6.0 Hz, 1H), 3.821 (s, 2H), 3.333 (t, J = 6.8 Hz, 2H), 2.721 (s, 3H), 2.481 (s, 3H), 1.961 (s, 3H), 1.722 (s, 3H), 1.522 (s, 9H). |
| 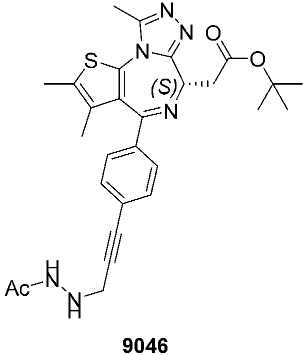 | **LCMS:** [M+H] ⁺ = 533.4; |
| | **¹H NMR:** (400 MHz, *d₆*-DMSO) |
| | δ 7.462 (q, *J* = 8.3 Hz, 4H), 4.582 (dd, *J* = 8.4, 6.0 Hz, 1H), 3.821 (s, 2H), 3.333 (t, *J* = 6.8 Hz, 2H), 2.721 (s, 3H), 2.481 (s, 3H), 1.961 (s, 3H), 1.722 (s, 3H), 1.522 (s, 9H). |
| 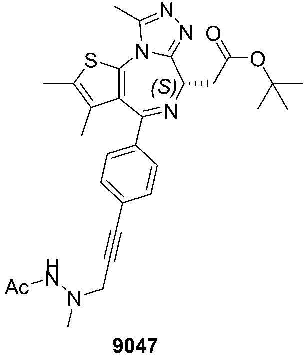 | **LCMS:** [M+H] ⁺ = 547.4; |
| | **¹H NMR:** (400 MHz, MeOD) |
| | δ 7.514 (d, J = 8.4 Hz, 2H), 7.459 (d, J = 8.4 Hz, 2H), 4.580 (dd, J = 8.4, 6.0 Hz, 1H), 3.791 (s, 2H), 3.478 - 3.422 (m, 2H), 2.719 (s, 3H), 2.672 (s, 3H), 2.478 (s, 3H), 1.924 (s, 3H), 1.723 (s, 3H), 1.516 (s, 9H). |
| 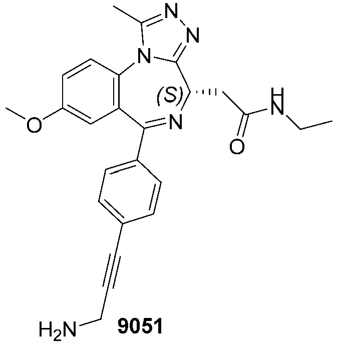 | **LCMS:** [M+H] ⁺ = 443.1; |
| | **¹H NMR:** (400 MHz, MeOD) |
| | δ 7.720 (d, J = 9.2 Hz, 1H), 7.553 (d, J = 8.4 Hz, 2H), 7.489 (d, J = 8.4 Hz, 2H), 7.380 (dd, J = 9.2, 2.8 Hz, 1H), 6.894 (d, J = 2.8 Hz, 1H), 4.635 (dd, J = 9.2, 5.2 Hz, 1H), 3.911 (br s, 2H), 3.819 (s, 3H), 3.395 (dd, J = 15.2, 9.2 Hz, 1H), 3.287 - 3.201 (m, 3H), 2.630 (s, 3H), 1.181 (t, J = 7.2 Hz, 3H). |
| 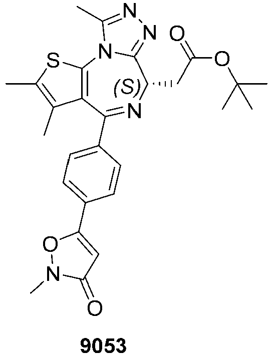 | **LC-MS:** (M+H) ⁺, 520.3; |
| | **¹H NMR:** (400 MHz, MeOD) |
| | δ 7.840 (d, J = 8.6 Hz, 2H), 7.622 (d, J = 8.4 Hz, 2H), 6.342 (s, 1H), 4.583 (s, 1H), 3.622 (s, 3H), 3.312 (dt, J = 3.2, 1.6 Hz, 2H), 2.702 (s, 3H), 2.463 (s, 3H), 1.711 (s, 3H), 1.503 (s, 9H). |
| 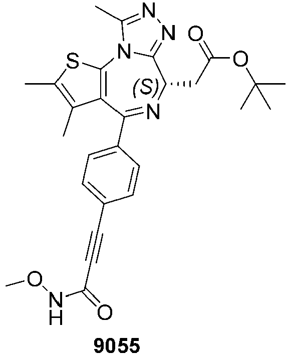 | **LCMS:** [M+H] ⁺ = 520.3; |
| | **¹H NMR:** (400 MHz, d₆-DMSO) |
| | δ 7.524 (d, J = 8.0 Hz, 2H), 7.435 (d, J = 8.0 Hz, 2H), 4.491 (m, 1H), 3.660 (s, 3H), 3.365 - 3.332 (m, 2H), 2.611 (s, 3H), 2.371 (s, 3H), 1.611 (s, 3H), 1.408 (s, 9H). |
| 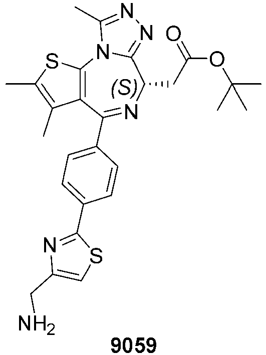 | **LCMS:** [M+H] ⁺ = 535.1; |
| | **¹H NMR:** (400 MHz, MeOD) |
| | δ 8.531 (s, 0.8H), 8.054 (d, J = 8.4 Hz, 2H), 7.632 (s, 1H), 7.585(d, J = 8.2 Hz, 2H), 4.593 (dd, J = 8.4, 6.2 Hz, 1H), 4.238 (s, 2H), 3.450 (t, J = 6.8 Hz, 2H), 2.710 (s, 3H), 2.471 (s, 3H), 1.745 (s, 3H), 1.507 (s, 9H). |
| 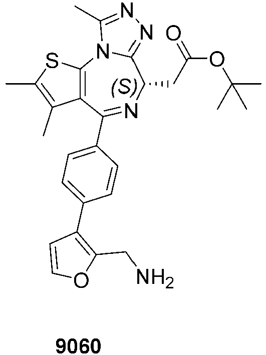 | **LCMS:** [M+H] ⁺ = 518.3; |
| | **¹H NMR:** (400 MHz, MeOD) |
| | δ 8.532 (s, 0.5H), 7.610 (d, J = 1.6 Hz, 1H), 7.514 (q, J = 8.5 Hz, 4H), 6.696 (d, J = 2.0 Hz, 1H), 4.593 - 4.556 (m, 1H), 4.111 (s, 2H), 3.459 - 3.417 (m, 2H), 2.706 (s, 3H), 2.466 (s, 3H), 1.742 (s, 3H), 1.503 (s, 9H). |
| 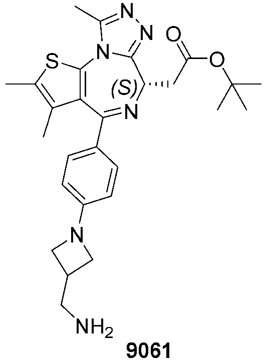 | **LCMS:** [M+H] ⁺ = 507.2; |
| | **¹H NMR:** (400 MHz, MeOD) |
| | δ 7.295 (d, J = 8.4 Hz, 2H), 6.437 (d, J = 8.8 Hz, 2H), 4.475 (dd, J = 8.4, 6.0 Hz, 1H), 4.069 (t, J = 8.0 Hz, 2H), 3.708 (dd, J = 8.0, 5.2 Hz, 2H), 3.410 - 3.361 (m, 2H), 3.248 (d, J = 7.6 Hz, 2H), 3.032 - 2.939 (m, 1H), 2.681 (s, 3H), 2.453 (s, 3H), 1.735 (s, 3H), 1.481 (s, 9H). |
| 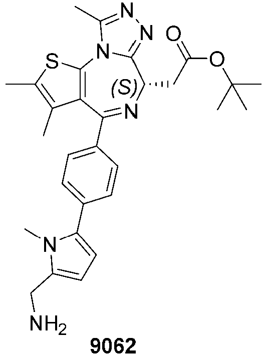 | **LCMS:** [M+H] ⁺ = 531.4; |
| | **¹H NMR:** (400 MHz, MeOD) |
| | δ 8.552 (s, 0.55H), 7.515 (d, J = 8.0 Hz, 2H), 7.440 (d, J = 8.4 Hz, 2H), 6.279 (d, J = 7.6 Hz, 1H), 6.234 (d, J = 3.2 Hz, 1H), 4.575 (m, 1H), 4.114 (s, 2H), 3.634 (s, 3H), 3.460 - 3.421 (m, 1H), 2.707 (s, 3H), 2.468 (s, 3H), 1.757 (s, 3H), 1.503 (s, 9H). |
| 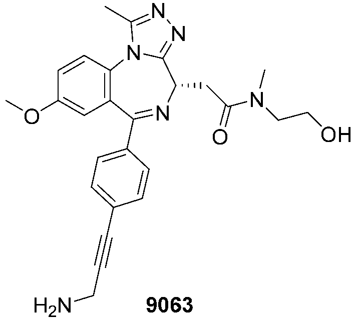 | **LCMS:** [M+H] ⁺ = 473.7; |
| | **¹H NMR:** (400 MHz, d₆-DMSO) |
| | δ 7.802 (d, J = 8.8 Hz, 1H), 7.429 (ddd, J = 20.4, 11.6, 5.4 Hz, 5H), 6.866 (d, J = 2.4 Hz, 1H), 4.562 - 4.523 (m, 1H), 3.792 (s, 3H), 3.683 - 3.316 (m, 10H), 3.194 (s, 1.5H), 2.853 (s, 1.5H), 2.531 (s, 3H). |
| 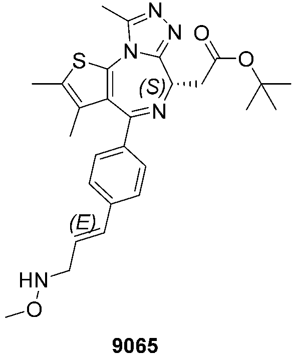 | **LCMS:** [M+H] ⁺ = 508.2; |
| | **¹H NMR:** (400 MHz, MeOD) |
| | δ 7.426 (q, J = 9.2 Hz, 8.4 Hz, 4H), 6.616 (d, J = 16.0 Hz, 1H), 6.412 (m, 1H), 4.560 - 4.524 (m, 1H), 3.631 (d, J = 6.4 Hz, 2H), 3.522 (s, 3H), 3.420 - 3.404 (m, 2H), 2.698 (s, 3H), 2.454 (s, 3H), 1.707 (s, 3H), 1.496 (s, 9H). |
| 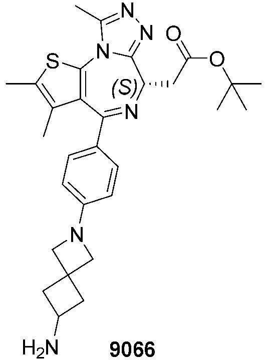 | **LCMS:** [M+H] ⁺ = 533.3; Rt = 0.828 min. |
| | **¹H NMR:** (400 MHz, MeOD) |
| | δ 8.529 (s, 1H), 7.270 (d, J = 8.0 Hz, 2H), 6.401 (d, J = 8.8 Hz, 2H), 4.466 (dd, J = 8.4, 6.4 Hz, 1H), 3.951 (s, 2H), 3.855 (s, 2H), 3.671 - 3.581 (m, 1H), 3.410 - 3.362 (m, 2H), 2.679 (s, 3H), 2.657 - 2.588 (m, 2H), 2.450 (s, 3H), 2.322 - 2.243 (m, 2H), 1.733 (s, 3H), 1.479 (s, 9H). |
| 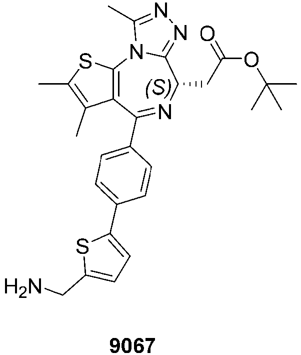 | **LCMS:** [M+H] ⁺ = 534.3; Rt = 1.107 min. |
| | **¹H NMR:** (400 MHz, CD₃OD) |
| | δ 8.544 (s, 0.3H), 7.665 (d, J = 8.4 Hz, 2H), 7.479 (d, J = 8.4 Hz, 2H), 7.378 (d, J = 4.0 Hz, 1H), 7.071 (d, J = 3.6 Hz, 1H), 4.562 (dd, J = 8.4, 2.8 Hz, 1H), 4.108 (s, 2H), 3.455 - 3.417 (m, 2H), 2.705 (s, 3H), 2.465 (s, 3H), 1.750 (s, 3H), 1.504 (s, 9H). |
| 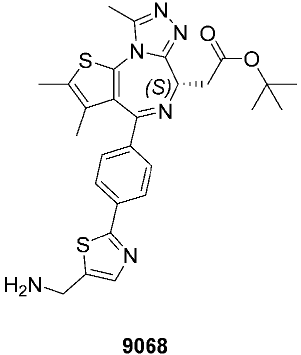 | **LCMS:** [M+H] ⁺ = 535.2; |
| | **¹H NMR:** (400 MHz, CD₃OD) |
| | δ 8.537 (s, 0.25H), 7.964 (d, J = 8.4 Hz, 2H), 7.786 (s, 1H), 7.572 (d, J = 8.4 Hz, 2H), 4.585 (dd, J = 6.0, 2.4 Hz, 1H), 4.152 (s, 2H), 3.508 - 3.404 (m, 2H), 2.709 (s, 3H), 2.466 (s, 3H), 1.739 (s, 3H), 1.507 (s, 9H). |
| 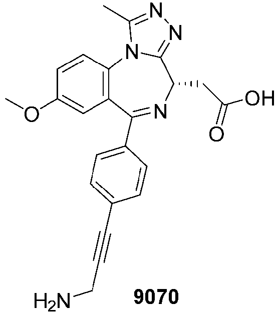 | **LCMS:** [M+H] ⁺ = 416.2; |
| | **¹H NMR:** (400 MHz, MeOD) |
| | δ 7.714 (d, J = 9.2 Hz, 1H), 7.562 (d, J = 8.0 Hz, 2H), 7.464 (d, J = 8.0 Hz, 2H), 7.358 (dd, J = 8.8, 2.4 Hz, 1H), 6.879 (d, J = 2.8 Hz, 1H), 4.603 (br s, 1H), 3.986 (br s, 2H), 3.808 (s, 3H), 3.490 - 3.330 (m, 2H), 2.611 (s, 3H). |
| 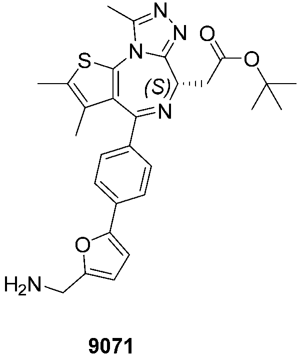 | **LCMS:** [M+H] ⁺ = 518.4; |
| | **¹H NMR:** (400 MHz, CD₃OD) |
| | δ 8.530 (s, 0.7H), 7.757 (d, J = 8.4 Hz, 2H), 7.495 (d, J = 8.0 Hz, 2H), 6.879 (d, J = 3.2 Hz, 1H), 6.536 (m, 1H), 4.564 (dd, J = 2.8, 5.6 Hz, 1H), 4.074 (s, 2H), 3.455 - 3.416 (m, 2H), 2.704 (s, 3H), 2.465 (s, 3H), 1.739 (s, 3H), 1.503 (s, 9H). |
| 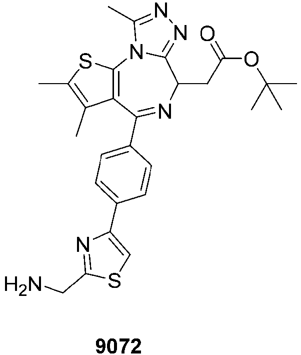 | **LCMS:** [M+H] ⁺ = 535.3; |
| | **¹H NMR:** (400 MHz, MeOD) |
| | δ 8.530 (s, 0.2H), 7.985 (d, J = 8.4 Hz 2H), 7.881 (s, 1H), 7.521 (d, J = 8.4 Hz, 2H), 4.572 (dd, J = 2.4, 6.0 Hz, 1H), 4.215 (s, 2H), 3.465 -3.422 (m, 2H), 2.712 (s, 3H), 2.467 (s, 3H), 1.741 (s, 3H), 1.507 (s, 9H). |
| 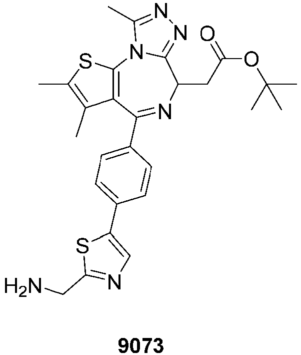 | **LCMS:** [M+H] ⁺ = 535.3; |
| | **¹H NMR:** (400 MHz, MeOD) |
| | δ 8.534 (s, 0.2H), 8.058 (s, 1H), 7.685 (d, J = 8.4 Hz 2H), 7.522 (d, J = 8.4 Hz, 2H), 4.571 (dd, J = 2.4, 6.0 Hz, 1H), 4.138 (s, 2H), 3.458 -3.419 (m, 2H), 2.706 (s, 3H), 2.465 (s, 3H), 1.745 (s, 3H), 1.503 (s, 9H). |
| 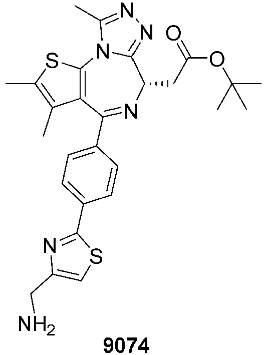 | **LCMS:** [M+H] ⁺ = 535.4; |
| | **¹H NMR:** (400 MHz, MeOD) |
| | δ 8.544 (s, 0.2H), 7.741 (d, J = 8.4 Hz, 2H), 7.637 (s, 1H), 7.572 (d, J = 8.4 Hz, 2H), 4.584 (dd, J = 8.4, 6.0 Hz, 1H), 4.029 (s, 2H), 3.502 - 3.385 (m, 2H), 2.707 (s, 3H), 2.466 (s, 3H), 1.742 (s, 3H), 1.505 (s, 9H). |
| 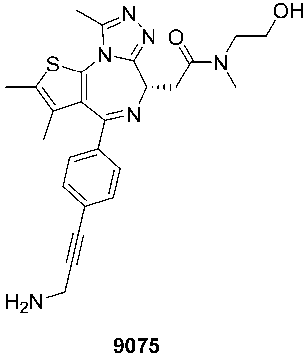 | **LCMS:** [M+H] ⁺ = 477.3; |
| | **¹H NMR:** (400 MHz, MeOD) |
| | δ 8.524 (s, 0.5H), 7.498 - 7.446 (m, 4H), 4.699 (dd, J = 7.2 Hz, 6.0 Hz, 1H), 3.905 (m, 1H), 3.838 - 3.824 (m, 1H), 3.738 - 3.549 (m, 5H), 3.315 (d, J = 1.6 Hz, 2H), 3.026 (s, 1H), 2.697 (d, J = 2.0 Hz, 3H), 2.445 (s, 3H), 1.681 (s, 3H). |
| 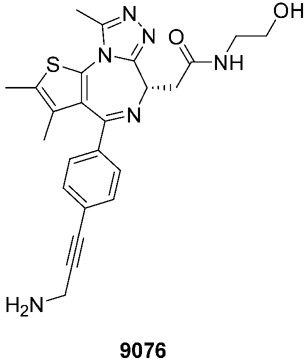 | **LCMS:** [M+H] ⁺ = 463.3; |
| | **¹H NMR:** (400 MHz, MeOD) |
| | δ 7.505 - 7.457 (m, 4H), 4.648 (dd, J = 3.2 Hz, 5.6 Hz, 1H), 3.655 (t, J = 5.6 Hz, 2H), 3.442 - 3.307 (m, 6H), 2.693 (s, 3H), 2.441 (s, 3H), 1.674 (s, 3H). |
| 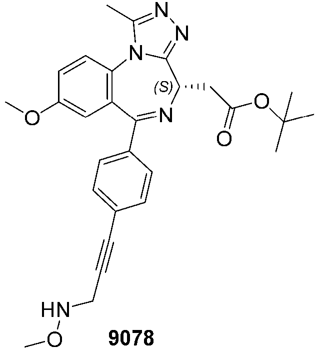 | **LCMS:** [M+H] ⁺ = 502.4; |
| | **¹H NMR:** (400 MHz, MeOD) |
| | δ 7.724 (d, J = 8.8 Hz, 4H), 7.515 (d, J = 8.4 Hz, 2H), 7.446 (d, J = 8.4 Hz, 2H), 7.382 (dd, J = 6.0 Hz, 2.8 Hz, 1H), 6.919 (d, J = 2.8 Hz, 1H), 4.553 (dd, J = 2.0 Hz, 6.0 Hz, 1H), 3.836 (s, 2H), 3.825 (s, 3H), 3.575 (s, 3H), 3.426 - 3.388 (m, 2H), 2.631 (s, 3H), 1.481 (s, 9H). |
| 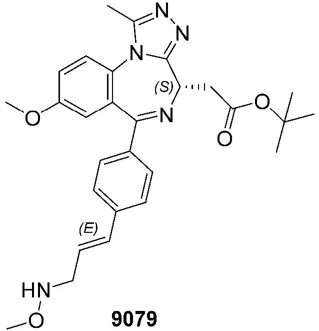 | **LCMS:** [M+H] ⁺ = 504.4; |
| | **¹H NMR:** (400 MHz, MeOD) |
| | δ 8.572 (s, 0.2H), 7.719 (d, J = 8.8 Hz, 1H), 7.497 (d, J = 8.4 Hz, 2H), 7.442 (d, J = 8.4 Hz, 2H), 7.377 (d, J = 8.8 Hz, 1H), 6.937 (d, J = 2.8 Hz, 1H), 6.622 (d, J = 16.0 Hz, 1H), 6.419 (dt, J = 15.6, 6.4 Hz, 1H), 4.542 (dd, J = 8.4, 6.0 Hz, 1H), 3.821 (s, 3H), 3.634 (d, J = 6.4 Hz, 2H), 3.522 (s, 3H), 3.404 (dd, J = 10.4, 7.2 Hz, 2H), 2.635 (s, 3H), 1.486 (s, 9H). |
| 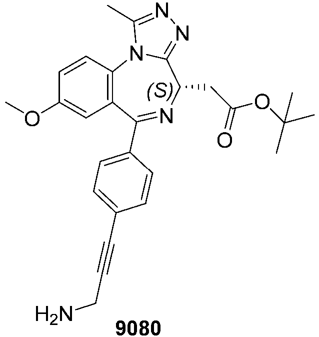 | **LCMS:** [M+H] ⁺ = 472.3; |
| | **¹H NMR:** (400 MHz, MeOD) |
| | δ 8.510 (s, 0.8H), 7.731 (d, J = 8.8 Hz, 4H), 7.533 (d, J = 8.4 Hz, 2H), 7.466 (d, J = 8.4 Hz, 2H), 7.389 (dd, J = 6.0 Hz, 2.8 Hz, 1H), 6.911 (d, J = 2.8 Hz, 1H), 4.558 (dd, J = 2.0 Hz, 6.0 Hz, 1H), 3.827 (s, 3H), 3.794 (s, 2H), 3.426 - 3.390 (m, 2H), 2.630 (s, 3H), 1.481 (s, 9H). |
| 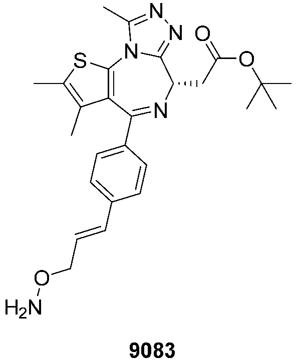 | **LCMS:** [M+H] ⁺ = 494.2; |
| | **¹H NMR:** (400 MHz, d₄-MeOD) |
| | δ 7.498 - 7.400 (m, 4H), 6.692 (s, 1H), 6.461 - 6.421 (d, J = 8.0 Hz, 1H), 4.687 - 4.372 (m, 3H), 3.406 - 3.309 (d, J = 8.0 Hz, 2H), 2.698 - 2.654 (m, 3H), 2.454 (s, 1H), 1.675 (s, 3H), 1.496 - 1.375 (m, 9H). |
| 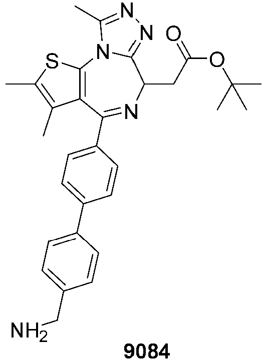 | **LCMS:** [M+H] ⁺ = 528.4; |
| | **¹H NMR:** (400 MHz, MeOD) |
| | δ 8.556 (s, 0.2H), 7.715 (dd, J = 11.6, 8.4 Hz, 4H), 7.531 (dd, J = 15.6, 8.4 Hz, 4H), 4.581 (dd, J = 8.4, 6.4 Hz, 1H), 4.071 (s, 2H), 3.492 - 3.386 (m, 2H), 2.712 (s, 3H), 2.469 (s, 3H), 1.749 (s, 3H), 1.508 (s, 9H). |
| 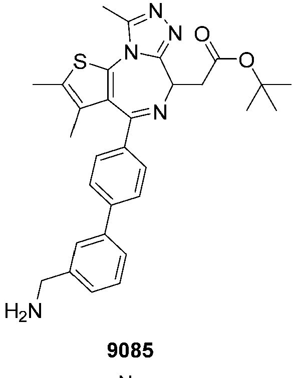 | **LCMS:** [M+H] ⁺ = 528.3; |
| | **¹H NMR:** (400 MHz, d₆-DMSO) |
| | δ 8.480 (s, 1H), 7.763 - 7.744 (dd, J = 7.6 Hz, 3H), 7.522 (d, J = 8.4 Hz, 1H), 7.445 (d, J = 11.2 Hz, 2H), 7.435 - 7.384 (m, 2H), 4.465 (d, J = 10.8 Hz, 1H), 3.905 (s, 1H), 3.345 (d, J =6.8 Hz, 2H), 2.625 (s,3H), 2.445 (s, 3H), 1.685 (s, 3H), 1.457 (s, 9H). |
| 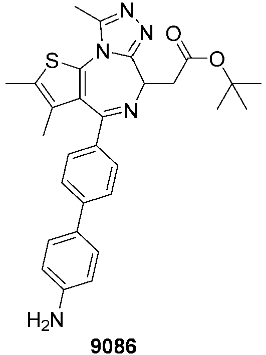 | **LCMS:** [M+H] ⁺ = 514.3; |
| | **¹H NMR:** (400 MHz, d₆-DMSO) |
| | δ 7.598 (d, J = 8.4 Hz, 2H), 7.502 - 7.394 (m, 4H), 6.762 (d, J = 8.4 Hz, 2H), 4.552 (dd, J = 8.4, 6.0 Hz, 1H), 3.531 - 3.384 (m, 2H), 2.707 (s, 3H), 2.465 (s, 3H), 1.750 (s, 3H), 1.504 (s, 9H). |
| 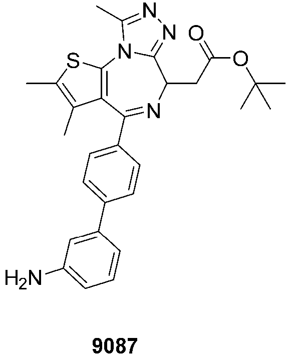 | **LCMS:** [M+H] ⁺ = 514.2; |
| | **¹H NMR:** (400 MHz, MeOD) |
| | δ 8.508 (s, 0.24H), 7.626 (d, J = 8.4 Hz, 2H), 7.498 (d, J = 8.4 Hz, 2H), 7.168 (t, J = 7.6 Hz, 1H), 6.981 (dd, J = 16.8, 4.8 Hz, 2H), 6.726 (dd, J = 7.6, 1.4 Hz, 1H), 4.568 (dd, J = 8.4, 6.0 Hz, 1H), 3.441 (qd, J = 16.4, 7.2 Hz, 2H), 2.711 (s, 3H), 2.465 (s, 3H), 1.743 (s, 3H), 1.507 (s, 9H). |
| 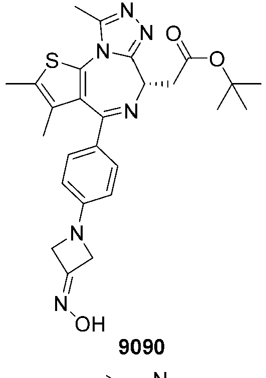 | **LCMS:** [M+H] ⁺ = 507.4; |
| | **¹H NMR:** (400 MHz, d₆-DMSO) |
| | δ 10.960 (s, 1H), 8.572 (s, 1H), 7.295 - 7.275 (dd, J = 11.6 Hz, 2H), 6.542 - 6.521 (dd, J = 8.4 Hz, 2H), 4.575 (d, J = 8.0 Hz, 4H), 4.328 (t, 1H), 3.262 (d, J = 7.6 Hz, 2H), 2.588 (s, 3H), 2.422 (s, 3H), 1.693 (s, 3H), 1.418 (s, 9H). |
| 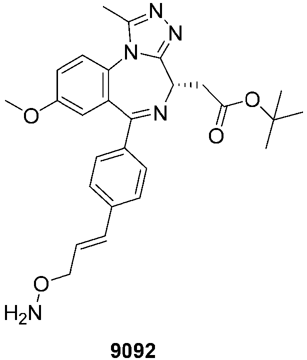 | **LCMS:** [M+H] ⁺ = 490.3; |
| | **¹H NMR:** (400 MHz, d₄-MeOD) |
| | δ 8.510 (s, 1H), 8.572 (s, 1H), 7.498 (d, J = 8.0 Hz, 1H), 7.395 - 7.366 (t, J = 11.6 Hz, 4H), 7.360 (d, J = 8.0 Hz, 1H), 6.940 (s, 1H), 6.707 - 6.667 (dd, J = 8.4 Hz, 1H), 6.466 - 6.426 (m, 1H), 4.575(d, J = 8.0 Hz, 1H), 4.310 (t, J = 7.6 Hz, 2H), 3.822 (s, 3H), 3.385 - 3.305 (m, 2H), 2.636 (s, 3H), 1.486 - 1.442 (m, 9H). |
| 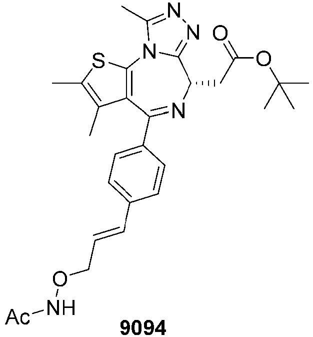 | **LCMS:** [M+H] ⁺ = 536.3.; |
| | **¹H NMR:** (400 MHz, d₄-MeOD) |
| | δ 7.489 (d, J = 8.4 Hz, 2H), 7.428 (d, J = 8.0 Hz, 2H), 6.717 (d, J = 16.0 Hz, 2H), 6.513 - 6.440 (m, 1H), 4.549 (dd, J = 2.4 Hz, 6.0 Hz, 1H), 4.484 (d, J = 6.4 Hz, 2H), 3.445 - 3.406 (m, 2H), 2.696 (s, 3H), 2.456 (s, 3H), 1.842 (s, 3H), 1.704 (s, 3H), 1.496 (s, 9H). |
| 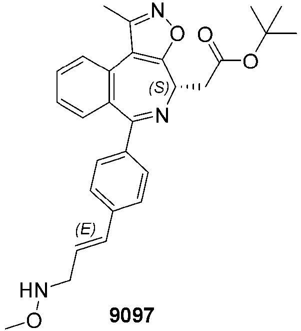 | **LCMS:** [M+H] ⁺ = 474.1; |
| | **¹H NMR:** (400 MHz, d₆-DMSO) |
| | δ 7.835 (d, J = 8.0 Hz, 1H), 7.721 (t, J = 7.2 Hz, 1H), 7.424 (t, J = 8.4 Hz, 4H), 7.260 (d, J = 8.4 Hz, 2H), 6.725 (t, J = 6.0 Hz, 1H), 6.546 (d, J = 16.0 Hz, 1H), 6.408 - 6.337 (m, 1H), 4.240 (s, 1H), 3.531 (dd, J = 13.2, 6.8 Hz, 2H), 3.381 (d, J = 11.2 Hz, 3H), 2.435(s, 2H), 2.525 (s, 3H), 1.440 (s, 9H). |
| 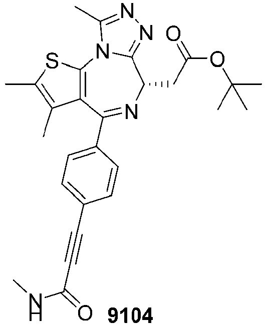 | **LCMS:** [M+H] ⁺ = 504.1; |
| | **¹H NMR:** (400 MHz, MeOD) |
| | δ 7.596 (d, J = 8.4 Hz, 2H), 7.508 (d, J = 8.4 Hz, 2H), 4.573 (dd, J = 8.4, 6.0 Hz, 1H), 3.432 (dd, J = 7.2, 6.0 Hz, 2H), 2.803 (s, 3H), 2.696 (s, 3H), 2.456 (s, 3H), 1.697 (s, 3H), 1.494 (s, 9H). |
| 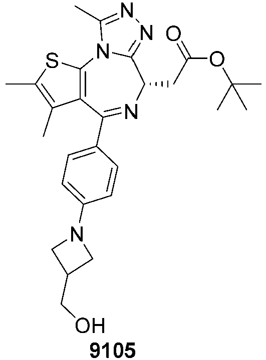 | **LCMS:** [M+H] ⁺ = 508.2; |
| | **¹H NMR:** (400 MHz, MeOD) |
| | δ 8.513 (s, 0.18H), 7.269 (d, J = 8.2 Hz, 2H), 6.393 (d, J = 8.4 Hz, 2H), 4.462 (dd, J = 8.4, 6.0 Hz, 1H), 3.960 (t, J = 7.6 Hz, 2H), 3.743 (d, J = 6.4 Hz, 2H), 3.673 (dd, J = 7.6, 5.2 Hz, 2H), 3.442 - 3.335 (m, 2H), 2.873 (dt, J = 12.4, 6.0 Hz, 1H), 2.681 (s, 3H), 2.452 (s, 3H), 1.745 (s, 3H), 1.480 (s, 9H). |
| 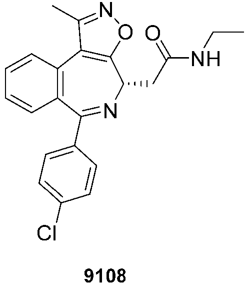 | **LCMS:** [M+H] ⁺ = 394.0; |
| | **¹H NMR:** (400 MHz, MeOD) |
| | δ 7.802 (d, J = 8.0 Hz, 1H), 7.732 - 7.685 (m, 1H), 7.407 (dd, J = 4.8, 1.2 Hz, 2H), 7.373 - 7.321 (m, 4H), 4.455 (s, 1H), 3.302 - 3.226 (m, 4H), 2.560 (s, 3H), 1.184 (t, J = 7.2 Hz, 3H). |
| 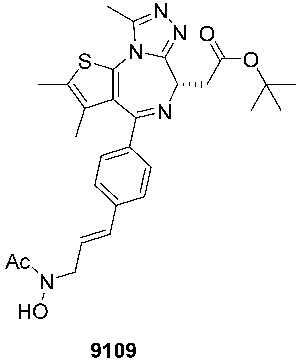 | **LCMS:** [M+H] ⁺ = 536.3; |
| | **¹H NMR:** (400 MHz, d₆-DMSO) |
| | δ 9.887 (s, 1H), 7.492 (d, J = 8.4 Hz, 2H), 7.378 (d, J = 8.4 Hz, 2H), 6.573 (d, J = 16.0 Hz, 1H), 6.363 - 6.293 (m, 1H), 4.403 (dd, J = 8.0, 6.4 Hz, 1H), 4.272 (d, J = 6.0 Hz, 2H), 3.304 - 3.263 (m, 2H), 2.604 (s, 3H), 2.418 (s, 3H), 2.018 (s, 3H), 1.631 (s, 3H), 1.428 (s, 9H). |
| 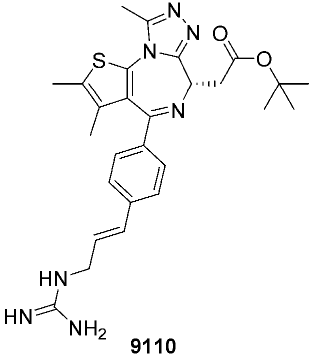 | **LCMS:** [M+H] ⁺ = 520.4; |
| | **¹H NMR:** (400 MHz, MeOD) |
| | δ 7.449 (dd, J = 19.2, 8.4 Hz, 4H), 6.655 (d, J = 16.0 Hz, 1H), 6.356 (dt, J = 16.0, 5.6 Hz, 1H), 4.553 (dd, J = 8.4, 6.0 Hz, 1H), 4.000 (dd, J = 5.6, 1.2 Hz, 2H), 3.492 - 3.373 (m, 2H), 2.690 (s, 3H), 2.455 (s, 3H), 1.704 (s, 3H), 1.495 (s, 9H). |
| 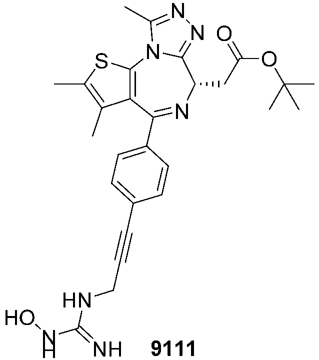 | **LCMS:** [M+H] ⁺ = 534.3; |
| | **¹H NMR:** (400 MHz, MeOD) |
| | δ 7.472 - 7.420 (m, 4H), 4.560 (dd, J = 8.4, 6.0 Hz, 1H), 4.206 (s, 2H), 3.425 (dd, J = 7.2, 5.2 Hz, 2H), 2.691 (s, 3H), 2.453 (s, 3H), 1.693 (s, 3H), 1.490 (s, 9H). |
| 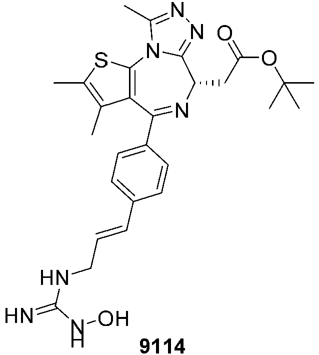 | **LCMS:** [M+H] ⁺ = 536.4; |
| | **¹H NMR:** (400 MHz, d₆-DMSO) |
| | δ 7.434 (dd, J = 8.4 Hz, 4H), 6.624 (d, J = 16.0 Hz, 1H), 6.368 (dt, J = 15.8, 5.6 Hz, 1H), 4.547 (dd, J = 8.4, 6.0 Hz, 1H), 3.950 (d, J = 4.8 Hz, 2H), 3.462 - 3.379 (m, 2H), 2.710 (d, J = 11.0 Hz, 3H), 2.455 (s, 3H), 1.703 (s, 3H), 1.495 (s, 9H). |
| 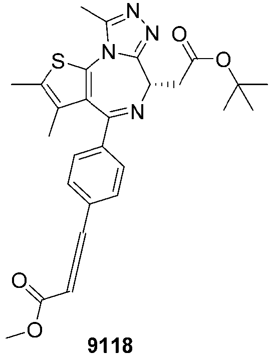 | **LCMS:** [M+H] ⁺ = 519.2; |
| | **¹H NMR:** (400 MHz, MeOD) |
| | δ 7.463 (d, J = 8.4 Hz, 2H), 7.382 (d, J = 8.4 Hz, 2H), 6.840 (d, J = 6.4 Hz, 1H), 6.131 (d, J = 6.4 Hz, 1H), 4.623 - 4.556 (m, 1H), 3.743 (d, J = 3.6 Hz, 3H), 3.491 - 3.385 (m, 2H), 2.725 (s, 3H), 2.462 (s, 3H), 1.712 (s, 3H), 1.496 (s, 9H). |
| 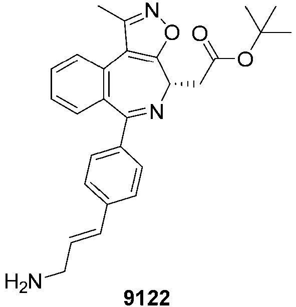 | **LCMS:** [M+H] ⁺ = 444.1.; |
| | **¹H NMR:** (400 MHz, d₄-MeOD) |
| | δ 7.828 (d, J = 8.0 Hz, 1H), 7.744 - 7.703 (m, 1H), 7.463 (d, J = 8.4 Hz, 2H), 7.433 - 7.410 (m, 2H), 7.348 (d, J = 8.4 Hz, 2H), 6.825 (d, J = 16.0 Hz, 1H), 6.356 (dt, J = 15.8, 6.8 Hz, 1H), 4.890 (d, J = 5.0 Hz, 1H), 4.851 (s, 2H), 3.737 (t, J = 10.4 Hz, 2H), 2.572 (s, 3H), 1.491 (s, 9H). |
| 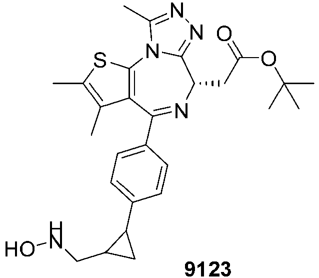 | **LCMS:** [M+H] ⁺ = 508.1; |
| | **¹H NMR:** (400 MHz, d₄-MeOD) |
| | δ 7.318 - 7.237 (m, 4H), 7.090 (s, 1H), 4.408 - 4.372 (m, 1H), 3.326 - 3.250 (m, 2H), 2.597 (s, 3H), 2.454 - 2.415 (m, 1H), 2.415 (s, 3H), 2.328 - 2.278 (m, 1H), 2.196 - 2.179 (m, 1H), 1.615 (s, 3H), 1.429 (s, 9H), 1.344 - 1.326 (m, 1H), 0.989 - 0.894 (m, 2H). |
| 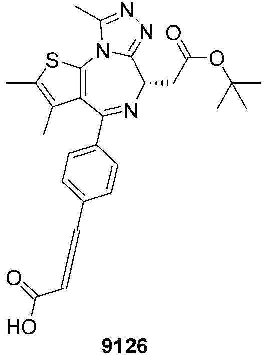 | **LCMS:** [M+H] ⁺ = 505.3; |
| | **¹H NMR:** (400 MHz, MeOD) |
| | δ 7.430 (s, 2H), 7.382 (s, 2H), 6.773 (d, J = 6.4 Hz, 1H), 6.049 (s, 1H), 4.552 (dd, J = 8.4, 6.0 Hz, 1H), 3.422 (s, 2H), 2.696 (s, 3H), 2.454 (s, 3H), 1.709 (s, 3H), 1.492 (s, 9H). |
| 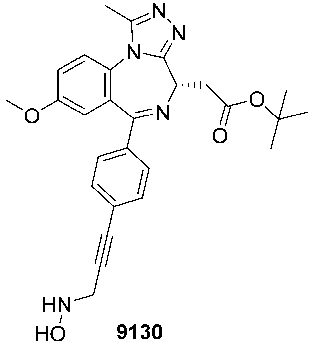 | **LCMS:** [M+H] ⁺ = 488.3; |
| | **¹H NMR:** (400 MHz, d₆-DMSO) |
| | δ 11.272 (br, 1 H), 10.727 (br, 1H), 7.820 (d, J = 9.2 Hz, 1H), 7.542 (dd, J = 14.4, 8.4 Hz, 4H), 7.402 (dd, J = 9.2, 2.8 Hz, 1H), 6.876 (d, J = 2.8 Hz, 1H), 4.427 (dd, J = 8.0, 6.4 Hz, 1H), 4.310 (s, 2H), 3.802 (d, J = 4.0 Hz, 3H), 3.323 (dd, J = 9.6, 7.2 Hz, 2H), 2.541 (s, 3H), 1.422 (s, 9H). |
| 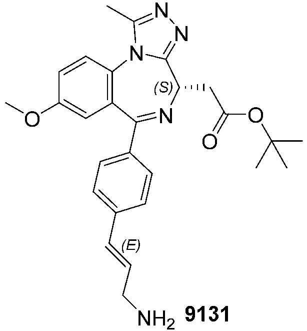 | **LCMS:** [M+H] ⁺ = 474.22; |
| | **¹H NMR:** (400 MHz, d₆-DMSO) |
| | δ 7.806 (d, J = 8.8 Hz, 1H), 7.374 (m, 4H), 7.297 (s, 1H), 6.879 (s, 1H), 6.638(d, J = 16.0 Hz, 1H), 6.429 (d, J = 16.0 Hz, 1H), 4.413 - 4.377 (dd, J = 7.2 Hz, 1H), 3.793 (s, 3H), 3.490 (s, 2H), 3.296 (m, 2H), 2.542 - 2.498 (m, 3H), 1.427 (s, 9H). |
| 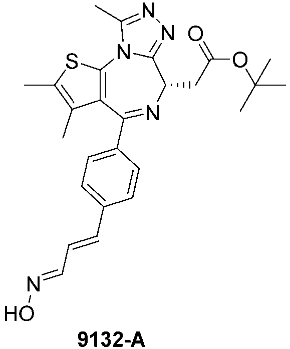 | **LCMS:** [M+H] ⁺ = 492.20; |
| | **¹H NMR:** (400 MHz, d₄-MeOD) |
| | δ 7.602 - 7.459 (m, 5H), 7.239 (d, J = 9.6 Hz, 1H), 6.961 (d, J = 16.2 Hz, 1H), 4.560 (dd, J = 8.4, 6.0 Hz, 1H), 3.463 (d, J = 10.6 Hz, 2H), 2.702 (s, 3H), 2.461 (s, 3H), 1.723 (s, 3H), 1.499 (s, 9H). |
| 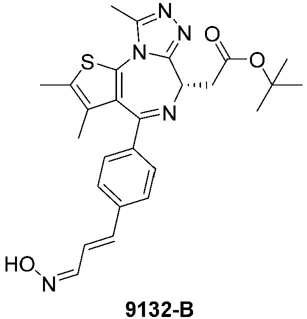 | **LCMS:** [M+H] ⁺ = 492.2; |
| | **¹H NMR:** (400 MHz, d₄-MeOD) |
| | δ 7.900 (d, J = 8.4 Hz, 1H), 7.540 (d, J = 8.4 Hz, 2H), 7.449 (d, J = 8.2 Hz, 2H), 6.975 - 6.866 (m, 2H), 4.557 - 4.536 (m, 1H), 3.477 - 3.426 (m, 2H), 2.702 (s, 3H), 2.459 (s, 3H), 1.719 (s, 3H), 1.498 (s, 9H). |
| 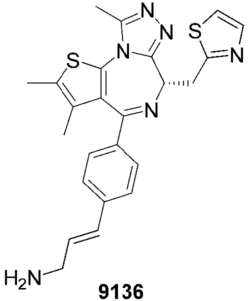 | **LCMS:** [M+H] ⁺ = 461.08; |
| | **¹H NMR:** (400 MHz, MeOD) |
| | δ 7.734 (d, J = 3.2 Hz, 1H), 7.545 (d, J = 3.2 Hz, 1H), 7.474 (q, J = 8.4 Hz, 4H), 6.807 (d, J = 16.0 Hz, 1H), 6.396 - 6.357 (m, 1H), 4.638 (dd, J = 8.8, 5.2 Hz, 1H), 4.234 - 4.122 (m, 2H), 3.701 (dd, J = 6.8, 1.2 Hz, 2H), 2.706 (s, 3H), 2.437 (s, 3H), 1.655 (s, 3H). |
| 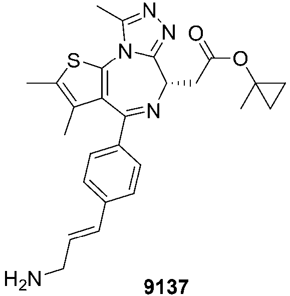 | **LCMS:** [M+H] ⁺ = 476.1; |
| | **¹H NMR:** (400 MHz, MeOD) |
| | δ 8.539 (s, 0.87H), 7.511 - 7.426 (dd, J = 25.6, 8.4 Hz, 4H), 6.814 - 6.774 (d, J = 16.0 Hz, 1H), 6.405 - 6.366 (m, 1H), 4.594 - 4.557 (dd, J = 8.4, 6.4 Hz, 1H), 3.690 - 3.674 (d, J = 6.4 Hz, 2H), 3.453 - 3.420 (m, 2H), 2.696 (s, 3H), 2.458 (s, 3H), 1.700 (s, 3H), 1.554 (s, 3H), 0.897 (t, J = 6.6 Hz, 2H), 0.676 (t, J = 6.6 Hz, 2H). |
| 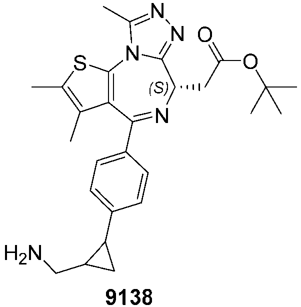 | **LCMS:** [M+H] ⁺ = 492.2; |
| | **¹H NMR:** (400 MHz, CD₃OD) |
| | δ 8.538 (s, 1H), 7.406 (d, J = 8.0 Hz, 2H), 7.307 (d, J = 7.6 Hz, 2H), 4.551 (dd, J = 8.4, 6.0 Hz, 1H), 3.474 - 3.365 (m, 2H), 2.845 (dd, J = 13.2, 6.0 Hz, 1H), 2.691 (s, 3H), 2.495 - 2.430 (m, 4H), 2.186 (ddd, J = 13.6, 9.6, 4.4 Hz, 1H), 1.691 (s, 4H), 1.511 - 1.458 (m, 10H), 1.266 - 1.183 (m, 1H), 1.126 - 1.040 (m, 1H). |
| 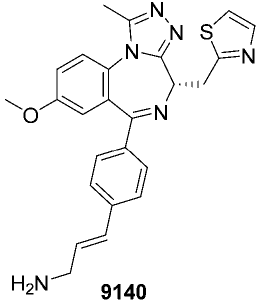 | **LCMS:** [M+H] ⁺ = 457.17; |
| | **¹H NMR:** (400 MHz, d₄-MeOD) |
| | δ 7.538 (s, 2H), 7.393 - 7.364 (m, 5H), 7.315 (d, J = 3.6 Hz, 1H), 6.889 (s, 1H), 6.780 (d, J = 5.2 Hz, 1H), 6.391 (t, J = 6.8 Hz, 1H), 4.609 (s, 1H), 4.120 (m, 2H), 3.791 (s, 3H), 3.683 (m, 2H), 2.642 (s, 3H). |
| 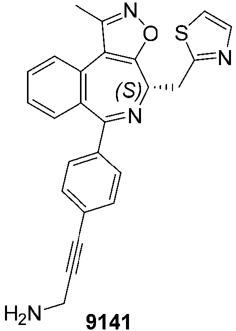 | **LCMS:** [M+H] ⁺ = 425.1; |
| | **¹H NMR:** (400 MHz, MeOD) |
| | δ 7.815 (d, J = 7.6 Hz, 1H), 7.736 - 7.685 (m, 2H), 7.539 (d, J = 3.2 Hz, 1H), 7.419 - 7.324 (m, 6H), 4.440 (s, 1H), 4.078 (s, 2H), 3.771 (s, 2H), 2.567 (s, 3H). |
| 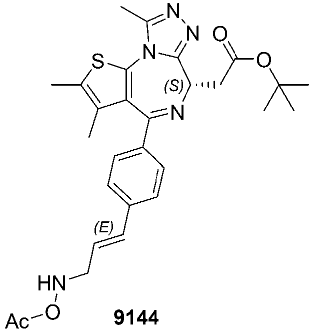 | **LCMS:** [M+H]⁺ = 536.50; |
| | **¹H NMR:** (400 MHz, d₆-DMSO) |
| | δ 8.04 (t, J = 5.2 Hz, 1H), 7.47 (d, J = 8.2 Hz, 2H), 7.38 (d, J = 8.0 Hz, 2H), 6.60 (d, J = 16.0 Hz, 1H), 6.46 - 6.31 (m, 1H), 4.40 (t, J = 7.0 Hz, 1H), 3.69 (s, 2H), 2.67 (s, 1H), 2.33 (s, 1H), 2.00 (s, 3H), 1.64 (s, 3H), 1.43 (s, 12H), 1.24 (s, 3H). |
| 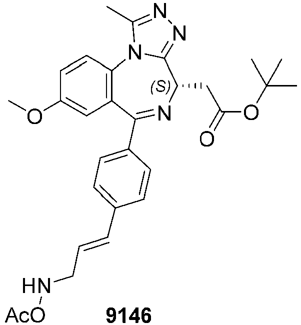 | **LCMS:** [M+H] ⁺ = 532.20; |
| | **¹H NMR:** (400 MHz, MeOD) |
| | δ 8.043 (t, J = 5.8 Hz, 1H), 7.799 (d, J = 9.0 Hz, 1H), 7.471 (s, 4H), 7.385 (dd, J = 9.0, 2.8 Hz, 1H), 6.876 (d, J = 2.8 Hz, 1H), 6.609(d, J = 16.2 Hz, 1H), 6.379 (dt, J = 15.8, 6.4 Hz, 1H), 4.391 (t, J = 7.2 Hz, 1H), 3.793 (s, 3H), 3.691 (s, 2H), 3.295 (s, 2H), 2.540 (s, 3H), 1.998 (s, 3H), 1.426 (s, 9H). |
| 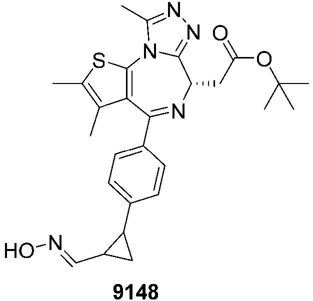 | **LCMS:** [M+H] ⁺ = 506.3; |
| | **¹H NMR:** (400 MHz, CDCl₃) |
| | δ 7.40 (d, J = 7.6 Hz, 2H), 7.23 (t, J = 8.8 Hz, 2H), 6.66 (d, J = 8.8 Hz, 1H), 5.86 (d, J = 8.4 Hz, 1H), 4.57 (d, J = 6.8 Hz, 1H), 3.56 (d, J = 7.2 Hz, 2H), 2.71 (d, J = 1.2 Hz, 3H), 2.64 - 2.45 (m, 1H), 2.41 (s, 3H), 2.07 (dt, J = 14.4, 8.8 Hz, 1H), 1.66 (d, J = 4.0 Hz, 3H), 1.49 (d, J = 7.2 Hz, 9H), 1.25 (s, 2H). |
| 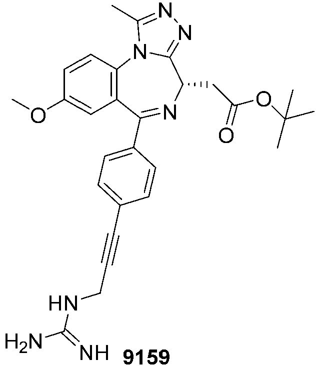 | **LCMS:** [M+H] ⁺ = 514.4; |
| | **¹H NMR:** (400 MHz, d₄-MeOD) |
| | δ 7.737 (d, J = 8.8 Hz, 1H), 7.540 (d, J = 8.4 Hz, 2H), 7.468 (d, J = 8.4 Hz, 2H), 7.393 (dd, J = 8.8, 2.8 Hz, 1H), 6.908 (d, J = 2.8 Hz, 1H), 4.561 (dd, J = 8.4, 6.0 Hz, 1H), 4.281 (s, 2H), 3.828 (s, 3H), 3.425 - 3.391 (m, 2H), 2.629 (s, 3H), 1.480 (s, 9H). |
| 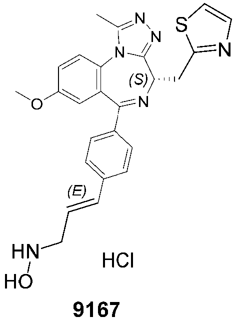 | **LC-MS:** (M+H) ⁺, 473.10; |
| | **¹H NMR:** (400 MHz, CD₃OD) |
| | δ 7.722 (d, J = 8.4 Hz, 2H), 7.524 (dd, J = 9.6, 5.6 Hz, 3H), 7.447 (d, J = 8.4 Hz, 2H), 7.393 - 7.355 (m, 1H), 6.902 (d, J = 2.8 Hz, 1H), 6.651 (d, J = 16.4 Hz, 1H), 6.494 - 6.383 (m, 1H), 4.612 (s, 1H), 4.288 - 4.146 (m, 1H), 4.102 (d, J = 15.2 Hz, 1H), 3.804 (s, 3H), 3.646 (d, J = 6.4 Hz, 2H), 2.652 (s, 3H). |
| 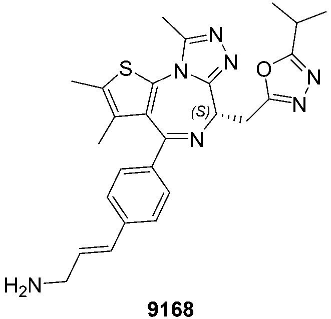 | **LCMS:** [M+H] ⁺ = 488.25; |
| | **¹H NMR:** (400 MHz, d₆-DMSO) |
| | δ 7.463 - 7.443 (d, J = 8.0 Hz, 2H), 7.337 - 7.317 (d, J = 8.0 Hz, 2H), 6.683 - 6.643 (d, J = 8.0 Hz, 1H), 6.456 - 6.367 (m, 1H), 4.683 - 4.646 (m, 1H), 3.977 - 3.861 (m, 2H), 3.517 - 3.504 (d, J = 5.2 Hz, 2H), 3.240 - 3.170 (m, 1H), 2.619 (s, 3H), 2.419 (s, 3H), 1.619 (s, 3H), 1.314 - 1.282 (t, J = 12.8 Hz, 6H). |
| 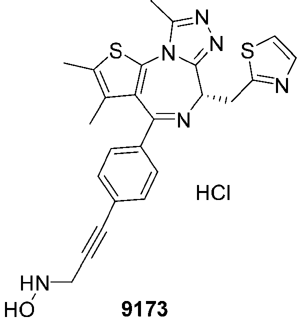 | **LCMS:**[M+H] ⁺ = 475.1; |
| | **¹H NMR:** (400 MHz, d₄-MeOD) |
| | δ 8.060 (d, J = 3.2 Hz, 1H), 7.844 (d, J = 3.2 Hz, 1H), 7.589 (s, 4H), 4.833 - 4.823 (m, 1H), 4.388 (s, 2H), 4.367 - 4.213 (m, 2H), 2.794 (s, 3H), 2.469 (s, 3H), 1.701 (s, 3H). |
| 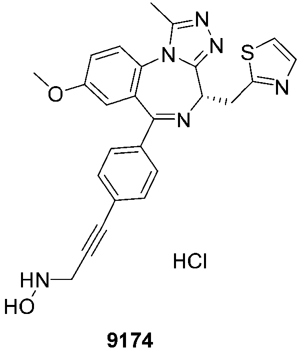 | **LCMS:** [M+H] ⁺ = 471.1; |
| | **¹H NMR:** (400 MHz, d₄-MeOD) |
| | δ 7.754 - 7.716 (m, 2H), 7.596 - 7.514 (m, 5H), 7.402 - 7.372 (m, 1H), 6.883 - 6.876 (m, 1H), 6.396 - 6.357 (m, 1H), 4.657 (dd, J = 9.2, 4.8 Hz, 1H), 4.330 (s, 2H), 4.212 - 4.085 (m, 2H), 3.808 (s, 3H), 2.654 (d, J = 10.0 Hz, 3H). |
| 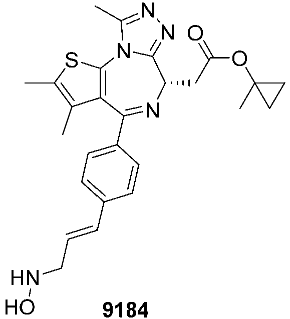 | **LCMS:** [M+H] ⁺ = 492.22; |
| | **¹H NMR:** (400 MHz, d₆-DMSO) |
| | δ 7.463 (m, 2H), 7.442 - 7.346 (m, 2H), 7.252 (s, 1H), 6.579 - 6.539 (dd, J = 16.0 Hz, 1H), 6.445 - 6.390 (m, 1H), 4.446 - 4.409 (t, J = 8.0 Hz, 1H), 3.490 (s, 1H), 3.346 - 3.318 (m, 2H), 2.603 (s, 3H), 2.421 (s, 3H), 1.639 (m, 3H), 1.487 (s, 3H), 0.794 (s, 2H), 0.668 (s, 2H). |
| 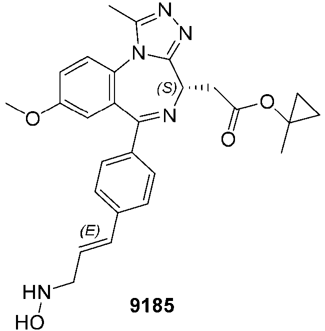 | **LC-MS:** (M+H) ⁺, 488.25; |
| | **¹H NMR:** (400 MHz, CD₃OD) |
| | δ 7.750 (d, J = 9.2 Hz, 1H), 7.592 - 7.495 (m, 4H), 7.403 (dd, J = 9.2, 2.8 Hz, 1H), 6.987 - 6.864 (m, 2H), 6.503 - 6.257 (m, 1H), 4.598 (dd, J = 8.4, 6.4 Hz, 1H), 4.021 (dd, J = 7.2, 0.8 Hz, 2H), 3.833 (s, 3H), 3.472 - 3.375 (m, 2H), 2.6525 (s, 3H), 1.54 (s, 3H), 0.884 (d, J = 5.6 Hz, 2H), 0.663 (d, J = 1.2 Hz, 2H). |
| 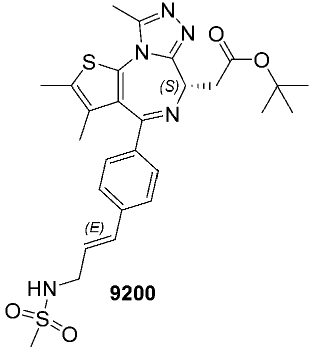 | **LCMS:** [M+H] ⁺ = 556.25; |
| | **¹H NMR:** (400 MHz, MeOD) |
| | δ 7.476 - 7.403 (m, 4H), 6.691 - 6.654 (d, J = 14.8 Hz, 1H), 6.415 - 6.345 (m, 1H), 4.578 - 4.526 (m, 1H), 3.876 - 3.861 (t, J = 6.0 Hz, 2H), 3.443 - 3.404 (d, J = 15.6 Hz, 2H), 2.955 (s, 3H), 2.693 (s, 3H), 2.455 (s, 3H), 1.706 (s, 3H), 1.495 (s, 9H). |
| 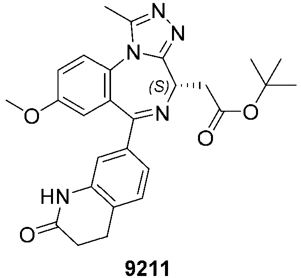 | **¹H NMR** (400 MHz, DMSO-d₆) δ 10.13 (s, 1H), 7.79 (d, J = 9.2 Hz, 1H), 7.40 - 7.37 (m, 1H), 7.19 (d, J = 7.6 Hz, 1H), 7.10 (s, 1H), 7.03 (d, J = 7.6 Hz, 1H), 6.90 (d, J = 2.8 Hz, 1H), 4.48 - 4.26 (m, 1H), 3.81 (s, 3 H), 3.33 - 3.22 (m, 2H), 2.93 - 2.87 (m, 2H), 2.52 (s, 3H), 2.47 - 2.37 (m, 2H). 1.42 (s, 9 H). |
| 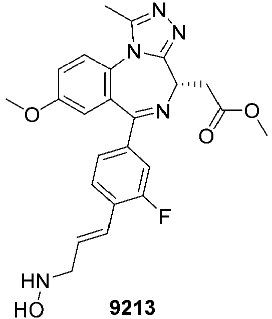 | **¹H NMR** (400 MHz, cdcl3) δ 7.64 - 7.50 (m, 1H), 7.44 (d, J = 8.8 Hz, 1H), 7.24 - 7.16 (m, 2H), 7.05 - 6.96 (m, 1H), 6.89 - 6.82 (m, 1H), 6.74 (d, J = 15.8 Hz, 1H), 6.60 (d, J = 16.2 Hz, 1H), 6.40 - 6.25 (m, 1H), 4.67 (t, J = 7.2 Hz, 1H), 4.54 - 4.44 (m, 1H), 3.99 (d, J = 7.2 Hz, 1H), 3.78 (dd, J = 16.8, 3.8 Hz, 6H), 3.64 - 3.51 (m, 3H), 2.76 (s, 3H). LC-MS: (ESI) m/z [M+H]⁺ 466.2. |
| 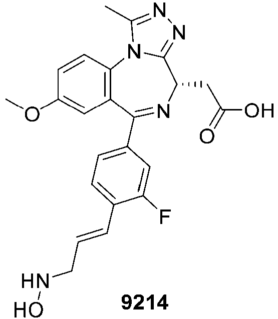 | **¹H NMR** (400 MHz, DMSO) δ 7.81 (d, J = 8.8 Hz, 1H), 7.49 (q, J = 8.2 Hz, 1H), 7.40 - 7.30 (m, 2H), 7.24 (d, J = 12.0 Hz, 1H), 6.79 (d, J = 2.8 Hz, 1H), 6.57 (d, J = 16.0 Hz, 1H), 6.47 (dt, J = 16.0, 5.8 Hz, 1H), 4.45 (t, J = 7.0 Hz, 1H), 3.76 (s, 3H), 3.50 (d, J = 5.6 Hz, 2H), 3.38 (d, J = 10.2 Hz, 1H), 3.26 (d, J = 9.0 Hz, 1H), 2.53 (s, 3H). ¹⁹F NMR (377 MHz, DMSO) δ -113.27. LC-MS: (ESI) m/z [M+H]⁺ 452.3. |
| 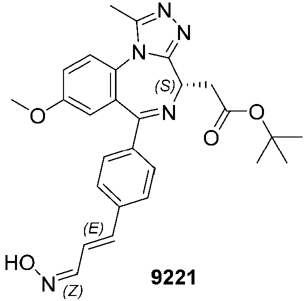 | **¹H NMR** (400 MHz, DMSO) δ 11.31 (s, 1H), 7.81 (d, J = 8.8 Hz, 1H), 7.62 (d, J = 8.4 Hz, 2H), 7.52 (d, J = 8.4 Hz, 2H), 7.44-7.38 (m, 2H), 7.32 (d, J = 9.4 Hz, 1H), 7.03 - 6.96 (m, 1H), 6.89 (d, J = 2.8 Hz, 1H), 4.41 (t, J = 7.2 Hz, 1H), 3.80 (s, 3H),3.31-3.28 (m, 2H), 2.54 (s, 3H), 1.43 (s, 9H). |
| | **LC-MS:** (ESI) m/z [M+H] ⁺ 488.2. |
| 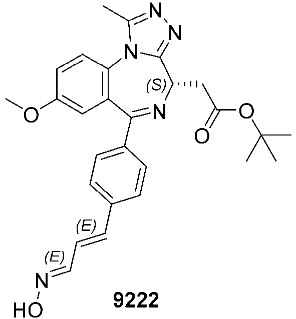 | **¹H NMR** (400 MHz, DMSO) δ 11.20 (s, 1H), 7.92 (d, J = 9.2 Hz, 1H), 7.80 (d, J = 8.8 Hz, 1H), 7.64 - 7.57 (m, 2H), 7.54-7.48 (m, 2H), 7.43-7.36 (m, 1H), 7.05-6.90 (m, 2H), 6.88 (d, J = 2.8 Hz, 1H), 4.43 - 4.38 (m, 1H), 3.80 (s, 3H), 3.40-3.35 (m, 2H), 2.54 (s, 3H), 1.43 (s, 9H). |
| | LC-MS: (ESI) m/z [M+H] ⁺ 488.2. |
| 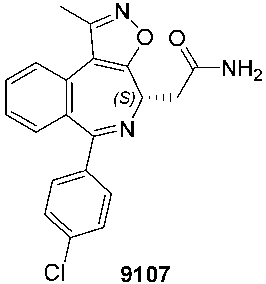 | **LCMS:** [M+H] ⁺ = 366.0; |
| | **¹H NMR:** (400 MHz, MeOD) |
| | δ 7.800 (d, J = 8.0 Hz, 1H), 7.722 - 7.674 (m, 1H), 7.425 - 7.386 (m, 2H), 7.352 (s, 4H), 4.440 (s, 1H), 2.653 (s, 2H), 2.560 (s, 3H). |
| 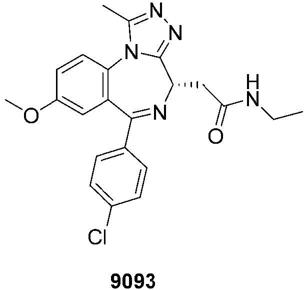 | **LCMS:** [M+H] ⁺ = 424.1; |
| | **¹H NMR:** (400 MHz, MeOD) |
| | δ 7.715 (d, J = 8.8 Hz, 1H), 7.540 (d, J = 8.4 Hz, 2H), 7.411 (d, J = 8.4 Hz, 2H), 7.374 (dd, J = 9.2, 2.8 Hz, 1H), 6.915 (d, J = 2.8 Hz, 1H), 4.620 (dd, J = 9.2, 5.2 Hz, 1H), 3.819 (s, 3H), 3.390 (dd, J = 14.8, 9.2 Hz, 1H), 3.281 - 3.224 (m, 2H), 3.230 - 3.192 (m, 1H), 2.630 (s, 3H), 1.181 (t, J = 7.2 Hz, 3H). |

### Test Example 1. Cell Proliferation Assay

The cell line used was 22RV1 or LNCaP, and the medium was RPMI-1640 containing 10% FBS and antibiotics. All incubations were performed at 37 °C in a humidified carbon dioxide incubator.

On day 1, cells were digested with 0.25% trypsin (containing EDTA) and collected by centrifugation. The cells were seeded in a 96-well plate (white wall/transparent bottom) at 5000 cells/well and cultured overnight.

On day 2, A stock solution of the compound at a concentration of 10 mM was subjected to an 8-fold serial dilution to obtain 8 concentration points. 2 µL of the compound solution at each concentration point was added to 98 µL of fresh medium, and the mixture was mixed homogeneously. Then, 5 µL of the compound solution diluted in the medium at each concentration point was added to 100 µL of overnight medium containing the cells, and 2 replicates were set for each concentration. The mixture was cultured for 72 h.

After 72 h of compound treatment, the plate was equilibrated to room temperature, 100 µL of CellTiterGlo (Promega) was added. The mixture was mixed homogeneously for 2 min, left to stand at room temperature for 10 min, and then read for luminescence values on a SpectraMax M5 microplate reader to evaluate cell viability. The positive control was 1% Tween-20 and the negative control was 0.1% DMSO. IC₅₀ was generated by 4-parameter non-linear regression fitting in GraphPad Prism.

| Compound | 22Rv1 ATP average IC₅₀ (nM) | LNCap ATP average IC₅₀ (nM) |
|---|---|---|
| Core1 | | |
| GNE-0011 | A | A |
| 9001 | D | C |
| 9002 | A | A |
| 9003 | D | B |
| 9010 | D | D |
| 9011 | A | A |
| 9012 | C | B |
| 9013 | C | A |
| 9017 | C | A |
| 9018 | D | D |
| 9019 | C | B |
| 9020 | D | D |
| 9021 | D | B |
| 9023 | D | D |
| 9025 | C | B |
| 9026 | D | D |
| 9038 | B | C |
| 9041 | B | A |
| 9045 | B | A |
| 9046 | A | B |
| 9047 | A | D |
| 9053 | B | B |
| 9055 | A | A |
| 9058 | D | C |
| 9059 | B | A |
| 9060 | C | B |
| 9061 | B | C |
| 9062 | B | B |
| 9064 | A | A |
| 9065 | A | A |
| 9066 | C | A |
| 9067 | B | B |
| 9068 | B | B |
| 9071 | B | B |
| 9072 | B | A |
| 9073 | B | A |
| 9074 | B | A |
| 9075 | C | A |
| 9076 | D | C |
| 9083 | A | - |
| 9084 | C | B |
| 9085 | D | B |
| 9086 | C | C |
| 9087 | D | C |
| 9090 | D | B |
| 9091 | B | C |
| 9094 | B | B |
| 9103 | A | A |
| 9104 | A | - |
| 9105 | C | - |
| 9109 | C | B |
| 9110 | D | D |
| 9111 | C | B |
| 9112 | B | B |
| 9113 | C | - |
| 9114 | B | - |
| 9118 | C | A |
| 9123 | A | A |
| 9126 | B | - |
| 9132-A | B | A |
| 9132-B | B | B |
| 9135 | B | A |
| 9136 | A | A |
| 9137 | A | A |
| 9138 | D | D |
| 9144 | A | A |
| 9148 | B | B |
| 9157 | C | C |
| 9160 | A | A |
| 9166 | A | A |
| 9168 | A | A |
| 9173 | A | A |
| 9184 | A | A |
| 9200 | B | B |
| 9230 | A | A |
| 9231 | B | A |
| 9234 | A | A |
| 9235 | C | B |
| 9236 | D | D |
| 9237 | D | D |
| 9240 | B | B |
| 9241 | - | - |
| 9242 | - | - |
| 9243 | A | A |
| 9244 | A | A |
| 9245 | D | D |
| 9246 | A | A |
| 9248 | A | A |
| 9249 | B | A |
| 9250 | A | A |
| 9253 | A | A |
| 9254 | A | A |
| 9255 | B | A |
| 9257 | B | - |
| 9258 | A | A |
| 9259 | A | A |
| 9260 | A | A |
| 9263 | D | D |
| 9264 | D | D |
| 9265 | A | A |

| Core 2 | | |
|---|---|---|
| 9051 | D | D |
| 9063 | D | D |
| 9069 | A | A |
| 9070 | D | D |
| 9077 | A | A |
| 9078 | A | A |
| 9079 | A | A |
| 9080 | A | B |
| 9092 | D | - |
| I-BET-762 | D | D |
| 9130 | A | A |
| 9131 | B | A |
| 9139 | B | A |
| 9140 | D | B |
| 9146 | A | A |
| 9149 | A | A |
| 9159 | D | D |
| 9167 | A | A |
| 9174 | C | B |
| 9185 | A | A |
| 9211 | D | D |
| 9213 | A | A |
| 9214 | D | D |
| 9221 | D | C |
| 9222 | C | B |
| 9256 | A | A |

| Core 3 | | |
|---|---|---|
| 9095 | B | B |
| 9097 | D | D |
| 9098 | D | D |
| 9122 | D | B |
| 9141 | D | D |

| | | |
|---|---|---|
| Note: A < 50 nM, 50 nM < B < 200 nM, 200 nM < C <500 nM, and 500 nM < D | | |

### Test Example 2. BRD4 Degradation Assay

The conventional Western-blot method was performed as follows:
1. Cells (most commonly 22RV1 and LnCap) were passaged and seeded one day in advance. The cell inoculation liquid was prepared at a density of 0.67×10⁶ cells/mL and seeded into a 12-well plate (0.75 mL/well) or a 24-well plate (0.375 mL/well). (that is, the 12-well plate: 0.5×10⁶ cells/well; the 24-well plate: 0.25× 10⁶ cells/well).
2. The compound was added after 24-26 h. The compound at a concentration 4 times the test concentration was dissolved in the medium: 150 µL (12-well plate) or 75 µL (24-well plate). The mixture was mixed homogeneously after the compound was added to culture wells.
3. Cell homogenates (12-well plate: 100 µL/well; 24-well plate: 50 µL/well) were prepared with an RIPA buffer at the indicated degradation assay time. The mixture was incubated three times under a condition of Vortex + ice. The mixture was centrifuged at high speed (15800 rpm, 20,000 g) at 4 °C for 10 min. The supernatant was stored and the protein concentration was determined by the BCA method.
4. The specimen, a sample buffer, and a sample reducing agent were mixed homogeneously according to a ratio. The protein sample was denatured at 75 °C for 10 min and then immediately cooled. The mixture was centrifuged for 5 min. 5 µg of total protein was added to gel sample wells. The molecular-weight size markers were loaded into wells on both sides. Gel electrophoresis was performed at room temperature until the dye front reached the opening below.
5. The protein was transferred from the gel to a PVDF membrane over 60 min at a constant voltage of 20 V using a mini blot module (refer to the manufacturer's manual for detailed steps).
6. The PVDF membrane was cut and labeled. The membrane was blocked with 5% milk in TBST for 1 h. The membrane was incubated with a primary antibody in 1% bovine serum albumin at 4 °C overnight. The mixture was incubated with anti-Rb-Ig G-HRP at 1:10,000 in 5% BSA/TBST for 1.5 h. The membranes were washed three times with TBST for 10 min at each step interval. Color was developed on ChemiDoc with a chemiluminescence reagent. Images were acquired and analyzed. Quantitative analysis was performed using software of Chemidoc. The blank control (DMSO) was considered as 100% and the degree of target degradation was expressed as a percentage of the control. The grading method was as follows: expressing target content as percentage of control: A < 20%, 20% < B <50%, 50% < C <80, and 80% < D < 100%.
7. Dose-response curves were obtained and plotted using GraphPad Prism.

Simple Western method:
1- 3. The same as those in the conventional Western-blot method.
4. Specimens were prepared and loaded into the assay plate using a kit provided by ProteinSimple according to the method of the short user manual of the Wes automatic protein expression analysis system.
5. Detection and result collection were performed on the Wes or Jess automatic protein expression analyzer.
6. The results were compiled and quantitatively analyzed using Compass SW software. The quantification and grading methods are the same as those of the conventional Western-blot.

| Compound | BRD4 degradation of 22RV1 (200 nM 5 h) | BRD4 degradation of LNCap (200 nM 5 h) |
|---|---|---|
| GNE-0011 | B | - |
| 9011 | A | - |
| 9065 | B | - |
| 9071 | C | - |
| 9103 | B | - |
| 9104 | D | - |
| 9109 | C | - |
| 9111 | D | - |
| 9112 | D | - |
| 9113 | D | - |
| 9114 | C | - |
| 9132-A | D | - |
| 9132-B | D | - |
| 9135 | B | - |
| 9136 | C | - |
| 9137 | B | - |
| 9148 | C | - |
| 9160 | C | - |
| 9166 | A | - |
| 9173 | C | - |
| 9184 | A | - |
| 9077 | A | - |
| 9078 | B | - |
| 9079 | A | - |
| 9080 | B | - |
| 9130 | B | A |
| 9131 | B | A |
| 9139 | B | A |
| 9140 | B | B |
| 9146 | A | A |
| 9149 | A | A |
| 9167 | - | A |
| 9174 | C | B |
| 9185 | B | A |
| 9230 | A | - |
| 9231 | A | - |
| 9250 | D | - |
| 9253 | C | - |
| 9256 | C | - |
| 9259 | B | - |
| 9260 | A | - |
| 9265 | A | - |

The embodiments of the technical solutions of the present disclosure have been described above by way of example. It should be understood that the protection scope of the present disclosure is not limited to the embodiments described above. Any modification, equivalent replacement, improvement, and the like made by those skilled in the art without departing from the spirit and principle of the present disclosure shall fall within the protection scope of the claims of the present application.

## Claims

1. A compound represented by formula (I) and a racemate, a stereoisomer, a tautomer, an isotopically labeled compound, a nitrogen oxide, a solvate, a polymorph, a metabolite, an ester, a prodrug or a pharmaceutically acceptable salt thereof: 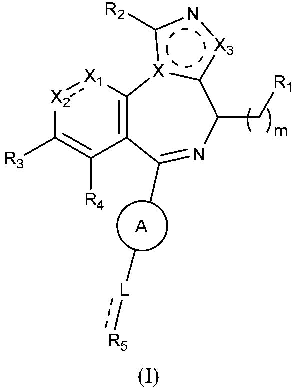 wherein,
------ represents a single bond or a double bond; 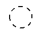 means that the ring in which it is present is aromatic; m is 1 or 2;
ring A is selected from the following groups unsubstituted or optionally substituted with one, two or more Rₐ: C₆₋₁₀ aryl and 5- to 10-membered heteroaryl; each Rₐ is identical or different and is independently selected from halogen, oxo (=O), C₁₋₁₂ alkyl, and C₁₋₁₂ alkoxy;
R₁ is selected from the following groups unsubstituted or optionally substituted with one, two or more R_{b}: 3- to 10-membered heterocyclyl, 5- to 10-membered heteroaryl, -C(O)OR₁₁, -C(O)N(R₁₂)(R₁₃), and -OC(O)R₁₄; R₁₁, R₁₂, R₁₃, and R₁₄ are identical or different and are independently selected from H, OH, NH₂, C₁₋₁₂ alkyl, C₃₋₁₂ cycloalkyl, 3- to 10-membered heterocyclyl, 5- to 10-membered heteroaryl, C₆₋₁₀ aryl, and 3- to 10-membered heterocyclyl-C₁₋₁₂ alkyl; each R_{b} is identical or different and is independently selected from OH, NH₂, C₁₋₁₂ alkyl, and C₂₋₁₂ alkynyl; R₂ is selected from C₁₋₁₂ alkyl, C₁₋₁₂ alkoxy, halogenated C₁₋₁₂ alkyl, and halogenated C₁₋₁₂ alkoxy;
R₃ and R₄ are identical or different and are independently selected from H, C₁₋₁₂ alkyl, C₁₋₁₂ alkoxy, halogenated C₁₋₁₂ alkyl, and halogenated C₁₋₁₂ alkoxy;
when 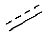 in 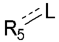
is a double bond, R₅ is selected from =NR₅₄ and 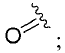
when 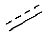 in 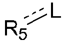
is a single bond, R₅ is selected from H, halogen, and the following groups unsubstituted or optionally substituted with one, two or more R_{c}: NH₂, C₁₋₁₂ alkyl, -C₁₋₁₂ alkyl-N(R₅₁)(R₅₂), -C(O)N(R₅₁)(R₅₂), -C₁₋₁₂ alkyl-O-R₅₃, -C(O)OR₅₃, -C₁₋₁₂ alkyl-NH-C(=NR₅₄)(N(R₅₁)(R₅₂)), -CH=N(R₅₄) , -S(O)₂R₅₅ and -C(O)R₅₅; R₅₁, R₅₂, R₅₃, R₅₄, and R₅₅ are identical or different and are independently selected from H, OH, NH₂, C₁₋₁₂ alkyl, C₁₋₁₂ alkoxy, C₁₋₁₂ alkyl-C(O)-, C₁₋₁₂ alkenyl -C(O)-, and C₁₋₁₂ alkyl-S(O)₂-; each R_{c} is identical or different and is independently selected from H, C₁₋₁₂ alkyl-C(O)-, Boc and Boc-O-;
X is selected from C and N;
X₁ is selected from S and C(R₆); R₆ is selected from H, C₁₋₁₂ alkyl, and C₁₋₁₂ alkoxy;
X₂ is absent or C(R₇); R₇ is selected from H, C₁₋₁₂ alkyl, and C₁₋₁₂ alkoxy;
X₃ is selected from N and C(R₈); R₈ is selected from H, C₁₋₁₂ alkyl, and C₁₋₁₂ alkoxy;
L is absent or selected from halogen, NH, and the following groups unsubstituted or optionally substituted with one, two or more R_{L}: C₁₋₁₂ alkylene, C₂₋₁₂ alkenylene, C₂₋₁₂ alkynylene, C₃₋₁₀ cycloalkylene, 3- to 10-membered heterocyclylene, C₆₋₁₀ arylene, and 5- to 10-membered heteroarylene; each R_{L} is identical or different and is independently selected from H, halogen, oxo (=O), C₁₋₁₂ alkyl, and C₁₋₁₂ alkoxy.

2. The compound and the racemate, the stereoisomer, the tautomer, the isotopically labeled compound, the nitrogen oxide, the solvate, the polymorph, the metabolite, the ester, the prodrug or the pharmaceutically acceptable salt thereof according to claim 1, wherein ring A is selected from the following groups unsubstituted or optionally substituted with one, two or more Rₐ: phenyl and 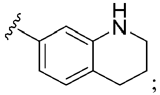 preferably, ring A is selected from

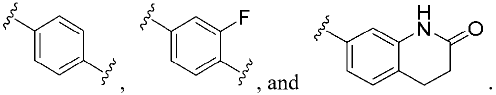

3. The compound and the racemate, the stereoisomer, the tautomer, the isotopically labeled compound, the nitrogen oxide, the solvate, the polymorph, the metabolite, the ester, the prodrug or the pharmaceutically acceptable salt thereof according to claim 1 or 2, wherein R₁ is selected from the following groups unsubstituted or optionally substituted with one, two or more R_{b}: C₁₋₆ alkyl, 5- to 8-membered heterocyclyl, 5- to 6-membered heteroaryl(such as thiazolyl, oxazolyl, oxadiazolyl, triazolyl), -C(O)OR₁₁, -C(O)N(R₁₂)(R₁₃), and -OC(O)R₁₄; R₁₁, R₁₂, R₁₃, and R₁₄ are identical or different and are independently selected from H, OH, NH₂, C₁₋₆ alkyl, C₃₋₈ cycloalkyl, 3- to 8-membered heterocyclyl, 5- to 6-membered heteroaryl, C₆₋₁₀ aryl, and 3- to 8-membered heterocyclyl-C₁₋₆ alkyl; each R_{b} is identical or different and is independently selected from OH, NH₂, C₁₋₆ alkyl, C₂₋₆ alkynyl and -OC(O)R₁₄; preferably, R₁₁, R₁₂, R₁₃ and R₁₄ are identical or different and are independently selected from H, OH, NH₂, methyl, ethyl, isopropyl, tert-butyl, hydroxyphenyl, propynyl, hydroxyethyl, methylcyclopropyl, 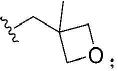
preferably, R₁ is selected from 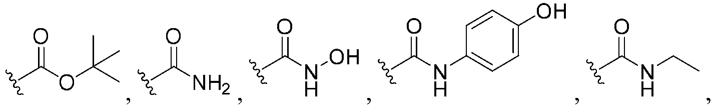 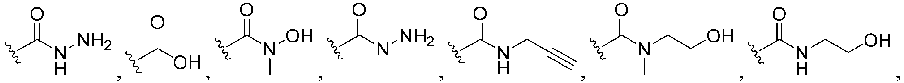 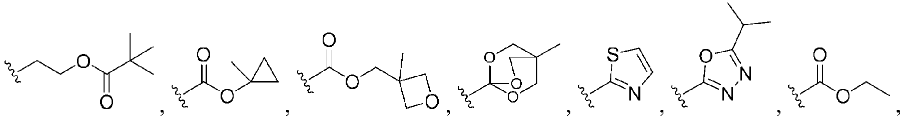 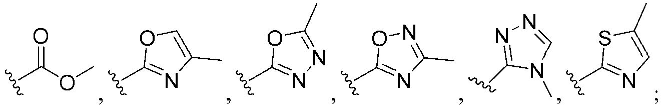
preferably, R₂ is selected from C₁₋₆ alkyl, e.g., methyl;
preferably, R₃ and R₄ are identical or different and are independently selected from H, C₁₋₆ alkyl, and C₁₋₆ alkoxy, e.g., H, methyl, and methoxy;
preferably, when 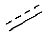 in 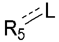
is a double bond, R₅ is selected from =NR₅₄, and 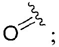 when 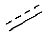 in 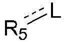
is a single bond, R₅ is selected from H, halogen, and the following groups unsubstituted or optionally substituted with one, two or more R_{c}: NH₂, C₁₋₆ alkyl, -C₁₋₆ alkyl-N(R₅₁)(R₅₂), -C(O)N(R₅₁)(R₅₂), -C₁₋₆ alkyl-O-R₅₃, -C(O)OR₅₃, -C₁₋₆ alkyl-NH-C(=NR₅₄)(N(R₅₁)(R₅₂)), -CH=N(R₅₄), -S(O)₂R₅₅ and -C(O)R₅₅; R₅₁, R₅₂, R₅₃, and R₅₄ are identical or different and are independently selected from H, OH, NH₂, C₁₋₆ alkyl, halogenated C₁₋₆ alkyl, C₂₋₆ alkynyl, C₁₋₆ alkoxy, C₁₋₆ alkyl-C(O)-, C₂₋₆ alkenyl-C(O)-, and C₁₋₆ alkyl-S(O)₂-; each R_{c} is identical or different and is independently selected from H,C₁₋₆ alkyl-C(O)-, Boc and Boc-O-;
preferably, when 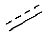 in 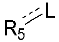
is a double bond, R₅ is selected from 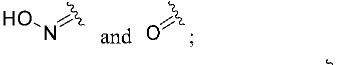
when 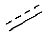 in 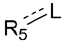
is a single bond, R₅ is selected from H, Cl, NH₂, methyl,

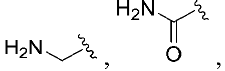

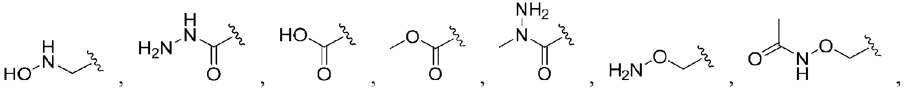

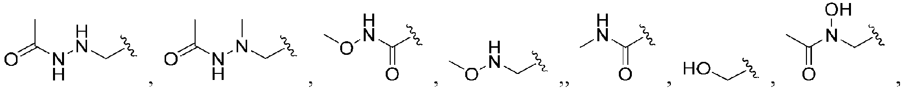

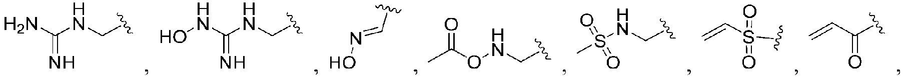

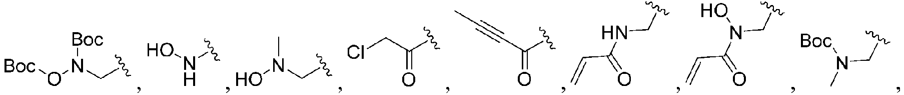

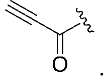

4. The compound and the racemate, the stereoisomer, the tautomer, the isotopically labeled compound, the nitrogen oxide, the solvate, the polymorph, the metabolite, the ester, the prodrug or the pharmaceutically acceptable salt thereof according to any one of claims 1 to 3, wherein X₁ is selected from S and C(R₆); R₆ is selected from H, C₁₋₆ alkyl, and C₁₋₆ alkoxy;
X₂ is absent or C(R₇); R₇ is selected from H, C₁₋₆ alkyl, and C₁₋₆ alkoxy;
when X₂ is absent, 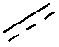 in 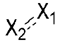
is a single bond, and X₁ is S; when X₂ is C(R₇), 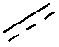 in 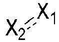
is a double bond, and X₁ is C(R₆);
preferably, X₁ is selected from S and CH, and X₂ is absent or CH; when X₂ is absent, 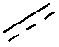 in 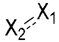
is a single
bond, and X₁ is S; when X₂ is CH, 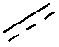 in 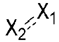
is a double bond, and X₁ is CH;
preferably, X₃ is selected from N and CH;
preferably, 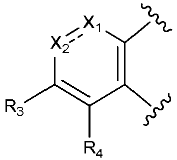
is selected from 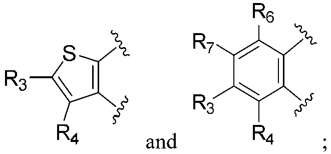
preferably, 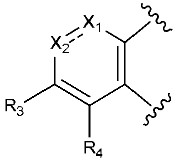
is selected from 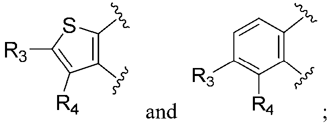
preferably, 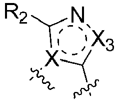
is selected from 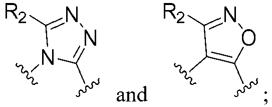 preferably, L is absent or selected from halogen, NH, and the following groups unsubstituted or optionally substituted with one, two or more R_{L}: C₁₋₆ alkylene, C₂₋₆ alkenylene, C₂₋₆ alkynylene, C₃₋₈ cycloalkylene, 3- to 8-membered heterocyclylene, C₆₋₁₀ arylene, and 5- to 6-membered heteroarylene; each R_{L} is identical or different and is independently selected from H, halogen, oxo (=O), C₁₋₆ alkyl, and C₁₋₆ alkoxy;
preferably, L is absent or selected from Cl, NH, and the following groups unsubstituted or optionally substituted with one, two or more R_{L}: vinylene, ethynylene, 2,3-dihydroisoxazolylene, pyrazolylene, thiazolylene, furylene, azetidinylene, pyrrolylene, 2-azaspiro[3.3]heptanylene, thienylene, phenylene, cyclopropylene, cyclohexenylene, or cyclopentenylene;
preferably, L is absent or selected from Cl, NH,

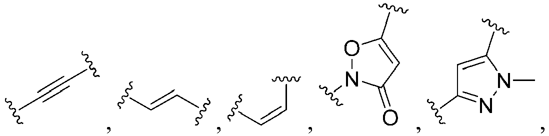

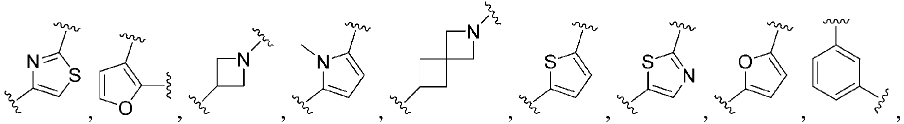

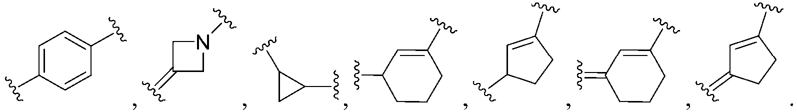

5. The compound and the racemate, the stereoisomer, the tautomer, the isotopically labeled compound, the nitrogen oxide, the solvate, the polymorph, the metabolite, the ester, the prodrug or the pharmaceutically acceptable salt thereof according to any one of claims 1 to 4, wherein an exemplary specific compound of the compound represented by formula (I) is as follows: 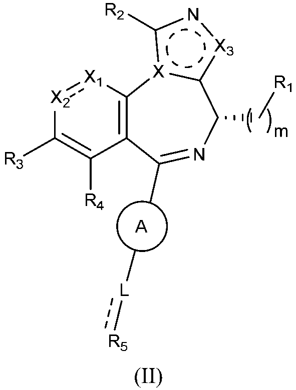
wherein, -̅ -̅ -̅ -̅ -̅ -̅ represents a single bond or a double bond; 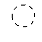
means that the ring in which it is present is aromatic; ring A, R₁, R₂, R₃, R₄, R₅, X₁, X₂, L, and m are as defined in any one of claims 1 to 4;
preferably, the compound represented by formula (I) is selected from the following structures: 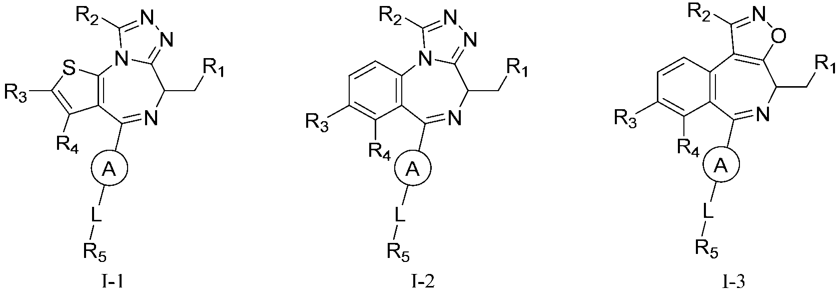
wherein, ring A, R₁, R₂, R₃, R₄, R₅, and L are as defined in any one of claims 1 to 4.

6. The compound and the racemate, the stereoisomer, the tautomer, the isotopically labeled compound, the nitrogen oxide, the solvate, the polymorph, the metabolite, the ester, the prodrug or the pharmaceutically acceptable salt thereof according to any one of claims 1 to 5, wherein the compound represented by formula (I) is selected from the following compounds: 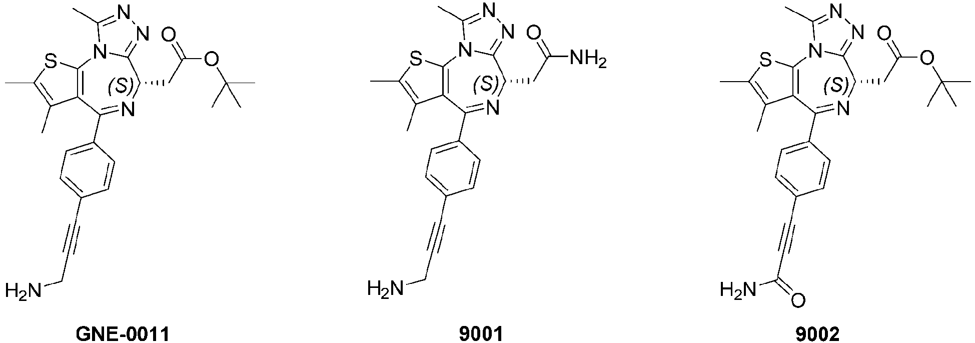 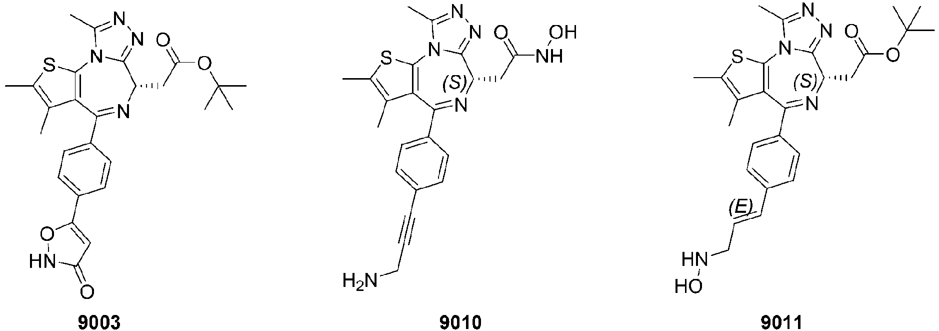 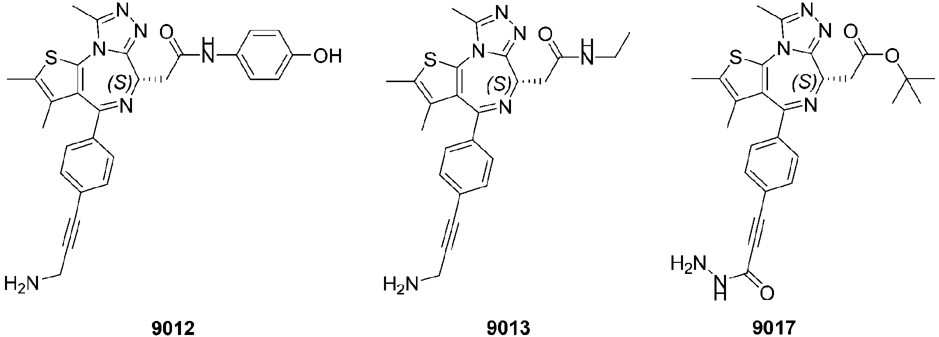 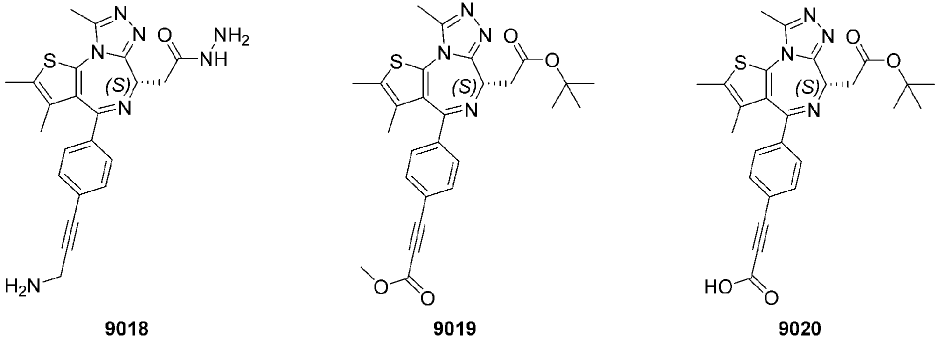 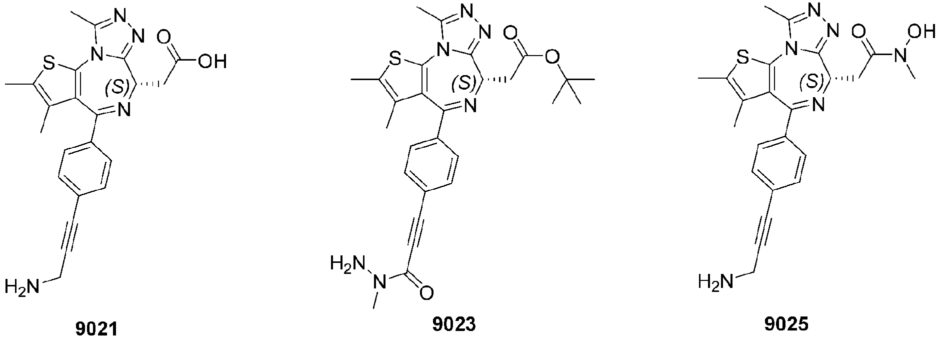 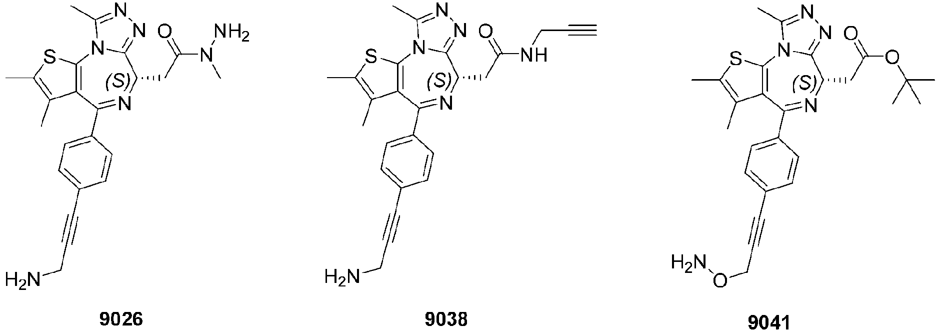 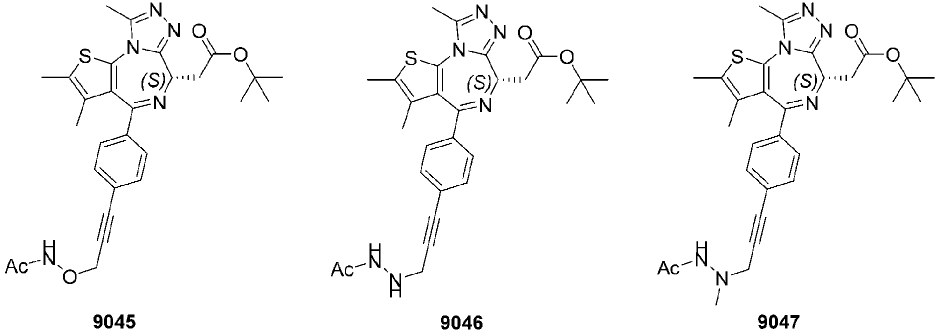 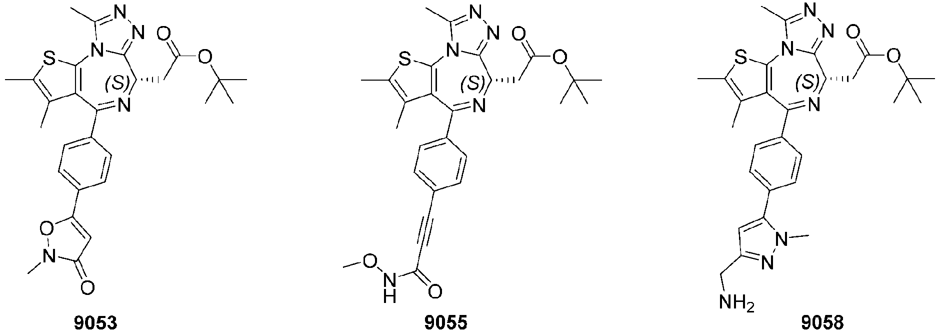 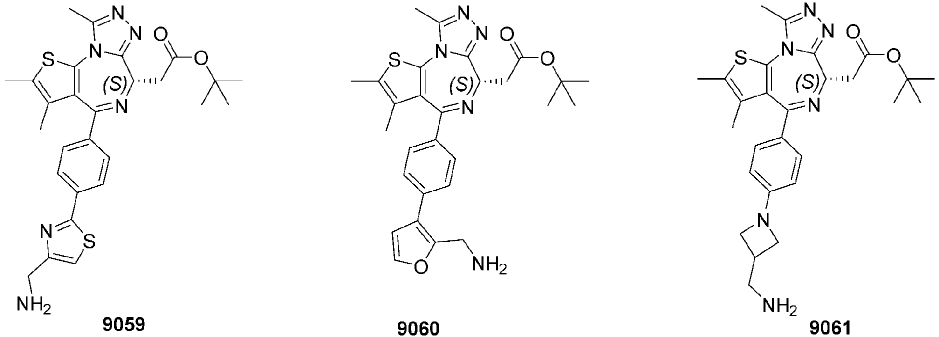 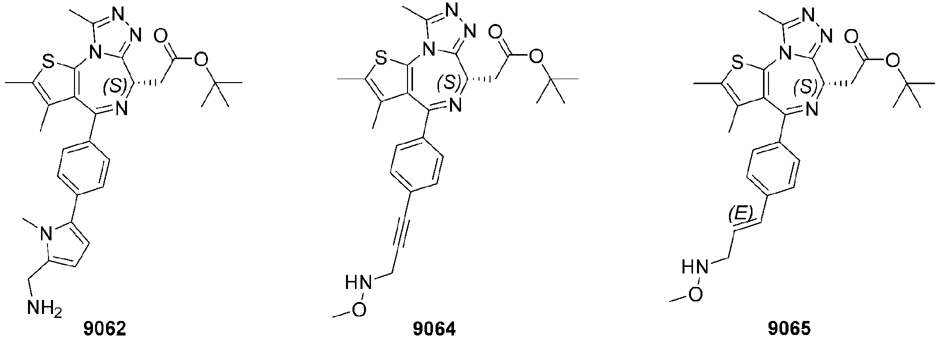 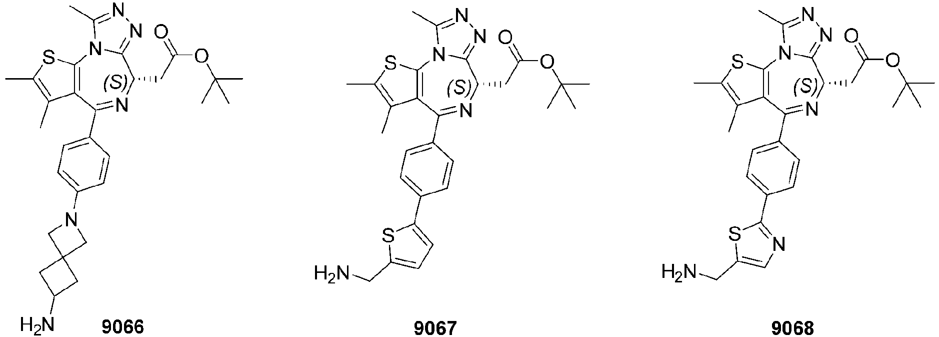 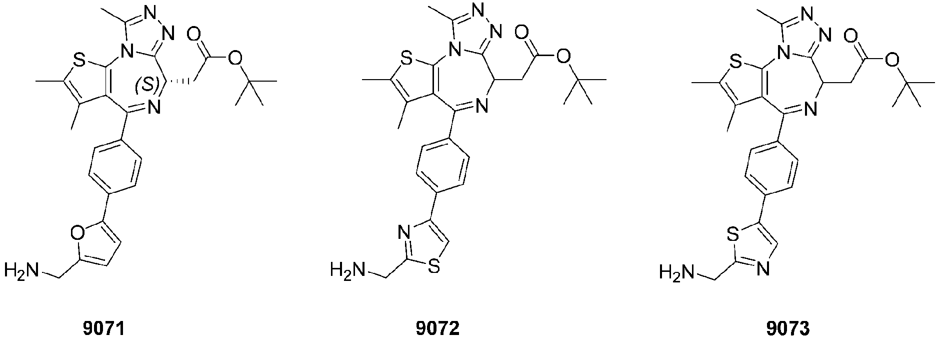 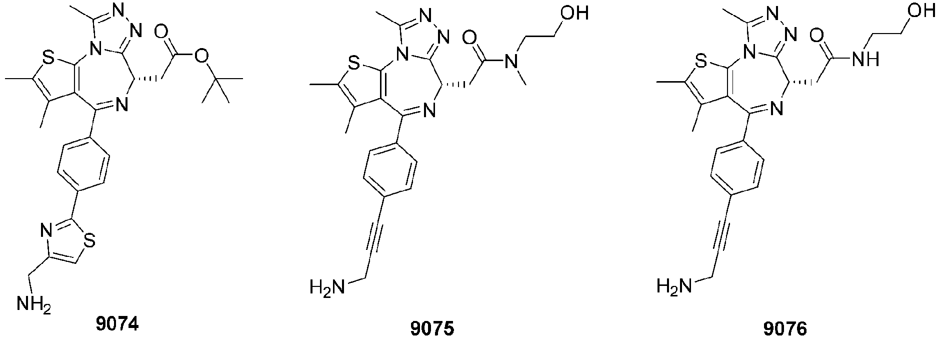 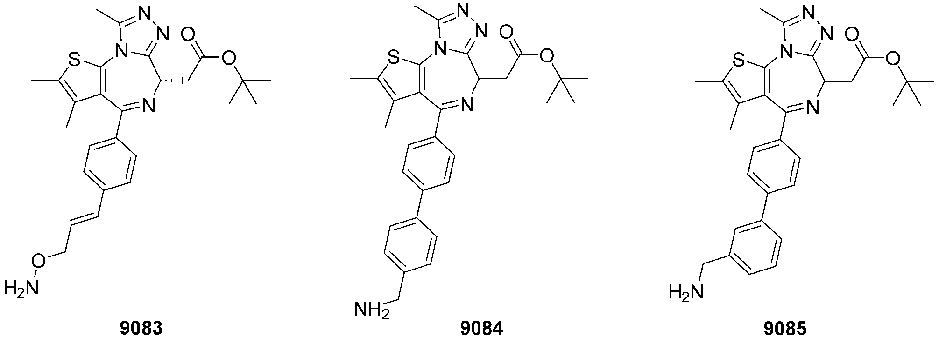 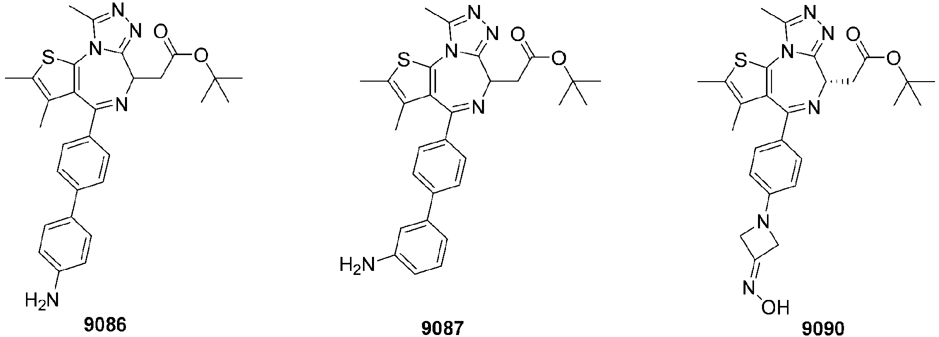 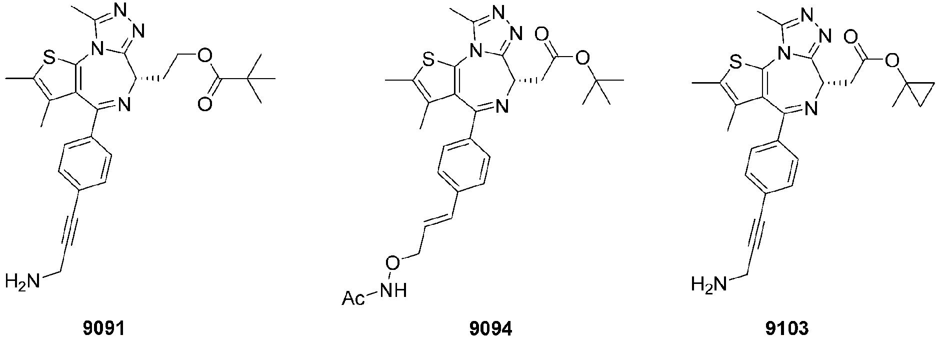 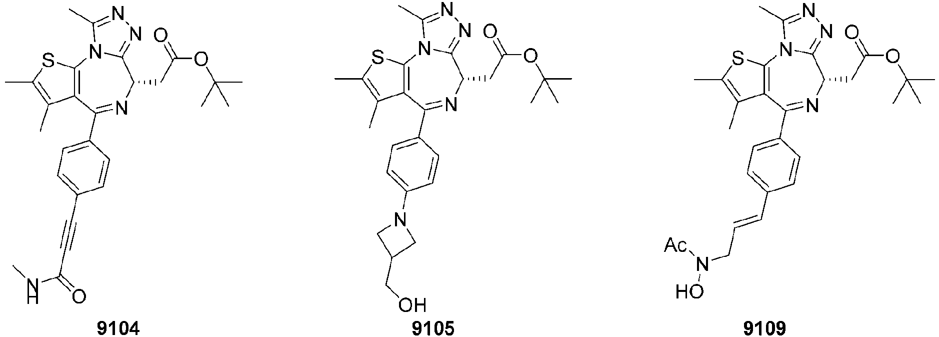 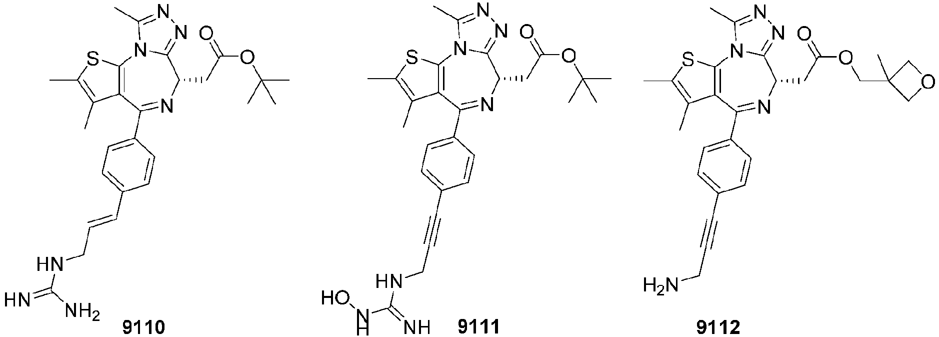 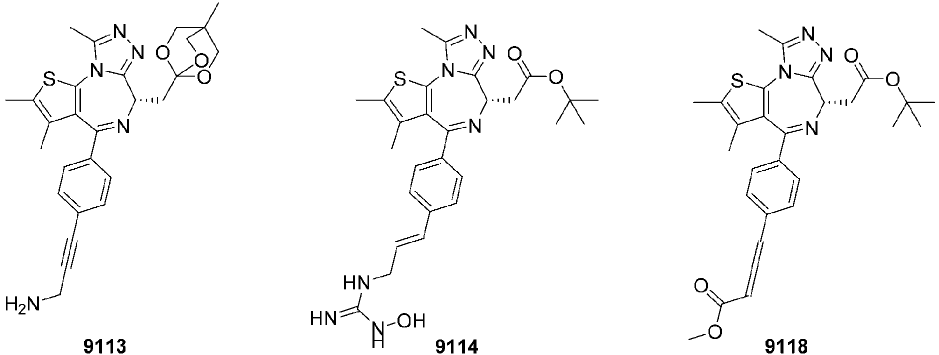 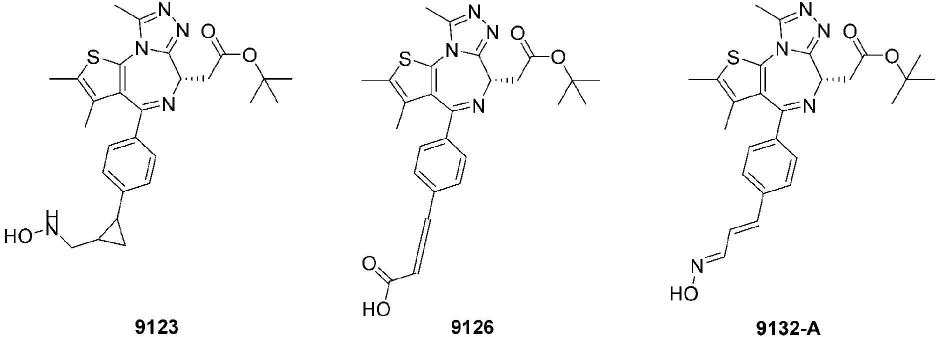 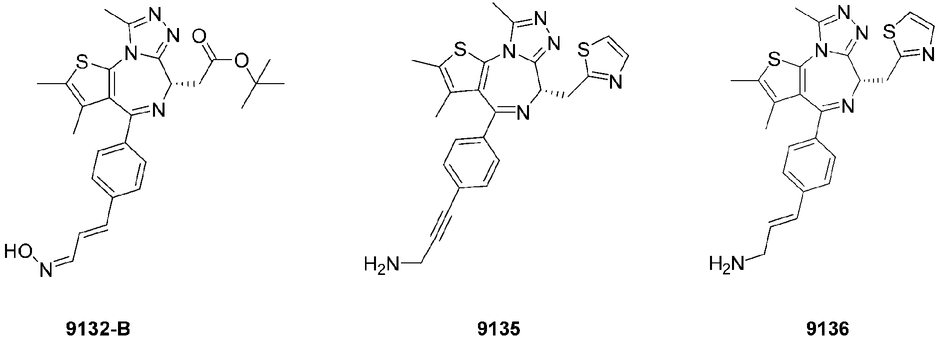 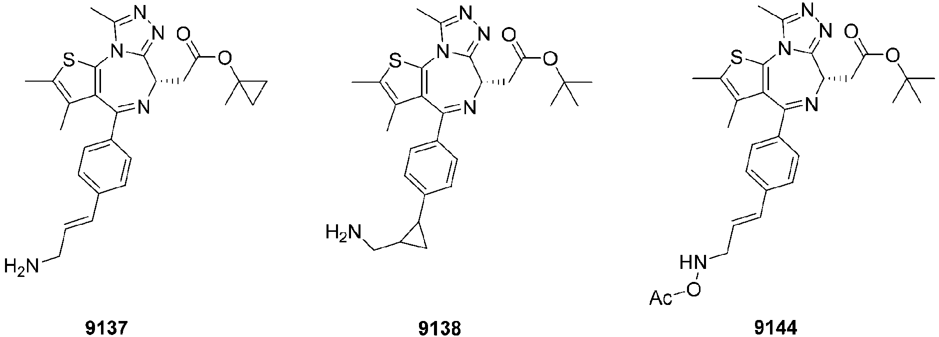 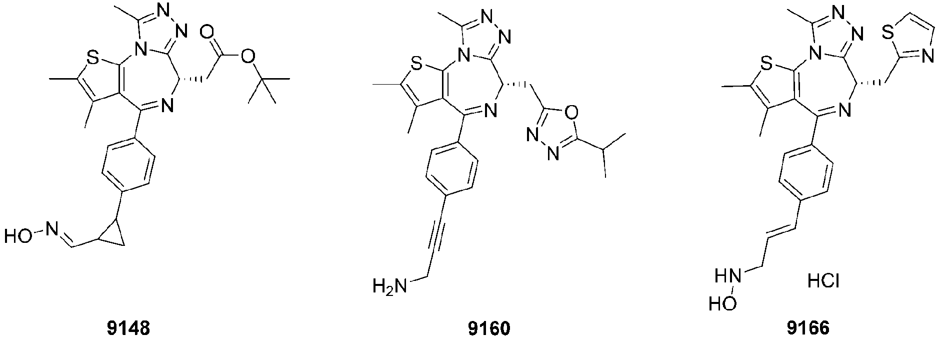 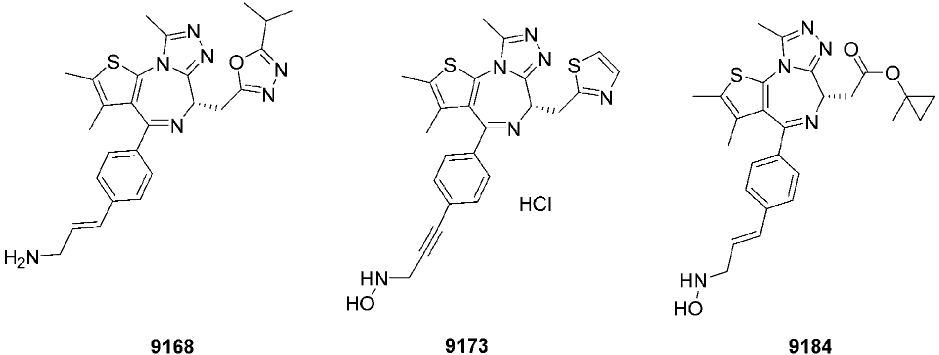 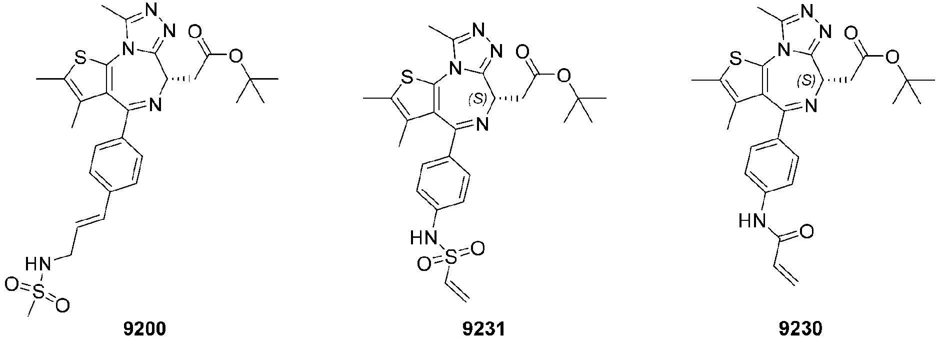 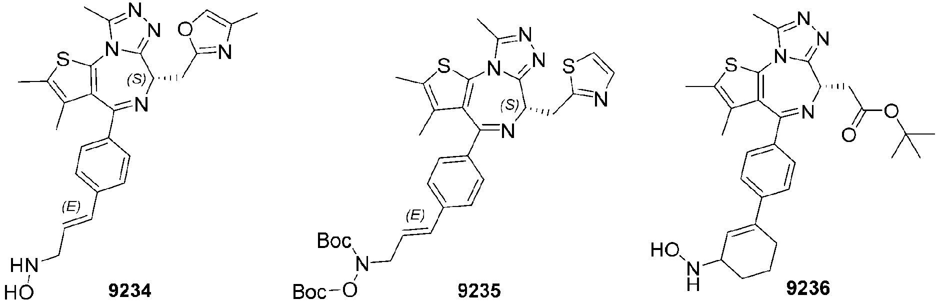 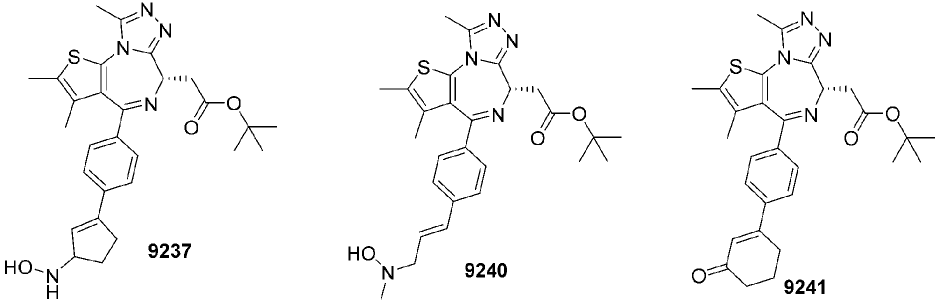 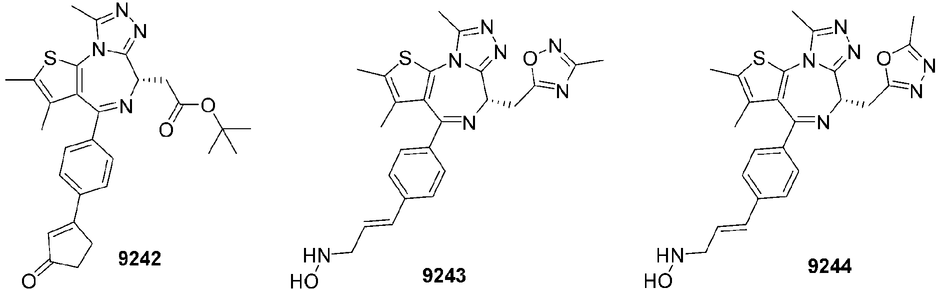 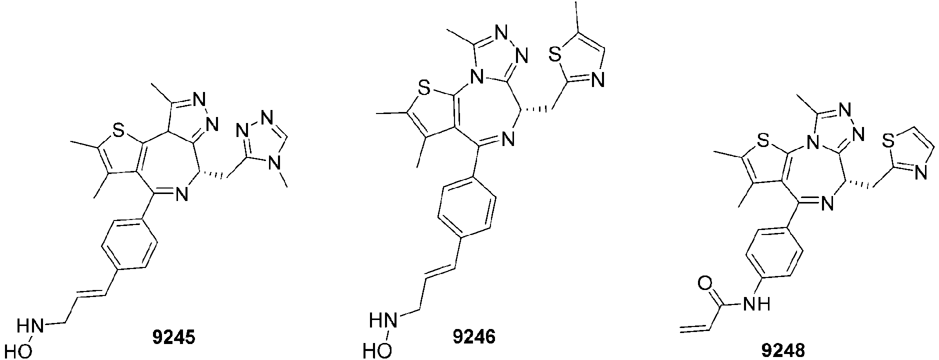 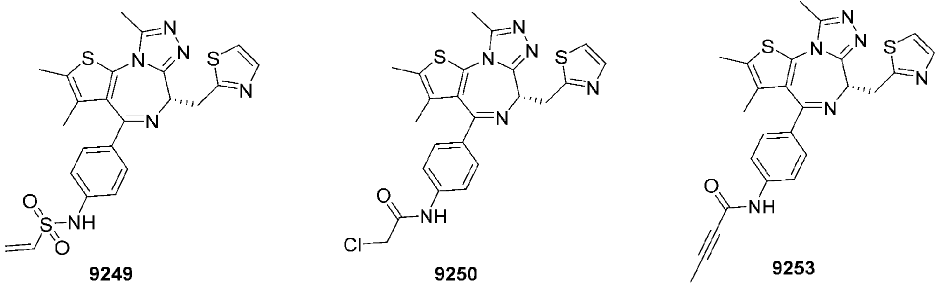 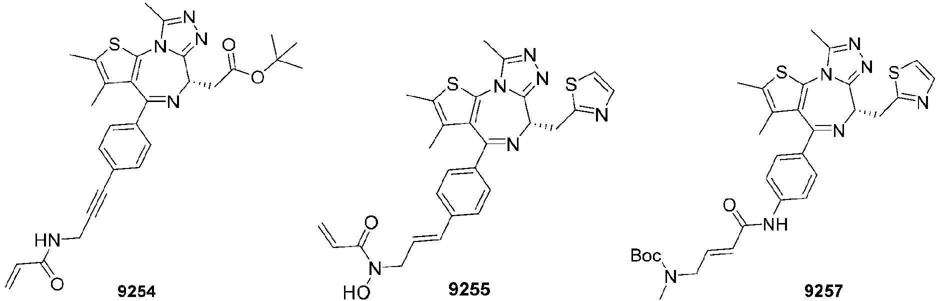 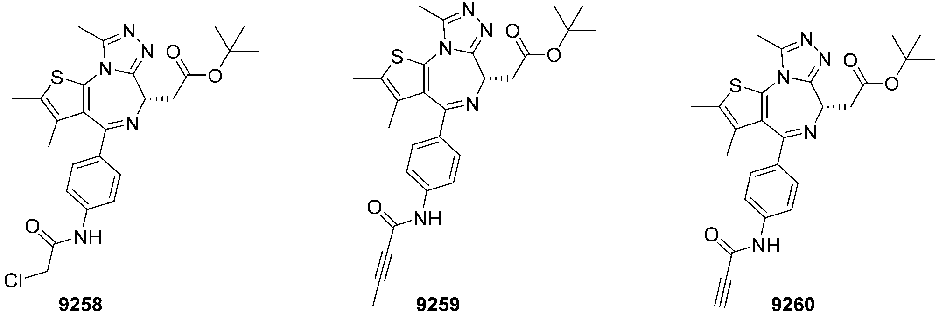 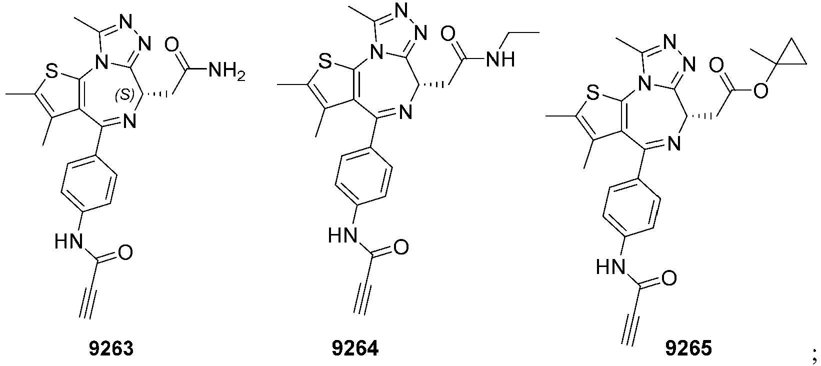
preferably, exemplary specific compounds of the compound represented by formula (I) are as follows: 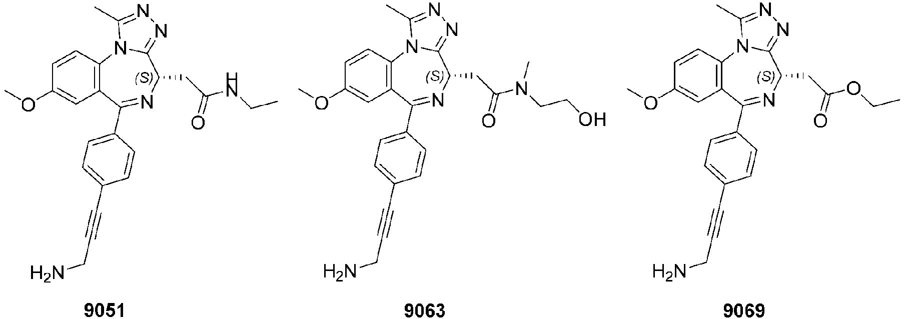 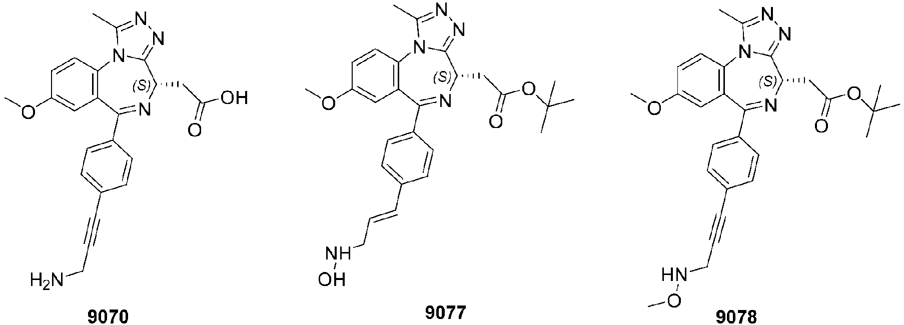 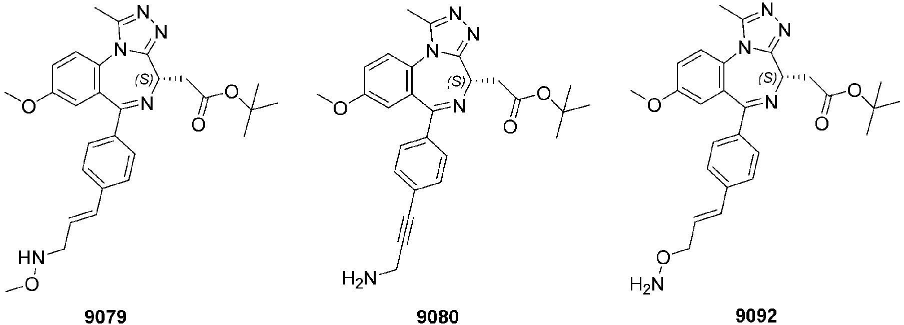 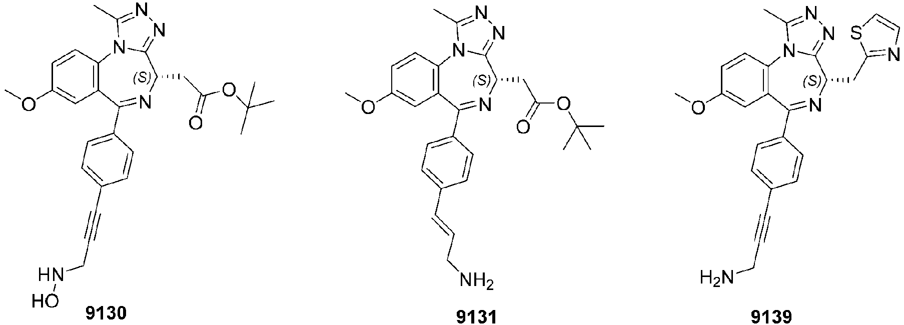 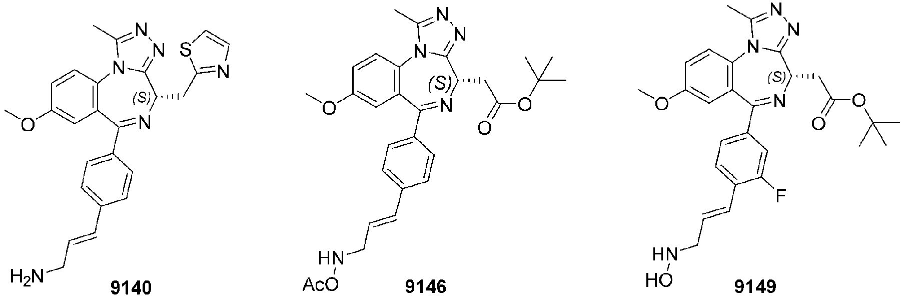 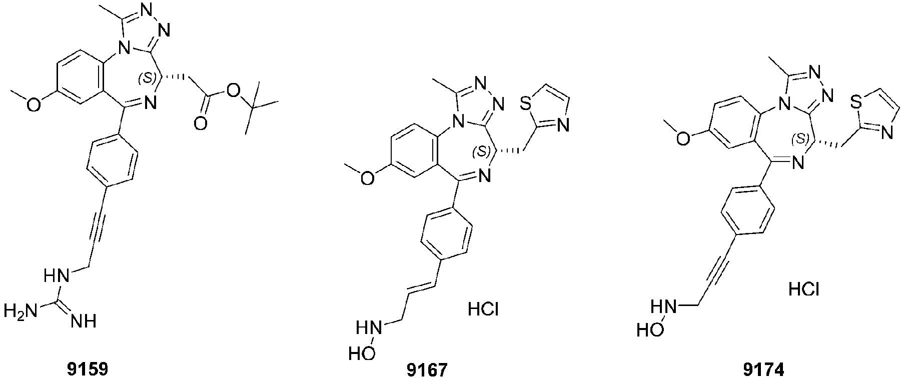 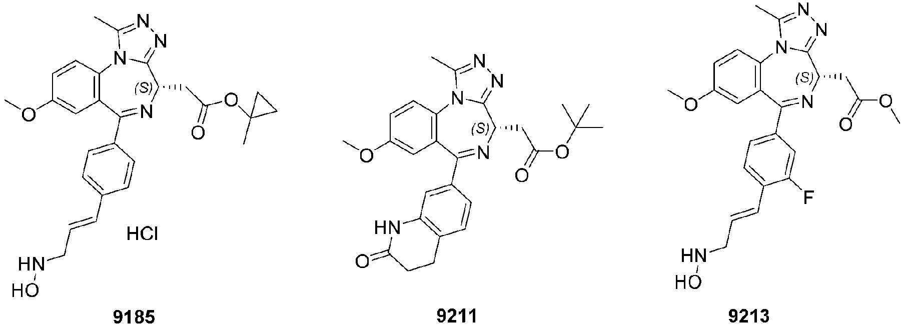 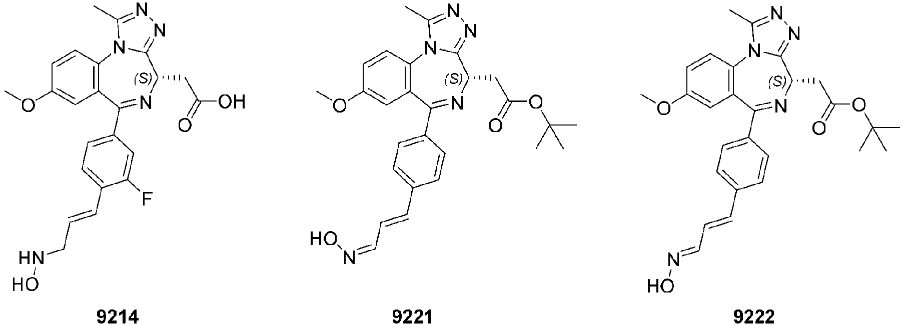 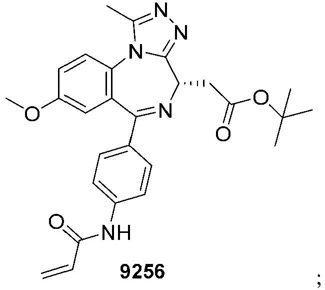
preferably, exemplary specific compounds of the compound represented by formula (I) are as follows:

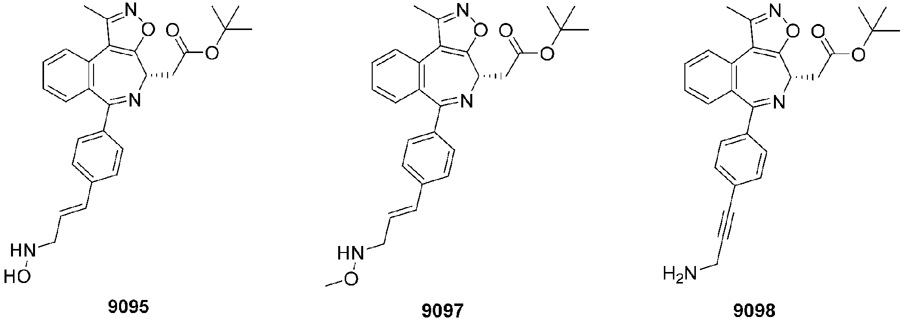

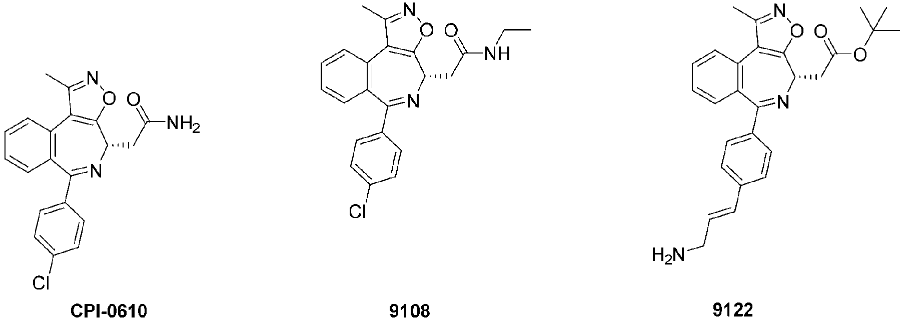

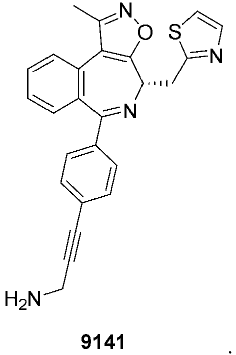

7. A compound represented by formula (III) and a racemate, a stereoisomer, a tautomer, an isotopically labeled compound, a nitrogen oxide, a solvate, a polymorph, a metabolite, an ester, a prodrug or a pharmaceutically acceptable salt thereof: 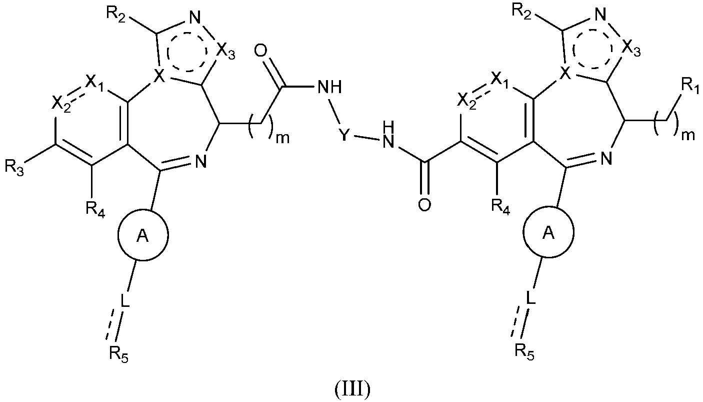
wherein, ring A, R₁, R₂, R₃, R₄, R₅, L, and m are as defined in any one of claims 1 to 6; Y is selected from -(CH₂)ₚ-[O-(CH₂)_{q}]ᵣ, wherein p is selected from 1, 2, 3, 4, and 5, q is selected from 1, 2, 3, 4, and 5, and r is selected from 0, 1, 2, 3, 4, and 5;
preferably, the compound represented by formula (III) has the following structure:

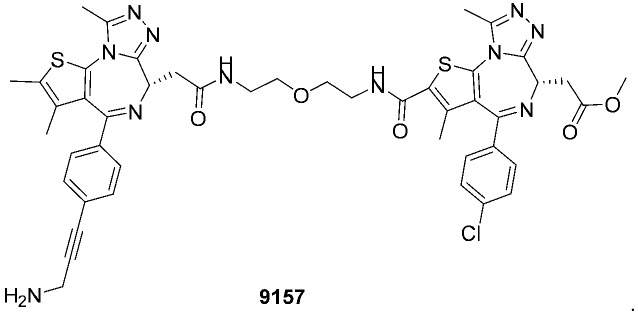

8. A pharmaceutical composition comprising the compound and the racemate, the stereoisomer, the tautomer, the isotopically labeled compound, the nitrogen oxide, the solvate, the polymorph, the metabolite, the ester, the prodrug or the pharmaceutically acceptable salt thereof according to any one of claims 1 to 7.

9. Use of the compound and the racemate, the stereoisomer, the tautomer, the isotopically labeled compound, the nitrogen oxide, the solvate, the polymorph, the metabolite, the ester, the prodrug or the pharmaceutically acceptable salt thereof according to any one of claims 1 to 7, and the pharmaceutical composition comprising the same for manufacturing a medicament for the prevention and/or treatment of a bromodomain-mediated disease or condition, wherein
preferably, a protein comprising the bromodomain is bromodomain-containing protein 4 (BRD4);
preferably, the disease is cancer;
preferably, the cancer is selected from the group consisting of: acoustic neuroma, acute leukemia, acute lymphocytic leukemia, acute myeloid leukemia, acute T cell leukemia, basal cell carcinoma, cholangiocarcinoma, bladder cancer, brain cancer, breast cancer, bronchial carcinoma, cervical cancer, chondrosarcoma, chordoma, choriocarcinoma, chronic leukemia, chronic lymphocytic leukemia, chronic myeloid (granulocytic) leukemia, chronic myelogenous leukemia, colon cancer, colorectal cancer, craniopharyngioma, cystadenocarcinoma, diffuse large B-cell lymphoma, dysplasia, embryonic carcinoma, endometrial cancer, endotheliosarcoma, ependymoma, epithelial cancer, erythroleukemia, esophagus cancer, estrogen receptor-positive breast cancer, essential thrombocythemia, Ewing's tumor, fibrosarcoma, follicular lymphoma, germ cell testicular cancer, glioma, glioblastoma, gliosarcoma, heavy chain disease, hemangioblastoma, hepatoma, hepatocellular carcinoma, hormone-insensitive prostate cancer, leiomyosarcoma, leukemia, liposarcoma, lung cancer, lymphangioendothelial sarcoma, lymphangiosarcoma, lymphoblastic leukemia, lymphomas (Hodgkin's lymphoma and non-Hodgkin's lymphoma), malignancies and hyperproliferative disorders of the bladder, breast, colon, lung, ovary, pancreas, prostate, skin, and uterus, lymphoid malignancies of T-cell or B-cell origin, medullary carcinoma, medulloblastoma, melanoma, meningioma, mesothelioma, multiple myeloma, myelogenous leukemia, myeloma, myxosarcoma, neuroblastoma, NUT midline carcinoma (NMC), non-small cell lung cancer, oligodendroglioma, oral cancer, osteogenic sarcoma, ovarian cancer, pancreatic cancer, papillary adenocarcinoma, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung cancer, solid tumors (carcinoma and sarcoma), small cell lung cancer, gastric cancer, squamous cell carcinoma, synovial tumor, sweat gland carcinoma, thyroid cancer, Waldenström macroglobulinemia, testiculoma, uterine cancer, and Wilms' tumor;
preferably, the disease is an autoimmune disease or inflammatory disease selected from the group consisting of: Addison's disease, acute gout, ankylosing spondylitis, asthma, atherosclerosis, Behcet's disease, bullous skin disease, chronic obstructive pulmonary disease (COPD), Crohn's disease, dermatitis, eczema, giant cell arteritis, glomerulonephritis, hepatitis, hypophysitis, inflammatory bowel disease, kawasaki disease, lupus nephritis, multiple sclerosis, myocarditis, myositis, nephritis, organ transplant rejection, osteoarthritis, pancreatitis, pericarditis, polyarteritis nodosa, pneumonia, primary biliary cirrhosis, psoriasis, psoriatic arthritis, rheumatoid arthritis, scleritis, sclerosing cholangitis, sepsis, systemic lupus erythematosus, Takayasu arteritis, toxic shock, thyroiditis, type I diabetes, ulcerative colitis, uveitis, vitiligo, vasculitis, and Wegener's granulomatosis;
preferably, the disease or condition is selected from the group consisting of: AIDS; chronic kidney disease, diabetic nephropathy, hypertensive nephropathy, HIV-associated nephropathy, glomerulonephritis, lupus nephritis, IgA nephropathy, focal segmental glomerulosclerosis, membranous glomerulonephritis, minimal change nephropathy, polycystic kidney disease, and tubulointerstitial nephritis; acute kidney injury or disease or condition; obesity; dyslipidemia; hypercholesterolemia; Alzheimer's disease; metabolic syndrome; hepatic steatosis; type II diabetes; insulin resistance; and diabetic retinopathy.

10. A method for degrading a bromodomain-containing protein in a cell, comprising exposing the cell to an effective amount of the compound and the racemate, the stereoisomer, the tautomer, the isotopically labeled compound, the nitrogen oxide, the solvate, the polymorph, the metabolite, the ester, the prodrug or the pharmaceutically acceptable salt thereof according to any one of claims 1 to 7, or a pharmaceutical composition comprising the same, wherein the compound achieves degradation of the bromodomain-containing protein;
preferably, the bromodomain-containing protein is bromodomain-containing protein 4 (BRD4).
